# EUROPEAN PATENT SPECIFICATION

(11) **EP 0 962 537 B1**
(45) Date of publication and mention of the grant of the patent: **17.06.2009**
(21) Application number: 99110780.6
(22) Date of filing: 23.01.1997
(51) Int. Cl.: C12Q 1/68, C07H 21/00, B01D 59/44, G01N 30/72

(54) **Methods for analyzing nucleic acid molecules utilizing sizing techniques**
Methoden zur Analyse von Nukleinsäure-Molekülen mittels Techniken zur Grössen-bestimmung
Procédé servant à analyser des molécules d'acides nucléiques au moyen de techniques de dimensionnement

(30) Priority: 23.01.1996 US 14536 P; 04.06.1996 US 20487 P
(43) Date of publication of application: 08.12.1999
(62) Divisional of application: 97905603.3
(73) Proprietor: Operon Biotechnologies, Inc., Huntsville, AL 35805 (US)
(72) Inventor: Van Ness, Jeffrey, Seattle, WA 98125 (US); Tabone, John C., Bothell, WA 98011 (US); Hobert, J. Jeffrey, Bellevue, WA 98005 (US); Mulligan, John T., Seattle, WA 98105 (US)
(74) Representative: Barton, Matthew Thomas

(56) References cited:
- WO-A-93/20233
- WO-A-94/08051
- WO-A-94/16101
- WO-A-95/04160
- WO-A-95/28640
- US-A- 5 003 059
- US-A- 5 118 605
- US-A- 5 324 631
- US-A- 5 346 670
- US-A- 5 360 819
- US-A- 5 516 931
- ABDEL-BAKY SAMY ET AL: "Detection of electrophore-labeled DNA and albumin by gas chromatography: Labile amide electrophoric release tags" ANALYTICAL CHEMISTRY, vol. 65, no. 4, 1993, pages 498-499, XP002264518 ISSN: 0003-2700
- JACOBSON K B ET AL: "APPLICATIONS OF MASS SPECTROMETRY TO DNA SEQUENCING" GENETIC ANALYSIS TECHNIQUES AND APPLICATIONS, vol. 8, no. 8, 1 December 1991 (1991-12-01), pages 223-229, XP000271820
- EGGERS M ET AL: "A REVIEW OF MICROFABRICATED DEVICES FOR GENE-BASED DIAGNOSTICS" HEMATOLOGIC PATHOLOGY, DEKKER, NEW YORK, NY, US, vol. 9, no. 1, 1995, pages 1-15, XP000856512 ISSN: 0886-0238

## Description

### TECHNICAL FIELD

The present invention relates generally to methods for analyzing nucleic acid molecules, using tags, wherein separation of nucleic acid molecules based on size is required.

### BACKGROUND OF THE INVENTION

Detection and analysis of nucleic acid molecules are among the most important techniques in biology. Such techniques are at the heart of molecular biology and play a rapidly expanding role in the rest of biology.

Generally, one type of analysis of nucleic acid reactions involves separation of nucleic acid molecules based on length. For example, one widely used technique, polymerase chain reaction (PCR) (*see,* U.S. Patent Nos. 4,683,195, 4,683,202, and 4,800,159) has become a widely utilized technique to both identify sequences present in a sample and to synthesize DNA molecules for further manipulation.

Briefly, in PCR, DNA sequences are amplified by enzymatic reaction that synthesizes new DNA strands in either a geometric or linear fashion. Following amplification, the DNA sequences must be detected and identified. Because of non-specific amplifications, which would otherwise confuse analysis, or the need for purity, the PCR reaction products are generally subjected to separation prior to detection. Separation based on the size (*i.e.,* length) of the products yields the most useful information. The method giving the highest resolution of nucleic acid molecules is electrophoretic separation. In this method, each individual PCR reaction is applied to an appropriate gel and subjected to a voltage potential. The number of samples that can be processed is limited by the number of wells in the gel. On most gel apparatus, from approximately 10 to 64 samples can be separated in a single gel. Thus, processing large numbers of samples is both labor and material intensive.

Electrophoretic separation must be coupled with some detection system in order to obtain data. Detection systems of nucleic acids commonly, and almost exclusively, utilize an intercalating dye or radioactive label, and less frequently, a non-radioactive label. Intercalating dyes, such as ethidium bromide, are simple to use. The dye is included in the gel matrix during electrophoresis or, following electrophoresis, the gel is soaked in a dye-containing solution. The dye can be directly visualized in some cases, but more often, and for ethidium bromide in particular, is excited by light (*e.g.,* UV) to fluoresce. In spite of this apparent ease of use, such dyes have some notable disadvantages. First, the dyes are insensitive and there must be a large mass amount of nucleic acid molecules in order to visualize the products. Second, the dyes are typically mutagenic or carcinogenic.

A more sensitive detection technique than dyes uses a radioactive (or nonradioactive) label. Typically, either a radiolabeled nucleotide or a radiolabeled primer is included in the PCR reaction. Following separation, the radiolabel is "visualized" by autoradiography. Although more sensitive, the detection suffers from film limitations, such as reciprocity failure and non-linearity. These limitations can be overcome by detecting the label by phosphor image analysis. However, radiolabels have safety requirements, increasing resource utilization and necessitating specialized equipment and personnel training. For such reasons, the use of nonradioactive labels has been increasing in popularity. In such systems, nucleotides contain a label, such as a fluorophore, biotin or digoxin, which can be detected by an antibody or other molecule (*e.g.,* other member of a ligand pair) that is labeled with an enzyme reactive with a chromogenic substrate. These systems do not have the safety concerns as described above, but use components that are often labile and may yield nonspecific reactions, resulting in high background (*i.e.,* low signal-to-noise ratio).

According to the present invention, there is provided a method for high through-put screening for a polymorphism in multiple individuals or multiple polymorphisms in an individual, comprising: forming a plurality of tagged nucleic acids using tagged PCR primers, where a tag is correlative with a particular Individual of the multiple individuals or a particular polymorphism of the multiple polymorphisms; subjecting the plurality to a separation procedure, where the separation procedure separates tagged nucleic acids based on size; cleaving the tags from the tagged nucleic acids, and detecting the cleaved tags by non-fluorescent spectrometry or potentiometry.

Preferred embodiments of the invention are set out in the appended dependent claims.

WO-A-9 504 160 discloses a tag reagent comprising an analyte moiety having two analyte residues linked to a tag moiety having a reporter group. A library of these reagents is used to sequence nucleic acids.

Jacobson et al. (G.A.T.A., Vol. 8, p.223:29, 1991) discloses a tag reagent comprising an inorganic tag.

WO-A-9 528 640 discloses a tag reagent and assay system wherein relevant nucleotide groups and corresponding reporter groups are added either side of a single tinker.

US-A-5 324 231 discloses a method of detecting RFLP using specific primers labelled with non-radioactive labels, such as chromophores.

The present invention provides novel polymorphium screening methods.

### SUMMARY OF THE INVENTION

According to the present invention, there is provided a method for high through-put screening for a polymorphism in multiple individuals or multiple polymorphisms in an Individual, comprising: forming a plurality of tagged nucleic acids using tagged PCR primers, where a tag **is** correlative with a particular individual of the multiple individuals or a particular polymorphism of the multiple polymorphisms; subjecting the plurality to a separation procedure, where the separation procedure separates tagged nucleic acids based on size; cleaving the tags from the tagged nucleic acids, and detecting the cleaved tags by non-fluorescent spectrometry or potentiometry.

Preferred embodiments of the Invention are set out in the appended dependent claims.

Furthermore, compositions and methods are described which may be utilized in a wide variety of ligand pair reactions wherein separation of molecules of interest, such as nucleic acid molecules, based on size is required. Representative examples of methods which may be enhanced given the disclosure provided herein include PCR, differential display, RNA fingerprinting, PCR-SSCP, oligo litations assays nuclease digestion methods (*e.g.,* exo- and endo- nuclease based assays), and dideoxy fingerprinting. The methods described herein may be utilized in a wide array of fields, including, for example, in the development of clinical or research-based diagnostics, the determination of polymorphisms, and the development of genetic maps.

Described herein are:
1) methods for determining the identity of a nucleic acid molecule, comprising the steps of (a) generating tagged nucleic acid molecules from one or more selected target nucleic acid molecules, wherein a tag is correlative with a particular nucleic acid fragment and detectable by non-fluorescent spectrometry or potentiometry, (b) separating the tagged fragments by size, (c) cleaving the tags from the tagged fragments, and (d) detecting tags by non-fluorescent spectrometry or potentiometry, and therefrom determining the identity of the nucleic acid molecules;
2)methods for detecting a selected nucleic acid molecule, comprising the steps of (a) combining tagged nucleic acid probes with target nucleic acid molecules under conditions and for a time sufficient to permit hybridization of a tagged nucleic acid probe to a complementary selected target nucleic acid sequence, wherein a tagged nucleic acid probe is detectable by non-fluorescent spectrometry or potentiometry, (b) altering the size of hybridized tagged probes, unhybridized probes or target molecules, or the probe:target hybrids, (c) separating the tagged probes by size, (d) cleaving tags from the tagged probes, and (e) detecting the tags by non-fluorescent spectrometry or potentiometry, and therefrom detecting the selected nucleic acid molecule.
3)methods for genotyping a selected organism, comprising the steps of (a) generating tagged nucleic acid molecules from a selected target molecule, wherein a tag is correlative with a particular fragment and may be detected by non-fluorescent spectrometry or potentiometry, (b) separating the tagged molecules by sequential length, (c) cleaving the tag from the tagged molecule, and (d) detecting the tag by non-fluorescent spectrometry or potentiometry, and therefrom determining the genotype of the organism.
4) methods for genotyping a selected organism, comprising the steps of (a) combining a tagged nucleic acid molecule with a selected target molecule under conditions and for a time sufficient to permit hybridization of the tagged molecule to the target molecule, wherein a tag is correlative with a particular fragment and may be detected by non-fluorescent spectrometry or potentiometry, (b) separating the tagged fragments by sequential length, (c) cleaving the tag from the tagged fragment, and (d) detecting the tag by non-fluorescent spectrometry or potentiometry, and therefrom determining the genotype of the organism.

Within the context of the present invention it should be understood that "biological samples" include not only samples obtained from living organisms (*e.g.*, mammals, fish, bacteria, parasites, viruses, fungi and the like) or from the environment (*e.g.,* air, water or solid samples), but biological materials which may be artificially or synthetically produced (*e.g.,* phage libraries, organic molecule libraries, pools of genomic clones, cDNA clones, RNA clones, or the like). Representative examples of biological samples include biological fluids (*e.g.*, blood, semen, cerebral spinal fluid, urine), biological cells (*e.g.,* stem cells, B or T cells, liver cells, fibroblasts and the like), and biological tissues. Finally, representative examples of organisms that may be genotyped include virtually any unicellular or multicellular organism, such as warm-blooded animals, mammals or vertebrates (*e.g.,* humans, chimps, macaques, horses, cows, pigs, sheep, dogs, cats, rats and mice, as well as cells from any of these), bacteria, parasites, viruses, fungi and plants.

Within various embodiments of the above-described methods, the nucleic acid probes and or molecules of the present invention may be generated by, for example, a ligation, cleavage or extension (*e.g.,* PCR) reaction. Within other related aspects the nucleic acid probes or molecules may be tagged by non-3' tagged oligonucleotide primers (*e.g.,* 5'-tagged oligonucleotide primers) or dideoxynucleotide terminators.

It is described that 4, 5, 10, 15, 20, 25, 30, 35, 40, 45, 50, 60 , 70, 80, 90, 100, 200, 250, 300, 350, 400, 450, or greater than 500 different and unique tagged molecules may be utilized within a given reaction simultaneously, wherein each tag is unique for a selected nucleic acid molecule or fragement, or probe, and may be separately identifed.

The tag(s) may be detected by fluorometry, mass spectrometry, infrared spectrometry, ultraviolet spectrometry, or, potentiostatic amperometry (*e.g.,* utilizing coulometric or amperometric detectors). Representative examples of suitable spectrometric techniques include time-of-flight mass spectrometry, quadrupole mass spectrometry, magnetic sector mass spectrometry and electric sector mass spectrometry. Specific embodiments of such techniques include ion-trap mass spectrometry, electrospray ionization mass spectrometry, ion-spray mass spectrometry, liquid ionization mass spectrometry, atmospheric pressure ionization mass spectrometry, electron ionization mass spectrometry, fast atom bombard ionization mass spectrometry, MALDI mass spectrometry, photo-ionization time-of-flight mass spectrometry, laser droplet mass spectrometry, MALDI-TOF mass spectrometry, APCI mass spectrometry, nano-spray mass spectrometry, nebulised spray ionization mass spectrometry, chemical ionization mass spectrometry, resonance ionization mass spectrometry, secondary ionization mass spectrometry and thermospray mass spectrometry.

The target molecules, hybridized tagged probes, unhybridized probes or target molecules, probe:target hybrids, or tagged nucleic acid probes or molecules are separated from other molecules utilizing methods which discriminate between the size of molecules (either actual linear size, or three-dimensional size). Representative examples of such methods include gel electrophoresis, capillary electrophoresis, micro-channel electrophoresis, HPLC, size exclusion chromatography, filtration, polyacrylamide gel electrophoresis, liquid chromatography, reverse size exclusion chromatography, ion-exchange chromatography, reverse phase liquid chromatography, pulsed-field electrophoresis, field-inversion electrophoresis, dialysis, and fluorescence-activated liquid droplet sorting. Alternatively, the target molecules, hybridized tagged probes, unhybridized probes or target molecules, probe:target hybrids, or tagged nucleic acid probes or molecules may be bound to a solid support (*e.g.,* hollow fibers (Amicon Corporation, Danvers, Mass.), beads (Polysciences, Warrington, Pa.), magnetic beads (Robbin Scientific, Mountain View, Calif.), plates, dishes and flasks (Corning Glass Works, Corning, N.Y.), meshes (Becton Dickinson, Mountain View, Calif.), screens and solid fibers (see Edelman et al., U.S. Patent No. 3,843,324; see also Kuroda etyal., U.S. Patent No. 4,416,777), membranes (Millipore Corp., Bedford, Mass.), and dipsticks). If the first or second member, or exposed nucleic acids are bound to a solid support, within certain embodiments of the invention the methods disclosed herein may further comprise the step of washing the solid support of unbound material.

The tagged nucleic acid molecules or probes may be cleaved by a methods such as chemical, oxidation, reduction, acid-labile, base labile, enzymatic, electrochemical, heat and photolabile methods. Within further embodiments, the steps of separating, cleaving and detecting may be performed in a continuous manner, for example, on a single device which may be automated.

It is described that the size of the hybridized tagged probes, unhybridized probes or target molecules, or probe:target hybrids are altered by a method selected from the group consisting of polymerase extension, ligation, exonuclease digestion, endonuclease digestion, restriction enzyme digestion, site-specific recombinase digestion, ligation, mismatch specific nuclease digestion, methylation-specific nuclease digestion, covalent attachment of probe to target and hybridization.

The methods an compositions described herein may be utilized in a wide variety of applications, including for example, identifying PCR amplicons, RNA fingerprinting, differential display, single-strand conformation polymorphism detection, dideoxyfingerprinting, restriction maps and restriction fragment length polymorphisms, DNA fingerprinting, genotyping, mutation detection, oligonucleotide ligation assay, sequence specific amplifications, for diagnostics, forensics, identification, developmental biology, biology, molecular medicine, toxicology, animal breeding,

These and other aspects of the present invention will become evident upon reference to the following detailed description and attached drawings.

### BRIEF DESCRIPTION OF THE DRAWINGS

Figure 1 depicts the flowchart for the synthesis of pentafluorophenyl esters of chemically cleavable mass spectroscopy tags, to liberate tags with carboxyl amide termini.
Figure 2 depicts the flowchart for the synthesis of pentafluorophenyl esters of chemically cleavable mass spectroscopy tags, to liberate tags with carboxyl acid termini.
Figures 3-6 and 8 depict the flowchart for the synthesis of tetrafluorophenyl esters of a set of 36 photochemically cleavable mass spectroscopy tags.
Figure 7 depicts the flowchart for the synthesis of a set of 36 amine-terminated photochemically cleavable mass spectroscopy tags.
Figure 9 depicts the synthesis of 36 photochemically cleavable mass spectroscopy tagged oligonucleotides made from the corresponding set of 36 tetrafluorophenyl esters of photochemically cleavable mass spectroscopy tag acids.
Figure 10 depicts the synthesis of 36 photochemically cleavable mass spectroscopy tagged oligonucleotides made from the corresponding set of 36 amine-terminated photochemically cleavable mass spectroscopy tags.
Figure 11 illustrates the simultaneous detection of multiple tags by mass spectrometry.
Figure 12 shows the mass spectrogram of the alpha-cyano matrix alone.

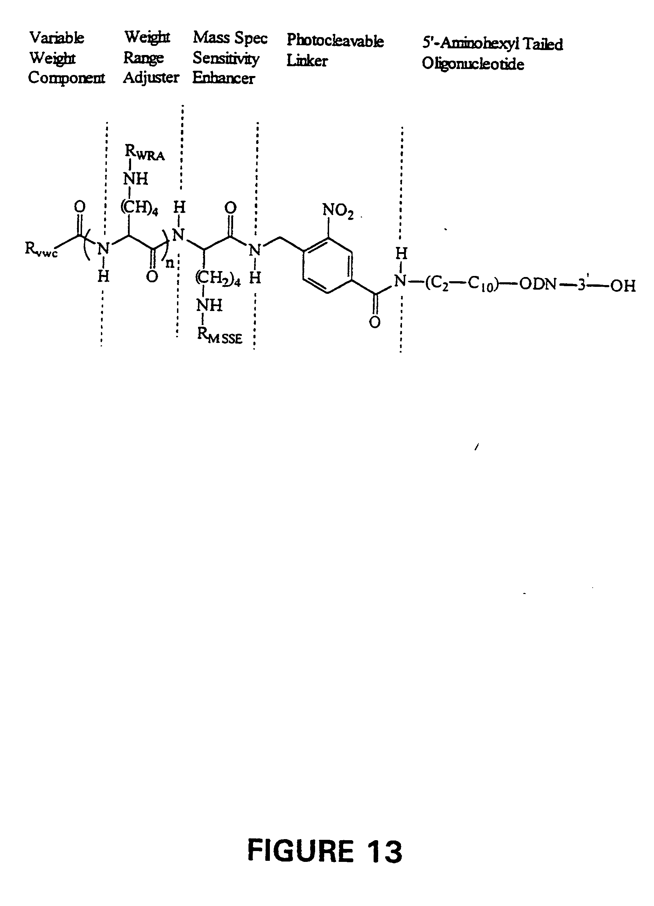

Figure 13 depicts a modularly-constructed tagged nucleic acid fragment.

### DETAILED DESCRIPTION OF THE INVENTION

As noted above, herein described are compositions and methods for analyzing nucleic acid molecules, wherein separation of nucleic acid molecules based on size is required. The present methods permit the simultaneous detection of molecules of interest, which include nucleic acids and fragments, proteins, peptides, etc.

Briefly stated herein described are compounds wherein a molecule of interest, or precursor thereto, is linked via a labile bond (or labile bonds) to a tag. Thus, these compounds may be viewed as having the general formula:

T-L-X

wherein T is the tag component, L is the linker component that either is, or contains, a labile bond, and X is either the molecule of interest (MOI) component or a functional group component (Lₕ) through which the MOI may be joined to T-L. Compounds of the invention may therefore be represented by the more specific general formulas:

T-L-MOI and T-L-Lₕ

For reasons described in detail below, sets of T-L-MOI compounds may be purposely subjected to conditions that cause the labile bond(s) to break, thus releasing a tag moiety from the remainder of the compound. The tag moiety is then characterized by one or more analytical techniques, to thereby provide direct information about the structure of the tag moiety, and (most importantly) indirect information about the identity of the corresponding MOI.

As a simple illustrative example of a representative compound wherein L is a direct bond, reference is made to the following structure (i):

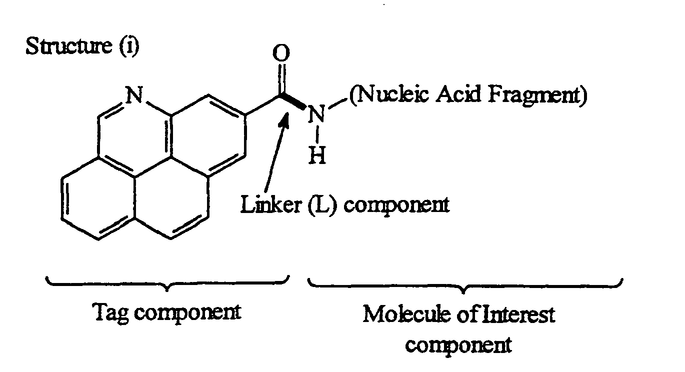

In structure (i), T is a nitrogen-containing polycyclic aromatic moiety bonded to a carbonyl group, X is a MOI (and specifically a nucleic acid fragment terminating in an amine group), and L is the bond which forms an amide group. The amide bond is labile relative to the bonds in T because, as recognized in the art, an amide bond may be chemically cleaved (broken) by acid or base conditions which leave the bonds within the tag component unchanged. Thus, a tag moiety (*i.e.,* the cleavage product that contains T) may be released as shown below:

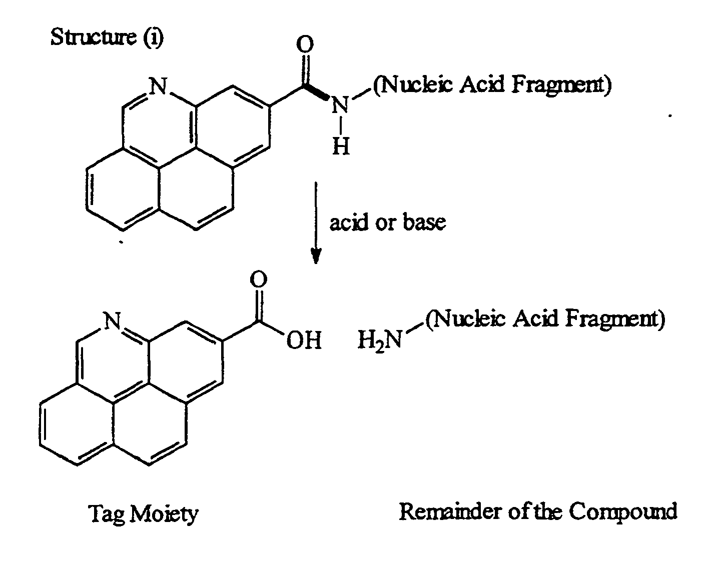

However, the linker L may be more than merely a direct bond, as shown in the following illustrative example, where reference is made to another representative compound of the invention having the structure (ii) shown below:

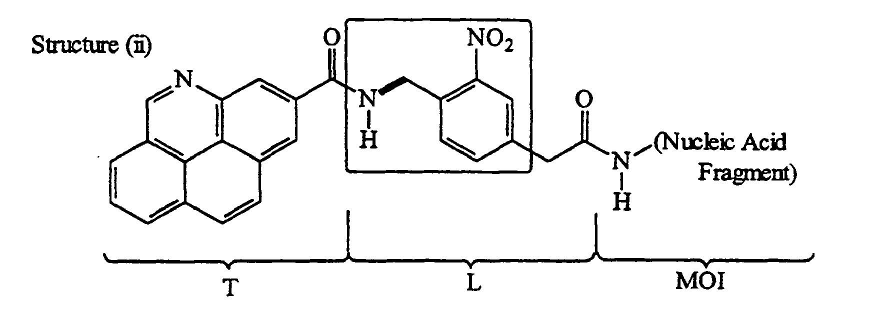

It is well-known that compounds having an *ortho*-nitrobenzylamine moiety (*see* boxed atoms within structure (ii)) are photolytically unstable, in that exposure of such compounds to actinic radiation of a specified wavelength will cause selective cleavage of the benzylamine bond (*see* bond denoted with heavy line in structure (ii)). Thus, structure (ii) has the same T and MOI groups as structure (i), however the linker group contains multiple atoms and bonds within which there is a particularly labile bond. Photolysis of structure (ii) thus releases a tag moiety (T-containing moiety) from the remainder of the compound, as shown below.

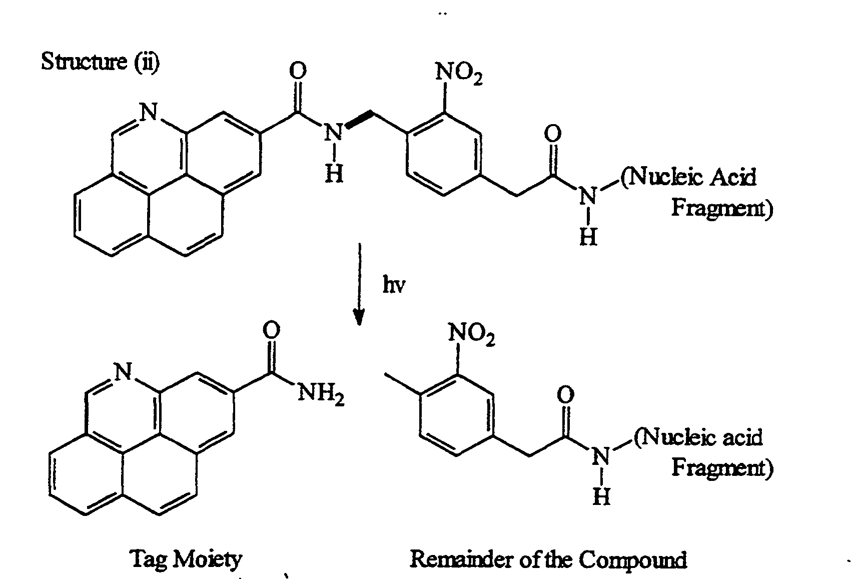

The described herein are thus compounds which, upon exposure to appropriate cleavage conditions, undergo a cleavage reaction so as to release a tag moiety from the remainder of the compound. Compounds of the invention may be described in terms of the tag moiety, the MOI (or precursor thereto, Lₕ), and the labile bond(s) which join the two groups together. Alternatively, the compounds of the invention may be described in terms of the components from which they are formed. Thus, the compounds may be described as the reaction product of a tag reactant, a linker reactant and a MOI reactant, as follows.

The tag reactant consists of a chemical handle (Tₕ) and a variable component (T_{vc}), so that the tag reactant is seen to have the general structure:

T_{vc}-Tₕ

To illustrate this nomenclature, reference may be made to structure (iii), which shows a tag reactant that may be used to prepare the compound of structure (ii). The tag reactant having structure (iii) contains a tag variable component and a tag handle, as shown below:

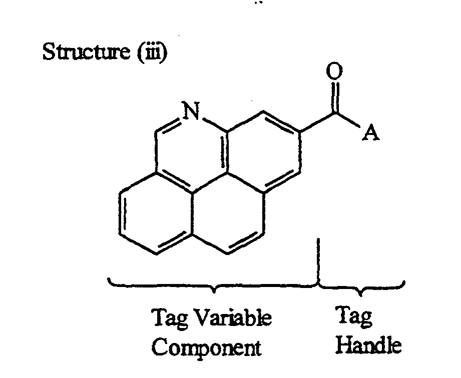

In structure (iii), the tag handle (-C(=O)-A) simply provides an avenue for reacting the tag reactant with the linker reactant to form a T-L moiety. The group "A" in structure (iii) indicates that the carboxyl group is in a chemically active state, so it is ready for coupling with other handles. "A" may be, for example, a hydroxyl group or pentafluorophenoxy, among many other possibilities. The invention provides for a large number of possible tag handles which may be bonded to a tag variable component, as discussed in detail below. The tag variable component is thus a part of "T" in the formula T-L-X, and will also be part of the tag moiety that forms from the reaction that cleaves L.

As also discussed in detail below, the tag variable component is so-named because, in preparing sets of compounds according to the present description, it is desired that members of a set have unique variable components, so that the individual members may be distinguished from one another by an analytical technique. As one example, the tag variable component of structure (iii) may be one member of the following set, where members of the set may be distinguished by their UV or mass spectra:

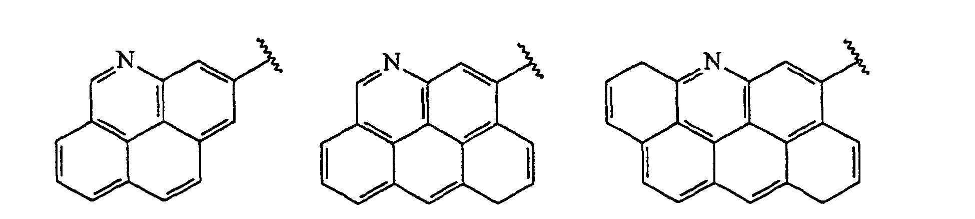

Likewise, the linker reactant may be described in terms of its chemical handles (there are necessarily at least two, each of which may be designated as Lₕ) which flank a linker labile component, where the linker labile component consists of the required labile moiety (L²) and optional labile moieties (L¹ and L³), where the optional labile moieties effectively serve to separate L² from the handles Lₕ, and the required labile moiety serves to provide a labile bond within the linker labile component. Thus, the linker reactant may be seen to have the general formula:

Lₕ-L¹-L²-L³-Lₕ

The nomenclature used to describe the linker reactant may be illustrated in view of structure (iv), which again draws from the compound of structure (ii):

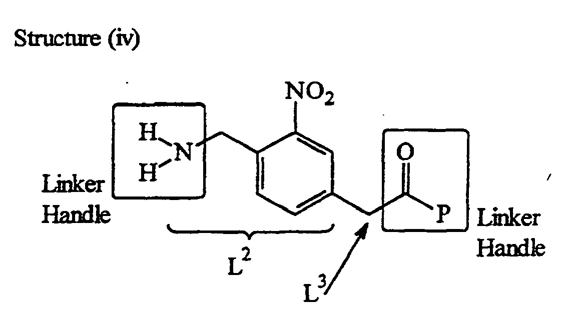

As structure (iv) illustrates, atoms may serve in more than one functional role. Thus, in structure (iv), the benzyl nitrogen functions as a chemical handle in allowing the linker reactant to join to the tag reactant via an amide-forming reaction, and subsequently also serves as a necessary part of the structure of the labile moiety L² in that the benzylic carbon-nitrogen bond is particularly susceptible to photolytic cleavage. Structure (iv) also illustrates that a linker reactant may have an L³ group (in this case, a methylene group), although not have an L¹ group. Likewise, linker reactants may have an L¹ group but not an L³ group, or may have L¹ and L³ groups, or may have neither of L¹ nor L³ groups. In structure (iv), the presence of the group "P" next to the carbonyl group indicates that the carbonyl group is protected from reaction. Given this configuration, the activated carboxyl group of the tag reactant (iii) may cleanly react with the amine group of the linker reactant (iv) to form an amide bond and give a compound of the formula T-L-Lₕ.

The MOI reactant is a suitably reactive form of a molecule of interest. Where the molecule of interest is a nucleic acid fragment, a suitable MOI reactant is a nucleic acid fragment bonded through its 5' hydroxyl group to a phosphodiester group and then to an alkylene chain that terminates in an amino group. This amino group may then react with the carbonyl group of structure (iv), (after, of course, deprotecting the carbonyl group, and preferably after subsequently activating the carbonyl group toward reaction with the amine group) to thereby join the MOI to the linker.

When viewed in a chronological order, the invention is seen to take a tag reactant (having a chemical tag handle and a tag variable component), a linker reactant (having two chemical linker handles, a required labile moiety and 0-2 optional labile moieties) and a MOI reactant (having a molecule of interest component and a chemical molecule of interest handle) to form T-L-MOI. Thus, to form T-L-MOI, either the tag reactant and the linker reactant are first reacted together to provide T-L-Lₕ, and then the MOI reactant is reacted with T-L-Lₕ so as to provide T-L-MOI, or else (less preferably) the linker reactant and the MOI reactant are reacted together first to provide Lₕ-L-MOI, and then Lₕ-L-MOI is reacted with the tag reactant to provide T-L-MOI. For purposes of convenience, compounds having the formula T-L-MOI will be described in terms of the tag reactant, the linker reactant and the MOI reactant which may be used to form such compounds. Of course, the same compounds of formula T-L-MOI could be prepared by other (typically, more laborious) methods, and still fall within the scope of the inventive T-L-MOI compounds.

In any event, the invention provides that a T-L-MOI compound be subjected to cleavage conditions, such that a tag moiety is released from the remainder of the compound. The tag moiety will comprise at least the tag variable component, and will typically additionally comprise some or all of the atoms from the tag handle, some or all of the atoms from the linker handle that was used to join the tag reactant to the linker reactant, the optional labile moiety L¹ if this group was present in T-L-MOI, and will perhaps contain some part of the required labile moiety L² depending on the precise structure of L² and the nature of the cleavage chemistry. For convenience, the tag moiety may be referred to as the T-containing moiety because T will typically constitute the major portion (in terms of mass) of the tag moiety.

The various components T, L and X will be described in detail. This description begins with the following definitions of certain terms, which will be used hereinafter in describing T, L and X.

As used herein, the term "nucleic acid fragment" means a molecule which is complementary to a selected target nucleic acid molecule (*i.e.,* complementary to all or a portion thereof), and may be derived from nature or synthetically or recombinantly produced, including non-naturally occurring molecules, and may be in double or single stranded form where appropriate; and includes an oligonucleotide (*e.g.*, DNA or RNA), a primer, a probe, a nucleic acid analog (*e.g.,* PNA), an oligonucleotide which is extended in a 5' to 3' direction by a polymerase, a nucleic acid which is cleaved chemically or enzymatically, a nucleic acid that is terminated with a dideoxy terminator or capped at the 3' or 5' end with a compound that prevents polymerization at the 5' or 3' end, and combinations thereof. The complementarity of a nucleic acid fragment to a selected target nucleic acid molecule generally means the exhibition of at least about 70% specific base pairing throughout the length of the fragment. Preferably the nucleic acid fragment exhibits at least about 80% specific base pairing; and most preferably at least about 90%. Assays for determining the percent mismatch (and thus the percent specific base pairing) are well known in the art and are based upon the percent mismatch as a function of the Tm when referenced to the fully base paired control.

As used herein, the term "alkyl," alone or in combination, refers to a saturated, straight-chain or branched-chain hydrocarbon radical containing from 1 to 10, preferably from 1 to 6 and more preferably from 1 to 4, carbon atoms. Examples of such radicals include, but are not limited to, methyl, ethyl, n-propyl, iso-propyl, n-butyl, iso-butyl, sec-butyl, tert-butyl, pentyl, iso-amyl, hexyl, decyl and the like. The term "alkylene" refers to a saturated, straight-chain or branched chain hydrocarbon diradical containing from 1 to 10, preferably from 1 to 6 and more preferably from 1 to 4, carbon atoms. Examples of such diradicals include, but are not limited to, methylene, ethylene (-CH₂-CH₂-), propylene, and the like.

The term "alkenyl," alone or in combination, refers to a straight-chain or branched-chain hydrocarbon radical having at least one carbon-carbon double bond in a total of from 2 to 10, preferably from 2 to 6 and more preferably from 2 to 4, carbon atoms. Examples of such radicals include, but are not limited to, ethenyl, E- and Z-propenyl, isopropenyl, E- and Z-butenyl, E- and Z-isobutenyl, E- and Z-pentenyl, decenyl and the like. The term "alkenylene" refers to a straight-chain or branched-chain hydrocarbon diradical having at least one carbon-carbon double bond in a total of from 2 to 10, preferably from 2 to 6 and more preferably from 2 to 4, carbon atoms. Examples of such diradicals include, but are not limited to, methylidene (=CH₂), ethylidene (-CH=CH-), propylidene (-CH₂-CH=CH-) and the like.

The term "alkynyl," alone or in combination, refers to a straight-chain or branched-chain hydrocarbon radical having at least one carbon-carbon triple bond in a total of from 2 to 10, preferably from 2 to 6 and more preferably from 2 to 4, carbon atoms. Examples of such radicals include, but are not limited to, ethynyl (acetylenyl), propynyl (propargyl), butynyl, hexynyl, decynyl and the like. The term "alkynylene", alone or in combination, refers to a straight-chain or branched-chain hydrocarbon diradical having at least one carbon-carbon triple bond in a total of from 2 to 10, preferably from 2 to 6 and more preferably from 2 to 4, carbon atoms. Examples of such radicals include, but are not limited, ethynylene (-C≡C-), propynylene (-CH₂-C≡C-) and the like.

The term "cycloalkyl," alone or in combination, refers to a saturated, cyclic arrangement of carbon atoms which number from 3 to 8 and preferably from 3 to 6, carbon atoms. Examples of such cycloalkyl radicals include, but are not limited to, cyclopropyl, cyclobutyl, cyclopentyl, cyclohexyl and the like. The term "cycloalkylene" refers to a diradical form of a cycloalkyl.

The term "cycloalkenyl," alone or in combination, refers to a cyclic carbocycle containing from 4 to 8, preferably 5 or 6, carbon atoms and one or more double bonds. Examples of such cycloalkenyl radicals include, but are not limited to, cyclopentenyl, cyclohexenyl, cyclopentadienyl and the like. The term "cycloalkenylene" refers to a diradical form of a cycloalkenyl.

The term "aryl" refers to a carbocyclic (consisting entirely of carbon and hydrogen) aromatic group selected from the group consisting of phenyl, naphthyl, indenyl, indanyl, azulenyl, fluorenyl, and anthracenyl; or a heterocyclic aromatic group selected from the group consisting of furyl, thienyl, pyridyl, pyrrolyl, oxazolyly, thiazolyl, imidazolyl, pyrazolyl, 2-pyrazolinyl, pyrazolidinyl, isoxazolyl, isothiazolyl, 1, 2, 3-oxadiazolyl, 1, 2, 3-triazolyl, 1, 3, 4-thiadiazolyl, pyridazinyl, pyrimidinyl, pyrazinyl, 1, 3, 5-triazinyl, 1, 3, 5-trithianyl, indolizinyl, indolyl, isoindolyl, 3H-indolyl, indolinyl, benzo[b]furanyl, 2, 3-dihydrobenzofuranyl, benzo[b]thiophenyl, 1H-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1, 8-naphthyridinyl, pteridinyl, carbazolyl, acridinyl, phenazinyl, phenothiazinyl, and phenoxazinyl.

"Aryl" groups, as defined in this application may independently contain one to four substituents which are independently selected from the group consisting of hydrogen, halogen, hydroxyl, amino, nitro, trifluoromethyl, trifluoromethoxy, alkyl, alkenyl, alkynyl, cyano, carboxy, carboalkoxy, 1,2-dioxyethylene, alkoxy, alkenoxy or alkynoxy, alkylamino, alkenylamino, alkynylamino, aliphatic or aromatic acyl, alkoxy-carbonylamino, alkylsulfonylamino, morpholinocarbonylamino, thiomorpholinocarbonylamino, N-alkyl guanidino, aralkylaminosulfonyl; aralkoxyalkyl; N-aralkoxyurea; N-hydroxylurea; N-alkenylurea; N,N-(alkyl, hydroxyl)urea; heterocyclyl; thioaryloxy-substituted aryl; N,N-(aryl, alkyl)hydrazino; Ar'-substituted sulfonylheterocyclyl; aralkyl-substituted heterocyclyl; cycloalkyl and cycloakenyl-substituted heterocyclyl; cycloalkyl-fused aryl; aryloxy-substituted alkyl; heterocyclylamino; aliphatic or aromatic acylaminocarbonyl; aliphatic or aromatic acyl-substituted alkenyl; Ar'-substituted aminocarbonyloxy; Ar', Ar'-disubstituted aryl; aliphatic or aromatic acyl-substituted acyl; cycloalkylcarbonylalkyl; cycloalkyl-substituted amino; aryloxycarbonylalkyl; phosphorodiamidyl acid or ester;

"Ar"' is a carbocyclic or heterocyclic aryl group as defined above having one to three substituents selected from the group consisting of hydrogen, halogen, hydroxyl, amino, nitro, trifluoromethyl, trifluoromethoxy, alkyl, alkenyl, alkynyl, 1,2-dioxymethylene, 1,2-dioxyethylene, alkoxy, alkenoxy, alkynoxy, alkylamino, alkenylamino or alkynylamino, alkylcarbonyloxy, aliphatic or aromatic acyl, alkylcarbonylamino, alkoxycarbonylamino, alkylsulfonylamino, N-alkyl or N,N-dialkyl urea.

The term "alkoxy," alone or in combination, refers to an alkyl ether radical, wherein the term "alkyl" is as defined above. Examples of suitable alkyl ether radicals include, but are not limited to, methoxy, ethoxy, n-propoxy, iso-propoxy, n-butoxy, iso-butoxy, sec-butoxy, tert-butoxy and the like.

The term "alkenoxy," alone or in combination, refers to a radical of formula alkenyl-O-, wherein the term "alkenyl" is as defined above provided that the radical is not an enol ether. Examples of suitable alkenoxy radicals include, but are not limited to, allyloxy, E- and Z-3-methyl-2-propenoxy and the like.

The term "alkynyloxy," alone or in combination, refers to a radical of formula alkynyl-O-, wherein the term "alkynyl" is as defined above provided that the radical is not an ynol ether. Examples of suitable alkynoxy radicals include, but are not limited to, propargyloxy, 2-butynyloxy and the like.

The term "thioalkoxy" refers to a thioether radical of formula alkyl-S-, wherein alkyl is as defined above.

The term "alkylamino," alone or in combination, refers to a mono- or di-alkyl-substituted amino radical (*i.e.,* a radical of formula alkyl-NH- or (alkyl)₂-N-), wherein the term "alkyl" is as defined above. Examples of suitable alkylamino radicals include, but are not limited to, methylamino, ethylamino, propylamino, isopropylamino, t-butylamino, N,N-diethylamino and the like.

The term "alkenylamino," alone or in combination, refers to a radical of formula alkenyl-NH- or (alkenyl)₂N-, wherein the term "alkenyl" is as defined above, provided that the radical is not an enamine. An example of such alkenylamino radicals is the allylamino radical.

The term "alkynylamino," alone or in combination, refers to a radical of formula alkynyl-NH- or (alkynyl)₂N-, wherein the term "alkynyl" is as defined above, provided that the radical is not an ynamine. An example of such alkynylamino radicals is the propargyl amino radical.

The term "amide" refers to either -N(R¹)-C(=O)- or -C(=O)-N(R¹)-where R¹ is defined herein to include hydrogen as well as other groups. The term "substituted amide" refers to the situation where R¹ is not hydrogen, while the term "unsubstituted amide" refers to the situation where R¹ is hydrogen.

The term "aryloxy," alone or in combination, refers to a radical of formula aryl-O-, wherein aryl is as defined above. Examples of aryloxy radicals include, but are not limited to, phenoxy, naphthoxy, pyridyloxy and the like.

The term "arylamino," alone or in combination, refers to a radical of formula aryl-NH-, wherein aryl is as defined above. Examples of arylamino radicals include, but are not limited to, phenylamino (anilido), naphthylamino, 2-, 3- and 4-pyridylamino and the like.

The term "aryl-fused cycloalkyl," alone or in combination, refers to a cycloalkyl radical which shares two adjacent atoms with an aryl radical, wherein the terms "cycloalkyl" and "aryl" are as defined above. An example of an aryl-fused cycloalkyl radical is the benzofused cyclobutyl radical.

The term "alkylcarbonylamino," alone or in combination, refers to a radical of formula alkyl-CONH, wherein the term "alkyl" is as defined above.

The term "alkoxycarbonylamino," alone or in combination, refers to a radical of formula alkyl-OCONH-, wherein -the term "alkyl" is as defined above.

The term "alkylsulfonylamino," alone or in combination, refers to a radical of formula alkyl-SO₂NH-, wherein the term "alkyl" is as defined above.

The term "arylsulfonylamino," alone or in combination, refers to a radical of formula aryl-SO₂NH-, wherein the term "aryl" is as defined above.

The term "N-alkylurea," alone or in combination, refers to a radical of formula alkyl-NH-CO-NH-, wherein the term "alkyl" is as defined above.

The term "N-arylurea," alone or in combination, refers to a radical of formula aryl-NH-CO-NH-, wherein the term "aryl" is as defined above.

The term "halogen" means fluorine, chlorine, bromine and iodine.

The term "hydrocarbon radical" refers to an arrangement of carbon and hydrogen atoms which need only a single hydrogen atom to be an independent stable molecule. Thus, a hydrocarbon radical has one open valence site on a carbon atom, through which the hydrocarbon radical may be bonded to other atom(s). Alkyl, alkenyl, cycloalkyl, etc. are examples of hydrocarbon radicals.

The term "hydrocarbon diradical" refers to an arrangement of carbon and hydrogen atoms which need two hydrogen atoms in order to be an independent stable molecule. Thus, a hydrocarbon radical has two open valence sites on one or two carbon atoms, through which the hydrocarbon radical may be bonded to other atom(s). Alkylene, alkenylene, alkynylene, cycloalkylene, etc. are examples of hydrocarbon diradicals.

The term "hydrocarbyl" refers to any stable arrangement consisting entirely of carbon and hydrogen having a single valence site to which it is bonded to another moiety, and thus includes radicals known as alkyl, alkenyl, alkynyl, cycloalkyl, cycloalkenyl, aryl (without heteroatom incorporation into the aryl ring), arylalkyl, alkylaryl and the like. Hydrocarbon radical is another name for hydrocarbyl.

The term "hydrocarbylene" refers to any stable arrangement consisting entirely of carbon and hydrogen having two valence sites to which it is bonded to other moieties, and thus includes alkylene, alkenylene, alkynylene, cycloalkylene, cycloalkenylene, arylene (without heteroatom incorporation into the arylene ring), arylalkylene, alkylarylene and the like. Hydrocarbon diradical is another name for hydrocarbylene.

The term "hydrocarbyl-O-hydrocarbylene" refers to a hydrocarbyl group bonded to an oxygen atom, where the oxygen atom is likewise bonded to a hydrocarbylene group at one of the two valence sites at which the hydrocarbylene group is bonded to other moieties. The terms "hydrocarbyl-S-hydrocarbylene", "hydrocarbyl-NH-hydrocarbylene" and "hydrocarbyl-amide-hydrocarbylene" have equivalent meanings, where oxygen has been replaced with sulfur, -NH- or an amide group, respectively.

The term N-(hydrocarbyl)hydrocarbylene refers to a hydrocarbylene group wherein one of the two valence sites is bonded to a nitrogen atom, and that nitrogen atom is simultaneously bonded to a hydrogen and a hydrocarbyl group. The term N,N-di(hydrocarbyl)hydrocarbylene refers to a hydrocarbylene group wherein one of the two valence sites is bonded to a nitrogen atom, and that nitrogen atom is simultaneously bonded to two hydrocarbyl groups.

The term "hydrocarbylacyl-hydrocarbylene" refers to a hydrocarbyl group bonded through an acyl (-C(=O)-) group to one of the two valence sites of a hydrocarbylene group.

The terms "heterocyclylhydrocarbyl" and "heterocylyl" refer to a stable, cyclic arrangement of atoms which include carbon atoms and up to four atoms (referred to as heteroatoms) selected from oxygen, nitrogen, phosphorus and sulfur. The cyclic arrangement may be in the form of a monocyclic ring of 3-7 atoms, or a bicyclic ring of 8-11 atoms. The rings may be saturated or unsaturated (including aromatic rings), and may optionally be benzofused. Nitrogen and sulfur atoms in the ring may be in any oxidized form, including the quaternized form of nitrogen. A heterocyclylhydrocarbyl may be attached at any endocyclic carbon or heteroatom which results in the creation of a stable structure. Preferred heterocyclylhydrocarbyls include 5-7 membered monocyclic heterocycles containing one or two nitrogen heteroatoms.

A substituted heterocyclylhydrocarbyl refers to a heterocyclylhydrocarbyl as defined above, wherein at least one ring atom thereof is bonded to an indicated substituent which extends off of the ring.

In referring to hydrocarbyl and hydrocarbylene groups, the term "derivatives of any of the foregoing wherein one or more hydrogens is replaced with an equal number of fluorides" refers to molecules that contain carbon, hydrogen and fluoride atoms, but no other atoms.

The term "activated ester" is an ester that contains a "leaving group" which is readily displaceable by a nucleophile, such as an amine, and alcohol or a thiol nucleophile. Such leaving groups are well known and include, without limitation, N-hydroxysuccinimide, N-hydroxybenzotriazole, halogen (halides), alkoxy including tetrafluorophenolates, thioalkoxy and the like. The term "protected ester" refers to an ester group that is masked or otherwise unreactive. See, e.g., Greene, "Protecting Groups In Organic Synthesis."

In view of the above definitions, other chemical terms used throughout this application can be easily understood by those of skill in the art. Terms may be used alone or in any combination thereof. The preferred and more preferred chain lengths of the radicals apply to all such combinations.

### A. GENERATION OF TAGGED NUCLEIC ACID FRAGMENTS

As noted above, one aspect of the present invention provides a general scheme for DNA sequencing which allows the use of more than 16 tags in each lane; with continuous detection, the tags can be detected and the sequence read as the size separation is occurring, just as with conventional fluorescence-based sequencing. This scheme is applicable to any of the DNA sequencing techniques based on size separation of tagged molecules. Suitable tags and linkers for use within the present invention, as well as methods for sequencing nucleic acids, are discussed in more detail below.

### 1. Tags

"Tag", as used herein, generally refers to a chemical moiety which is used to uniquely identify a "molecule of interest", and more specifically refers to the tag variable component as well as whatever may be bonded most closely to it in any of the tag reactant, tag component and tag moiety.

A tag which is useful in the present invention possesses several attributes:
1) It is capable of being distinguished from all other tags. This discrimination from other chemical moieties can be based on the chromatographic behavior of the tag (particularly after the cleavage reaction), its spectroscopic or potentiometric properties, or some combination thereof. Spectroscopic methods by which tags are usefully distinguished include mass spectroscopy (MS), infrared (IR), ultraviolet (UV), and fluorescence, where MS, IR and UV are preferred, and MS most preferred spectroscopic methods. Potentiometric amperometry is a preferred potentiometric method.
2) The tag is capable of being detected when present at 10⁻²² to 10⁻⁶ mole.
3) The tag possesses a chemical handle through which it can be attached to the MOI which the tag is intended to uniquely identify. The attachment may be made directly to the MOI, or indirectly through a "linker" group.
4) The tag is chemically stable toward all manipulations to which it is subjected, including attachment and cleavage from the MOI, and any manipulations of the MOI while the tag is attached to it.
5) The tag does not significantly interfere with the manipulations performed on the MOI while the tag is attached to it. For instance, if the tag is attached to an oligonucleotide, the tag must not significantly interfere with any hybridization or enzymatic reactions (*e.g.,* PCR sequencing reactions) performed on the oligonucleotide. Similarly, if the tag is attached to an antibody, it must not significantly interfere with antigen recognition by the antibody.

A tag moiety which is intended to be detected by a certain spectroscopic or potentiometric method should possess properties which enhance the sensitivity and specificity of detection by that method. Typically, the tag moiety will have those properties because they have been designed into the tag variable component, which will typically constitute the major portion of the tag moiety. In the following discussion, the use of the word "tag" typically refers to the tag moiety (*i.e.,* the cleavage product that contains the tag variable component), however can also be considered to refer to the tag variable component itself because that is the portion of the tag moiety which is typically responsible for providing the uniquely detectable properties. In compounds of the formula T-L-X, the "T" portion will contain the tag variable component. Where the tag variable component has been designed to be characterized by, *e.g.,* mass spectrometry, the "T" portion of T-L-X may be referred to as T^{ms}. Likewise, the cleavage product from T-L-X that contains T may be referred to as the T^{ms}-containing moiety. The following spectroscopic and potentiometric methods may be used to characterize T^{ms}-containing moieties.

### a. Characteristics of MS Tags

Where a tag is analyzable by mass spectrometry (*i.e.,* is a MS-readable tag, also referred to herein as a MS tag or "T^{ms}-containing moiety"), the essential feature of the tag is that it is able to be ionized. It is thus a preferred element in the design of MS-readable tags to incorporate therein a chemical functionality which can carry a positive or negative charge under conditions of ionization in the MS. This feature confers improved efficiency of ion formation and greater overall sensitivity of detection, particularly in electrospray ionization. The chemical functionality that supports an ionized charge may derive from T^{ms} or L or both. Factors that can increase the relative sensitivity of an analyte being detected by mass spectrometry are discussed in, *e.g.,* Sunner, J., et al., Anal. Chem. 60:1300-1307 (1988).

A preferred functionality to facilitate the carrying of a negative charge is an organic acid, such as phenolic hydroxyl, carboxylic acid, phosphonate, phosphate, tetrazole, sulfonyl urea, perfluoro alcohol and sulfonic acid.

Preferred functionality to facilitate the carrying of a positive charge under ionization conditions are aliphatic or aromatic amines. Examples of amine functional groups which give enhanced detectability of MS tags include quaternary amines (*i.e.,* amines that have four bonds, each to carbon atoms, *see* Aebersold, U.S. Patent No. 5,240,859) and tertiary amines (*i.e.,* amines that have three bonds, each to carbon atoms, which includes C=N-C groups such as are present in pyridine, see Hess et al., Anal. Biochem. 224:373, 1995; Bures et al., Anal. Biochem. 224:364, 1995). Hindered tertiary amines are particularly preferred. Tertiary and quaternary amines may be alkyl or aryl. A T^{ms}-containing moiety must bear at least one ionizable species, but may possess more than one ionizable species. The preferred charge state is a single ionized species per tag. Accordingly, it is preferred that each T-containing moiety (and each tag variable component) contain only a single hindered amine or organic acid group.

Suitable amine-containing radicals that may form part of the T^{ms}-containing moiety include the following:

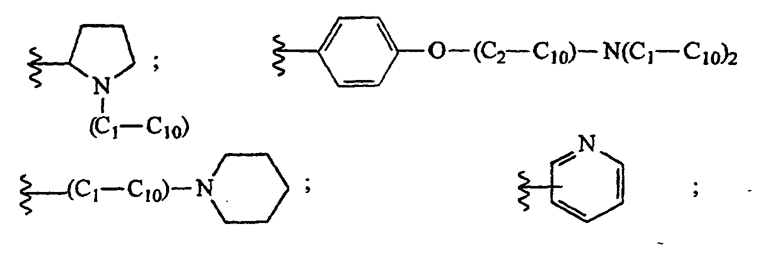

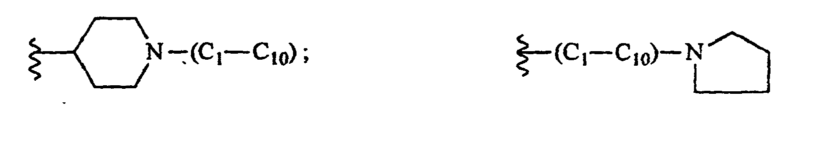

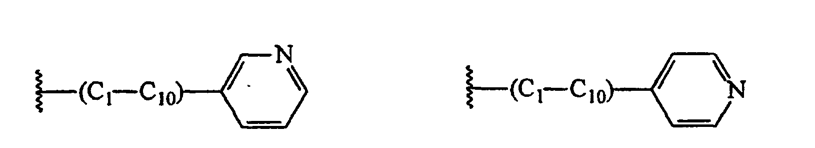

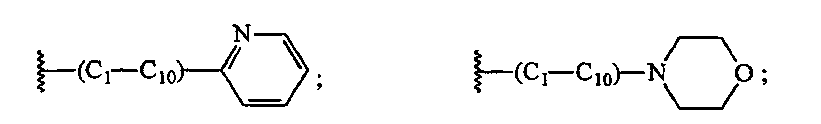

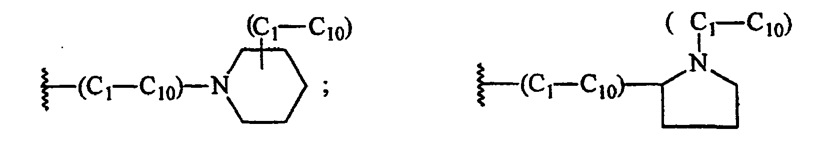

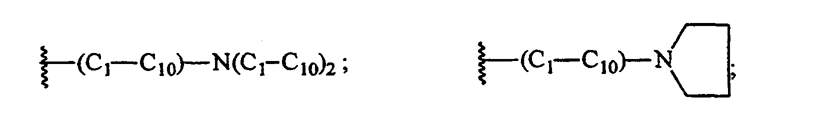

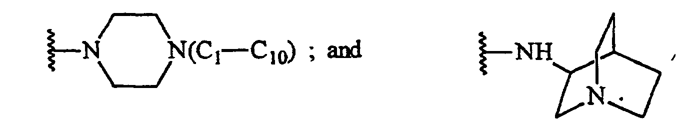

The identification of a tag by mass spectrometry is preferably based upon its molecular mass to charge ratio (m/z). The preferred molecular mass range of MS tags is from about 100 to 2,000 daltons, and preferably the T^{ms}-containing moiety has a mass of at least about 250 daltons, more preferably at least about 300 daltons, and still more preferably at least about 350 daltons. It is generally difficult for mass spectrometers to distinguish among moieties having parent ions below about 200-250 daltons (depending on the precise instrument), and thus preferred T^{ms}-containing moieties of the invention have masses above that range.

As explained above, the T^{ms}-containing moiety may contain atoms other than those present in the tag variable component, and indeed other than present in T^{ms} itself. Accordingly, the mass of T^{ms} itself may be less than about 250 daltons, so long as the T^{ms}-containing moiety has a mass of at least about 250 daltons. Thus, the mass of T^{ms} may range from 15 (*i.e.*, a methyl radical) to about 10,000 daltons, and preferably ranges from 100 to about 5,000 daltons, and more preferably ranges from about 200 to about 1,000 daltons.

It is relatively difficult to distinguish tags by mass spectrometry when those tags incorporate atoms that have more than one isotope in significant abundance. Accordingly, preferred T groups which are intended for mass spectroscopic identification (T^{ms} groups), contain carbon, at least one of hydrogen and fluoride, and optional atoms selected from oxygen, nitrogen, sulfur, phosphorus and iodine. While other atoms may be present in the T^{ms}, their presence can render analysis of the mass spectral data somewhat more difficult. Preferably, the T^{ms} groups have only carbon, nitrogen and oxygen atoms, in addition to hydrogen and/or fluoride.

Fluoride is an optional yet preferred atom to have in a T^{ms} group. In comparison to hydrogen, fluoride is, of course, much heavier. Thus, the presence of fluoride atoms rather than hydrogen atoms leads to T^{ms} groups of higher mass, thereby allowing the T^{ms} group to reach and exceed a mass of greater than 250 daltons, which is desirable as explained above. In addition, the replacement of hydrogen with fluoride confers greater volatility on the I-containing moiety, and greater volatility of the analyte enhances sensitivity when mass spectrometry is being used as the detection method.

The molecular formula of T^{ms} falls within the scope of C₁₋₅₀₀N₀₋₁₀₀O₀₋₁₀₀O₀₋₁₀₀S₀₋₁₀P₀₋₁₀H_{α}F_{β}I_{δ} wherein the sum of α, β and δ is sufficient to satisfy the otherwise unsatisfied valencies of the C, N, O, S and P atoms. The designation C₁₋₅₀₀N₀₋₁₀₀O₀₋₁₀₀S₀₋₁₀P₀₋₁₀H_{α}F_{β}I_{δ} means that T^{ms} contains at least one, and may contain any number from 1 to 500 carbon atoms, in addition to optionally containing as many as 100 nitrogen atoms ("N₀₋" means that T^{ms} need not contain any nitrogen atoms), and as many as 100 oxygen atoms, and as many as 10 sulfur atoms and as many as 10 phosphorus atoms. The symbols α, β and δ represent the number of hydrogen, fluoride and iodide atoms in T^{ms}, where any two of these numbers may be zero, and where the sum of these numbers equals the total of the otherwise unsatisfied valencies of the C, N, O, S and P atoms. Preferably, T^{ms} has a molecular formula that falls within the scope of C₁₋₅₀N₀₋₁₀O₀₋₁₀H_{α}F_{β} where the sum of α and β equals the number of hydrogen and fluoride atoms, respectively, present in the moiety.

### b. Characteristics of IR Tags

There are two primary forms of IR detection of organic chemical groups: Raman scattering IR and absorption IR. Raman scattering IR spectra and absorption IR spectra are complementary spectroscopic methods. In general, Raman excitation depends on bond polarizability changes whereas IR absorption depends on bond dipole moment changes. Weak IR absorption lines become strong Raman lines and vice versa. Wavenumber is the characteristic unit for IR spectra. There are 3 spectral regions for IR tags which have separate applications: near IR at 12500 to 4000 cm⁻¹, mid IR at 4000 to 600 cm⁻¹, far IR at 600 to 30 cm⁻¹. For the uses described herein where a compound is to serve as a tag to identify an MOI, probe or primer, the mid spectral regions would be preferred. For example, the carbonyl stretch (1850 to 1750 cm⁻¹) would be measured for carboxylic acids, carboxylic esters and amides, and alkyl and aryl carbonates, carbamates and ketones. N-H bending (1750 to 160 cm⁻¹) would be used to identify amines, ammonium ions, and amides. At 1400 to 1250 cm⁻¹, R-OH bending is detected as well as the C-N stretch in amides. Aromatic substitution patterns are detected at 900 to 690 cm⁻¹ (C-H bending, N-H bending for ArNH₂). Saturated C-H, olefins, aromatic rings, double and triple bonds, esters, acetals, ketals, ammonium salts, N-O compounds such as oximes, nitro, N-oxides, and nitrates, azo, hydrazones, quinones, carboxylic acids, amides, and lactams all possess vibrational infrared correlation data (see Pretsch et al., Spectral Data for Structure Determination of Organic Compounds, Springer-Verlag, New York, 1989). Preferred compounds would include an aromatic nitrile which exhibits a very strong nitrile stretching vibration at 2230 to 2210 cm⁻¹. Other useful types of compounds are aromatic alkynes which have a strong stretching vibration that gives rise to a sharp absorption band between 2140 and 2100 cm⁻¹. A third compound type is the aromatic azides which exhibit an intense absorption band in the 2160 to 2120 cm⁻¹ region. Thiocyanates are representative of compounds that have a strong absorption at 2275 to 2263 cm⁻¹.

### c. Characteristics of UV Tags

A compilation of organic chromophore types and their respective UV-visible properties is given in Scott (Interpretation of the UV Spectra of Natural Products, Permagon Press, New York, 1962). A chromophore is an atom or group of atoms or electrons that are responsible for the particular light absorption. Empirical rules exist for π to π* maxima in conjugated systems (see Pretsch et al., Spectral Data for Structure Determination of Organic Compounds, p. B65 and B70, Springer-Verlag, New York, 1989). Preferred compounds (with conjugated systems) would possess n to π* and π to π* transitions. Such compounds are exemplified by Acid Violet 7, Acridine Orange, Acridine Yellow G, Brilliant Blue G, Congo Red, Crystal Violet, Malachite Green oxalate, Metanil Yellow, Methylene Blue, Methyl Orange, Methyl Violet B, Naphtol Green B, Oil Blue N, Oil Red O, 4-phenylazophenol, Safranie O, Solvent Green 3, and Sudan Orange G, all of which are commercially available (Aldrich, Milwaukee, WI). Other suitable compounds are listed in, *e.g.,* Jane, I., et al., J. Chrom. 323:191-225 (1985).

### d. Characteristic of a Fluorescent Tag

Fluorescent probes are identified and quantitated most directly by their absorption and fluorescence emission wavelengths and intensities. Emission spectra (fluorescence and phosphorescence) are much more sensitive and permit more specific measurements than absorption spectra. Other photophysical characteristics such as excited-state lifetime and fluorescence anisotropy are less widely used. The most generally useful intensity parameters are the molar extinction coefficient (ε) for absorption and the quantum yield (QY) for fluorescence. The value of ε is specified at a single wavelength (usually the absorption maximum of the probe), whereas QY is a measure of the total photon emission over the entire fluorescence spectral profile. A narrow optical bandwidth (<20 nm) is usually used for fluorescence excitation (via absorption), whereas the fluorescence detection bandwidth is much more variable, ranging from full spectrum for maximal sensitivity to narrow band (∼20 nm) for maximal resolution. Fluorescence intensity per probe molecule is proportional to the product of s and QY. The range of these parameters among fluorophores of current practical importance is approximately 10,000 to 100,000 cm⁻¹M⁻¹ for ε and 0.1 to 1.0 for QY. Compounds that can serve as fluorescent tags are as follows: fluorescein, rhodamine, lambda blue 470, lambda green, lambda red 664, lambda red 665, acridine orange, and propidium iodide, which are commercially available from Lambda Fluorescence Co. (Pleasant Gap, PA). Fluorescent compounds such as nile red, Texas Red, lissamine™, BODIPY™ s are available from Molecular Probes (Eugene, OR).

### e. Characteristics of Potentiometric Tags

The principle of electrochemical detection (ECD) is based on oxidation or reduction of compounds which at certain applied voltages, electrons are either donated or accepted thus producing a current which can be measured. When certain compounds are subjected to a potential difference, the molecules undergo a molecular rearrangement at the working electrodes' surface with the loss (oxidation) or gain (reduction) of electrons, such compounds are said to be electronic and undergo electrochemical reactions. EC detectors apply a voltage at an electrode surface over which the HPLC eluent flows. Electroactive compounds eluting from the column either donate electrons (oxidize) or acquire electrons (reduce) generating a current peak in real time. Importantly the amount of current generated depends on both the concentration of the analyte and the voltage applied, with each compound having a specific voltage at which it begins to oxidize or reduce. The currently most popular electrochemical detector is the amperometric detector in which the potential is kept constant and the current produced from the electrochemical reaction is then measured. This type of spectrometry is currently called "potentiostatic amperometry". Commercial amperometers are available from ESA, Inc., Chelmford, MA.

When the efficiency of detection is 100%, the specialized detectors are termed "coulometric". Coulometric detectors are sensitive which have a number of practical advantages with regard to selectivity and sensitivity which make these types of detectors useful in an array. In coulometric detectors, for a given concentration of analyte, the signal current is plotted as a function of the applied potential (voltage) to the working electrode. The resultant sigmoidal graph is called the current-voltage curve or hydrodynamic voltammagram (HDV). The HDV allows the best choice of applied potential to the working electrode that permits one to maximize the observed signal. A major advantage of ECD is its inherent sensitivity with current levels of detection in the subfemtomole range.

Numerous chemicals and compounds are electrochemically active including many biochemicals, pharmaceuticals and pesticides. Chromatographically coeluting compounds can be effectively resolved even if their half-wave potentials (the potential at half signal maximum) differ by only 30-60 mV.

Recently developed coulometric sensors provide selectivity, identification and resolution of co-eluting compounds when used as detectors in liquid chromatography based separations. Therefore, these arrayed detectors add another set of separations accomplished in the detector itself. Current instruments possess 16 channels which are in principle limited only by the rate at which data can be acquired. The number of compounds which can be resolved on the EC array is chromatographically limited (*i.e.,* plate count limited). However, if two or more compounds that chromatographically co-elute have a difference in half wave potentials of 30-60 mV, the array is able to distinguish the compounds. The ability of a compound to be electrochemically active relies on the possession of an EC active group (*i.e.,* -OH, -O,-N, -S).

Compounds which have been successfully detected using coulometric detectors include 5-hydroxytryptamine, 3-methoxy-4-hydroxyphenyl-glycol, homogentisic acid, dopamine, metanephrine, 3-hydroxykynureninr, acetominophen, 3-hydroxytryptophol, 5-hydroxyindoleacetic acid, octanesulfonic acid, phenol, o-cresol, pyrogallol, 2-nitrophenol, 4-nitrophenol, 2,4-dinitrophenol, 4,6-dinitrocresol, 3-methyl-2-nitrophenol, 2,4-dichlorophenol, 2,6-dichlorophenol, 2,4,5-trichlorophenol, 4-chloro-3-methylphenol, 5-methylphenol, 4-methyl-2-nitrophenol, 2-hydroxyaniline, 4-hydroxyaniline, 1,2-phenylenediamine, benzocatechin, buturon, chlortholuron, diuron, isoproturon, linuron, methobromuron, metoxuron, monolinuron, monuron, methionine, tryptophan, tyrosine, 4-aminobenzoic acid, 4-hydroxybenzoic acid, 4-hydroxycoumaric acid, 7-methoxycoumarin, apigenin baicalein, caffeic acid, catechin, centaurein, chlorogenic acid, daidzein, datiscetin, diosmetin, epicatechin gallate, epigallo catechin, epigallo catechin gallate, eugenol, eupatorin, ferulic acid, fisetin, galangin, gallic acid, gardenin, genistein, gentisic acid, hesperidin, irigenin, kaemferol, leucoyanidin, luteolin, mangostin, morin, myricetin, naringin, narirutin, pelargondin, peonidin, phloretin, pratensein, protocatechuic acid, rhamnetin, quercetin, sakuranetin, scutellarein, scopoletin, syringaldehyde, syringic acid, tangeritin, troxerutin, umbelliferone, vanillic acid, 1,3-dimethyl tetrahydroisoquinoline, 6-hydroxydopamine, r-salsolinol, N-methyl-r-salsolinol, tetrahydroisoquinoline, amitriptyline, apomorphine, capsaicin, chlordiazepoxide, chlorpromazine, daunorubicin, desipramine, doxepin, fluoxetine, flurazepam, imipramine, isoproterenol, methoxamine, morphine, morphine-3-glucuronide, nortriptyline, oxazepam, phenylephrine, trimipramine, ascorbic acid, N-acetyl serotonin, 3,4-dihydroxybenzylamine, 3,4-dihydroxymandelic acid (DOMA), 3,4-dihydroxyphenylacetic acid (DOPAC), 3,4-dihydroxyphenylalanine (L-DOPA), 3,4-dihydroxyphenylglycol (DHPG), 3-hydroxyanthranilic acid, 2-hydroxyphenylacetic acid (2HPAC), 4-hydroxybenzoic acid (4HBAC), 5-hydroxyindole-3-acetic acid (5HIAA), 3-hydroxykynurenine, 3-hydroxymandelic acid, 3-hydroxy-4-methoxyphenylethylamine, 4-hydroxyphenylacetic acid (4HPAC), 4-hydroxyphenyllactic acid (4HPLA), 5-hydroxytryptophan (5HTP), 5-hydroxytryptophol (5HTOL), 5-hydroxytryptamine (5HT), 5-hydroxytryptamine sulfate, 3-methoxy-4-hydroxyphenylglycol (MHPG), 5-methoxytryptamine, 5-methoxytryptophan, 5-methoxytryptophol, 3-methoxytyramine (3MT), 3-methoxytyrosine (3-OM-DOPA), 5-methylcysteine, 3-methylguanine, bufotenin, dopamine dopamine-3-glucuronide, dopamine-3-sulfate, dopamine-4-sulfate, epinephrine, epinine, folic acid, glutathione (reduced), guanine, guanosine, homogentisic acid (HGA), homovanillic acid (HVA), homovanillyl alcohol (HVOL), homoveratic acid, hva sulfate, hypoxanthine, indole, indole-3-acetic acid, indole-3-lactic acid, kynurenine, melatonin, metanephrine, N-methyltryptamine, N-methyltyramine, N,N-dimethyltryptamine, N,N-dimethyltyramine, norepinephrine, normetanephrine, octopamine, pyridoxal, pyridoxal phosphate, pyridoxamine, synephrine, tryptophol, tryptamine, tyramine, uric acid, vanillylmandelic acid (vma), xanthine and xanthosine. Other suitable compounds are set forth in, *e.g.*, Jane, I., et al. J. Chrom. 323:191-225 (1985) and Musch, G., et al., J. Chrom. 348:97-110 (1985). These compounds can be incorporated into compounds of formula T-L-X by methods known in the art. For example, compounds having a carboxylic acid group may be reacted with amine, hydroxyl, etc. to form amide, ester and other linkages between T and L.

In addition to the above properties, and regardless of the intended detection method, it is preferred that the tag have a modular chemical structure. This aids in the construction of large numbers of structurally related tags using the techniques of combinatorial chemistry. For example, the T^{ms} group desirably has several properties. It desirably contains a functional group which supports a single ionized charge state when the T^{ms}-containing moiety is subjected to mass spectrometry (more simply referred to as a "mass spec sensitivity enhancer" group, or MSSE). Also, it desirably can serve as one member in a family of T^{ms}-containing moieties, where members of the family each have a different mass/charge ratio, however have approximately the same sensitivity in the mass spectrometer. Thus, the members of the family desirably have the same MSSE. In order to allow the creation of families of compounds, it has been found convenient to generate tag reactants via a modular synthesis scheme, so that the tag components themselves may be viewed as comprising modules.

In a preferred modular approach to the structure of the T^{ms} group, T^{ms} has the formula

T²-(J-T³-)ₙ-

wherein T² is an organic moiety formed from carbon and one or more of hydrogen, fluoride, iodide, oxygen, nitrogen, sulfur and phosphorus, having a mass range of 15 to 500 daltons; T³ is an organic moiety formed from carbon and one or more of hydrogen, fluoride, iodide, oxygen, nitrogen, sulfur and phosphorus, having a mass range of 50 to 1000 daltons; J is a direct bond or a functional group such as amide, ester, amine, sulfide, ether, thioester, disulfide, thioether, urea, thiourea, carbamate, thiocarbamate, Schiff base, reduced Schiff base, imine, oxime, hydrazone, phosphate, phosphonate, phosphoramide, phosphonamide, sulfonate, sulfonamide or carbon-carbon bond; and n is an integer ranging from 1 to 50, such that when n is greater than 1, each T³ and J is independently selected.

The modular structure T²-(J-T³)ₙ- provides a convenient entry to families of T-L-X compounds, where each member of the family has a different T group. For instance, when T is T^{ms}, and each family member desirably has the same MSSE, one of the T³ groups can provide that MSSE structure. In order to provide variability between members of a family in terms of the mass of T^{ms}, the T² group may be varied among family members. For instance, one family member may have T² = methyl, while another has T² = ethyl, and another has T² = propyl, etc.

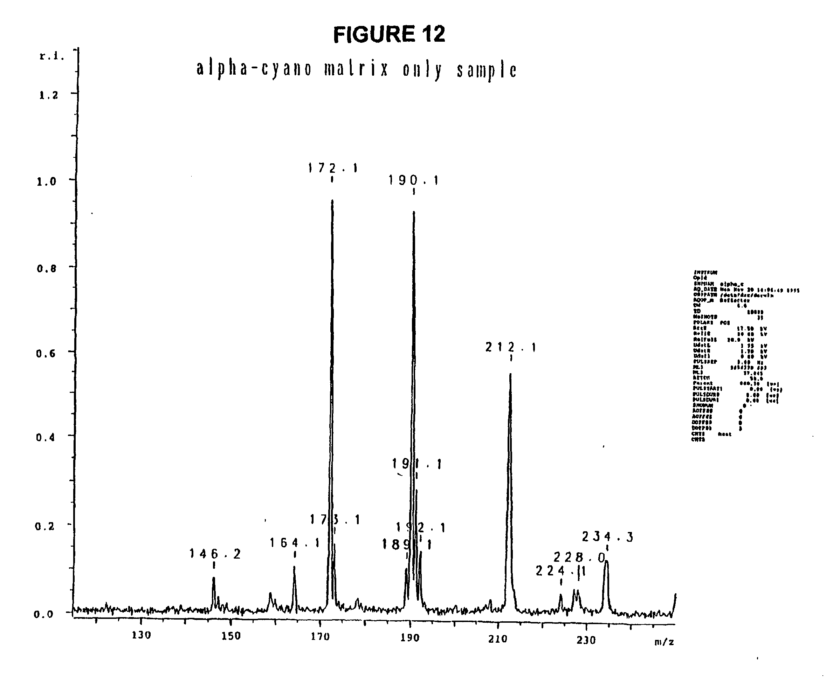

In order to provide "gross" or large jumps in mass, a T³ group may be designed which adds significant (*e.g.,* one or several hundreds) of mass units to T-L-X. Such a T³ group may be referred to as a molecular weight range adjuster group("WRA"). A WRA is quite useful if one is working with a single set of T² groups, which will have masses extending over a limited range. A single set of T² groups may be used to create T^{ms} groups having a wide range of mass simply by incorporating one or more WRA T³ groups into the T^{ms}. Thus, using a simple example, if a set of T² groups affords a mass range of 250-340 daltons for the T^{ms}, the addition of a single WRA, having, as an exemplary number 100 dalton, as a T³ group provides access to the mass range of 350-440 daltons while using the same set of T² groups. Similarly, the addition of two 100 dalton MWA groups (each as a T³ group) provides access to the mass range of 450-540 daltons, where this incremental addition of WRA groups can be continued to provide access to a very large mass range for the T^{ms} group. Preferred compounds of the formula T²-(J-T³-)ₙ-L-X have the formula R_{VWC}-(R_{WRA})_{w}-R_{MSSE}-L-X where VWC is a "T²" group, and each of the WRA and MSSE groups are "T³" groups. This structure is illustrated in Figure 12, and represents one modular approach to the preparation of T^{ms}.

In the formula T²-(J-T³-)ₙ-, T² and T³ are preferably selected from hydrocarbyl, hydrocarbyl-O-hydrocarbylene, hydrocarbyl-S-hydrocarbylene, hydrocarbyl-NH-hydrocarbylene, hydrocarbyl-amide-hydrocarbylene, N-(hydrocarbyl)hydrocarbylene, N,N-di(hydrocarbyl)hydrocarbylene, hydrocarbylacyl-hydrocarbylene, heterocyclylhydrocarbyl wherein the heteroatom(s) are selected from oxygen, nitrogen, sulfur and phosphorus, substituted heterocyclylhydrocarbyl wherein the heteroatom(s) are selected from oxygen, nitrogen, sulfur and phosphorus and the substituents are selected from hydrocarbyl, hydrocarbyl-O-hydrocarbylene, hydrocarbyl-NH-hydrocarbylene, hydrocarbyl-S-hydrocarbylene, N-(hydrocarbyl)hydrocarbylene, N,N-di(hydrocarbyl)hydrocarbylene and hydrocarbylacyl-hydrocarbylene. In addition, T² and/or T³ may be a derivative of any of the previously listed potential T² / T³ groups, such that one or more hydrogens are replaced fluorides.

Also regarding the formula T²-(J-T³-)ₙ-, a preferred T³ has the formula -G(R²)-, wherein G is C₁₋₆ alkylene chain having a single R² substituent. Thus, if G is ethylene (-CH₂-CH₂-) either one of the two ethylene carbons may have a R² substituent, and R² is selected from alkyl, alkenyl, alkynyl, cycloalkyl, aryl-fused cycloalkyl, cycloalkenyl, aryl, aralkyl, aryl-substituted alkenyl or alkynyl, cycloalkyl-substituted alkyl, cycloalkenyl-substituted cycloalkyl, biaryl, alkoxy, alkenoxy, alkynoxy, aralkoxy, aryl-substituted alkenoxy or alkynoxy, alkylamino, alkenylamino or alkynylamino, aryl-substituted alkylamino, aryl-substituted alkenylamino or alkynylamino, aryloxy, arylamino, N-alkylurea-substituted alkyl, N-arylurea-substituted alkyl, alkylcarbonylamino-substituted alkyl, aminocarbonyl-substituted alkyl, heterocyclyl, heterocyclyl-substituted alkyl, heterocyclyl-substituted amino, carboxyalkyl substituted aralkyl, oxocarbocyclyl-fused aryl and heterocyclylalkyl; cycloalkenyl, aryl-substituted alkyl and, aralkyl, hydroxy-substituted alkyl, alkoxy-substituted alkyl, aralkoxy-substituted alkyl, alkoxy-substituted alkyl, aralkoxy-substituted alkyl, amino-substituted alkyl, (aryl-substituted alkyloxycarbonylamino)-substituted alkyl, thiol-substituted alkyl, alkylsulfonyl-substituted alkyl, (hydroxy-substituted alkylthio)-substituted alkyl, thioalkoxy-substituted alkyl, hydrocarbylacylamino-substituted alkyl, heterocyclylacylamino-substituted alkyl, hydrocarbyl-substituted-heterocyclylacylamino-substituted alkyl, alkylsulfonylamino-substituted alkyl, arylsulfonylamino-substituted alkyl, morpholino-alkyl, thiomorpholino-alkyl, morpholino carbonyl-substituted alkyl, thiomorpholinocarbonyl-substituted alkyl, [N-(alkyl, alkenyl or alkynyl)- or N,N-[dialkyl, dialkenyl, dialkynyl or (alkyl, alkenyl)-amino]carbonyl-substituted alkyl, heterocyclylaminocarbonyl, heterocylylalkyleneaminocarbonyl, heterocyclylaminocarbonyl-substituted alkyl, heterocylylalkyleneaminocarbonyl-substituted alkyl, N,N-[dialkyl]alkyleneaminocarbonyl, N,N-[dialkyl]alkyleneaminocarbonyl-substituted alkyl, alkyl-substituted heterocyclylcarbonyl, alkyl-substituted heterocyclylcarbonyl-alkyl, carboxyl-substituted alkyl, dialkylamino-substituted acylaminoalkyl and amino acid side chains selected from arginine, asparagine, glutamine, S-methyl cysteine, methionine and corresponding sulfoxide and sulfone derivatives thereof, glycine, leucine, isoleucine, allo-isoleucine, tert-leucine, norleucine, phenylalanine, tyrosine, tryptophan, proline, alanine, ornithine, histidine, glutamine, valine, threonine, serine, aspartic acid, beta-cyanoalanine, and allothreonine; alynyl and heterocyclylcarbonyl, aminocarbonyl, amido, mono- or dialkylaminocarbonyl, mono- or diarylaminocarbonyl, alkylarylaminocarbonyl, diarylaminocarbonyl, mono- or diacylaminocarbonyl, aromatic or aliphatic acyl, alkyl optionally substituted by substituents selected from amino, carboxy, hydroxy, mercapto, mono- or dialkylamino, mono- or diarylamino, alkylarylamino, diarylamino, mono- or diacylamino, alkoxy, alkenoxy, aryloxy, thioalkoxy, thioalkenoxy, thioalkynoxy, thioaryloxy and heterocyclyl.

A preferred compound of the formula T²-(J-T³-)ₙ-L-X has the structure: 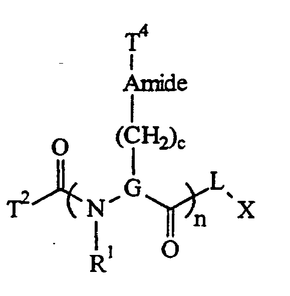 wherein G is (CH₂)₁₋₆ such that a hydrogen on one and only one of the CH₂ groups represented by a single "G" is replaced with-(CH₂)_{c}-Amide-T⁴; T² and T⁴ are organic moieties of the formula C₁₋₂₅N₀₋₉O₀₋₉H_{α}F_{β} such that the sum of α and β is sufficient to satisfy the otherwise unsatisfied valencies of the C, N, and O atoms; amide is 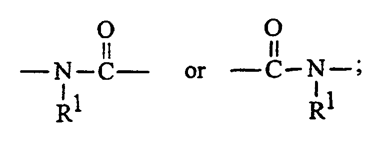 R¹ is hydrogen or C₁₋₁₀ alkyl; c is an integer ranging from 0 to 4; and n is an integer ranging from 1 to 50 such that when n is greater than 1, G, c, Amide, R¹ and T⁴ are independently selected.

In a further preferred embodiment, a compound of the formula T²-(J-T³-)ₙ-L-X has the structure: 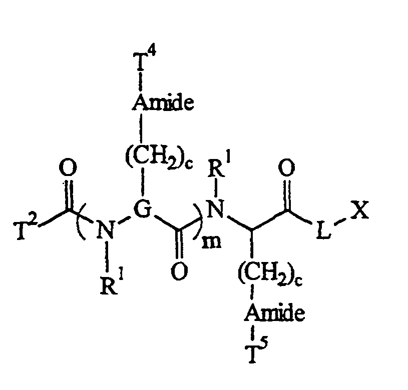 wherein T⁵ is an organic moiety of the formula C₁₋₂₅N_{0.9}O₀₋₉H_{α}F_{β} such that the sum of α and β is sufficient to satisfy the otherwise unsatisfied valencies of the C, N, and O atoms; and T⁵ includes a tertiary or quaternary amine or an organic acid; m is an integer ranging from 0-49, and T², T⁴, R¹, L and X have been previously defined.

Another preferred compound having the formula T²-(J-T³-)ₙ-L-X has the particular structure: 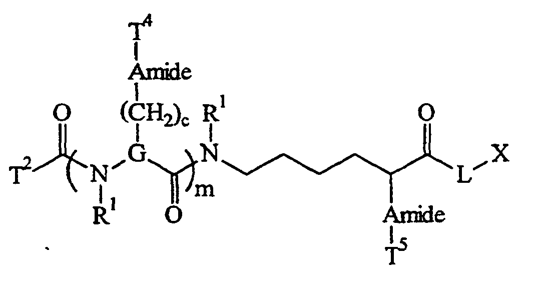 wherein T⁵ is an organic moiety of the formula C₁₋₂₅N₀₋₉O₀₋₉H_{α}F_{β} such that the sum of α and β is sufficient to satisfy the otherwise unsatisfied valencies of the C, N, and O atoms; and T⁵ includes a tertiary or quaternary amine or an organic acid; m is an integer ranging from 0-49, and T², T⁴, c, R¹, "Amide", L and X have been previously defined.

In the above structures that have a T⁵ group, -Amide-T⁵ is preferably one of the following, which are conveniently made by reacting organic acids with free amino groups extending from "G":

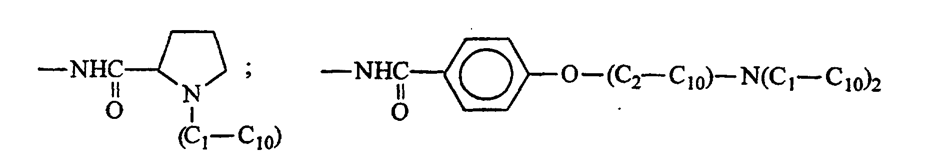

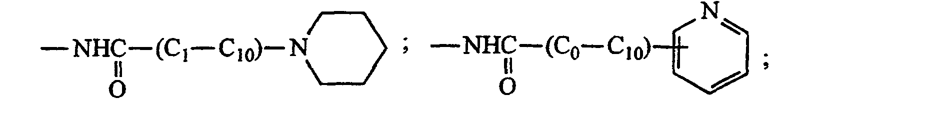

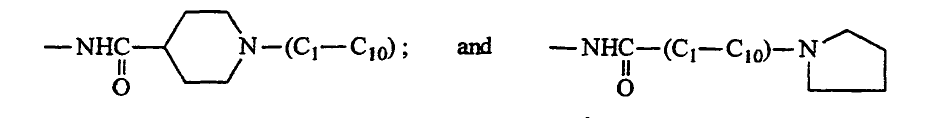

Where the above compounds have a T⁵ group, and the "G" group has a free carboxyl group (or reactive equivalent thereof), then the following are preferred -Amide- T group, which may conveniently be prepared by reacting the appropriate organic amine with a free carboxyl group extending from a "G" group:

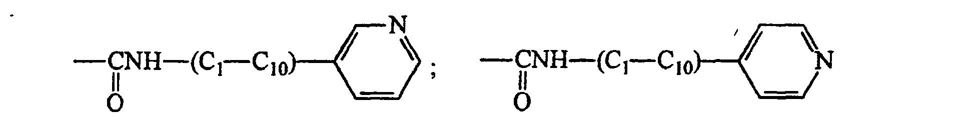

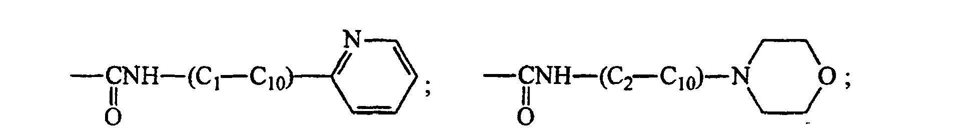

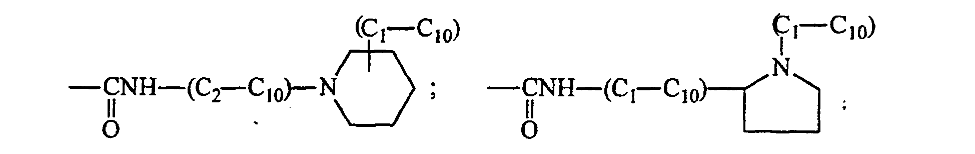

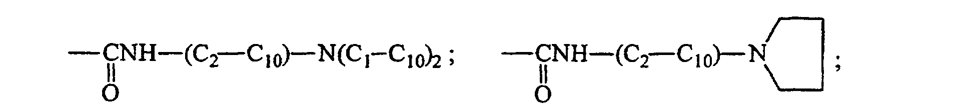

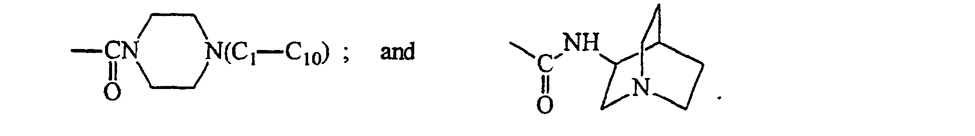

In three preferred embodiments of the invention, T-L-MOI has the structure: 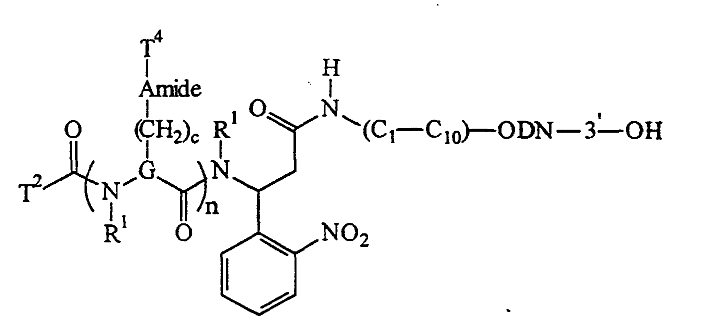 or the structure: 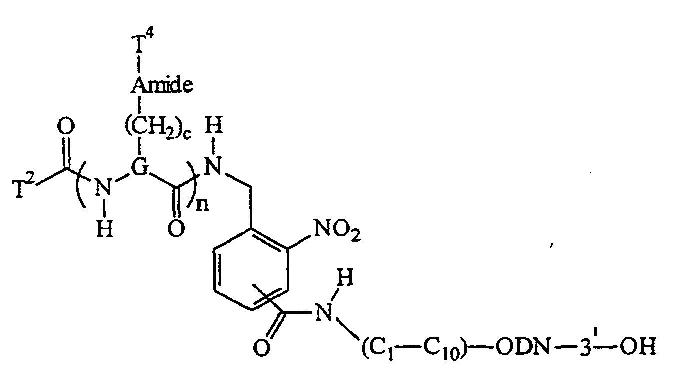 or the structure: 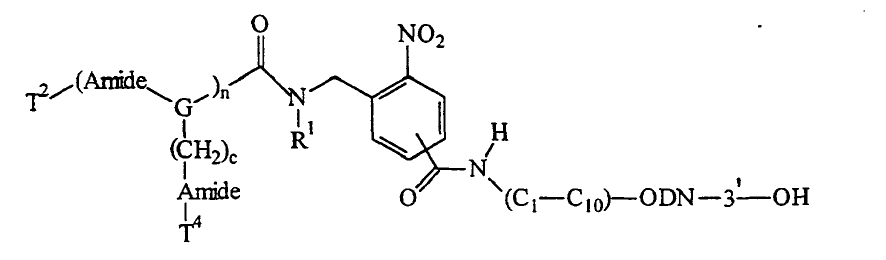 wherein T² and T⁴ are organic moieties of the formula C₁₋₂₅N₀₋₉O₀₋₉S₀₋₃P₀₋₃H_{α}F_{β}I_{δ} such that the sum of α, β and δ is sufficient to satisfy the otherwise unsatisfied valencies of the C, N, O, S and P atoms; G is (CH₂)₁₋₆ wherein one and only one hydrogen on the CH₂ groups represented by each G is replaced with -(CH₂)_{c}-Amide-T⁴; Amide is 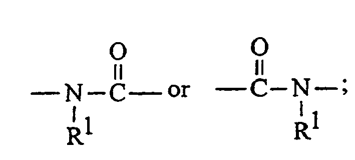 R¹ is hydrogen or C₁₋₁₀ alkyl; c is an integer ranging from 0 to 4; "C₂-C₁₀" represents a hydrocarbylene group having from 2 to 10 carbon atoms, "ODN-3'-OH" represents a nucleic acid fragment having a terminal 3' hydroxyl group (*i.e.,* a nucleic acid fragment joined to (C₁-C₁₀) at other than the 3' end of the nucleic acid fragment); and n is an integer ranging from 1 to 50 such that when n is greater than 1, then G, c, Amide, R¹ and T⁴ are independently selected. Preferably there are not three heteroatoms bonded to a single carbon atom.

In structures as set forth above that contain a T²-C(=O)-N(R¹)- group, this group may be formed by reacting an amine of the formula HN(R¹)- with an organic acid selected from the following, which are exemplary only and do not constitute an exhaustive list of potential organic acids: Formic acid, Acetic acid, Propiolic acid, Propionic acid, Fluoroacetic acid, 2-Butynoic acid, Cyclopropanecarboxylic acid, Butyric acid, Methoxyacetic acid, Difluoroacetic acid, 4-Pentynoic acid, Cyclobutanecarboxylic acid, 3,3-Dimethylacrylic acid, Valeric acid, N,N-Dimethylglycine, N-Formyl-Gly-OH, Ethoxyacetic acid, (Methylthio)acetic acid, Pyrrole-2-carboxylic acid, 3-Furoic acid, Isoxazole-5-carboxylic acid, trans-3-Hexenoic acid, Trifluoroacetic acid, Hexanoic acid, Ac-Gly-OH, 2-Hydroxy-2-methylbutyric acid, Benzoic acid, Nicotinic acid, 2-Pyrazinecarboxylic acid, 1-Methyl-2-pyrrolecarboxylic acid, 2-Cyclopentene-1-acetic acid, Cyclopentylacetic acid, (S)-(-)-2-Pyrrolidone-5-carboxylic acid, N-Methyl-L-proline, Heptanoic acid, Ac-b-Ala-OH, 2-Ethyl-2-hydroxybutyric acid, 2-(2-Methoxyethoxy)acetic acid, p-Toluic acid, 6-Methylnicotinic acid, 5-Methyl-2-pyrazinecarboxylic acid, 2,5-Dimethylpyrrole-3-carboxylic acid, 4-Fluorobenzoic acid, 3,5-Dimethylisoxazole-4-carboxylic acid, 3-Cyclopentylpropionic acid, Octanoic acid, N,N-Dimethylsuccinamic acid, Phenylpropiolic acid, Cinnamic acid, 4-Ethylbenzoic acid, p-Anisic acid, 1,2,5-Trimethylpyrrole-3-carboxylic acid, 3-Fluoro-4-methylbenzoic acid, Ac-DL-Propargylglycine, 3-(Trifluoromethyl)butyric acid, 1-Piperidinepropionic acid, N-Acetylproline, 3,5-Difluorobenzoic acid, Ac-L-Val-OH, Indole-2-carboxylic acid, 2-Benzofurancarboxylic acid, Benzotriazole-5-carboxylic acid, 4-n-Propylbenzoic acid, 3-Dimethylaminobenzoic acid, 4-Ethoxybenzoic acid, 4-(Methylthio)benzoic acid, N-(2-Furoyl)glycine, 2-(Methylthio)nicotinic acid, 3-Fluoro-4-methoxybenzoic acid, Tfa-Gly-OH, 2-Napthoic acid, Quinaldic acid, Ac-L-Ile-OH, 3-Methylindene-2-carboxylic acid, 2-Quinoxalinecarboxylic acid, 1-Methylindole-2-carboxylic acid, 2,3,6-Trifluorobenzoic acid, N-Formyl-L-Met-OH, 2-[2-(2-Methoxyethoxy)ethoxy]acetic acid, 4-n-Butylbenzoic acid, N-Benzoylglycine, 5-Fluoroindole-2-carboxylic acid, 4-n-Propoxybenzoic acid, 4-Acetyl-3,5-dimethyl-2-pyrrolecarboxylic acid, 3,5-Dimethoxybenzoic acid, 2,6-Dimethoxynicotinic acid, Cyclohexanepentanoic acid, 2-Naphthylacetic acid, 4-(IH-Pyrrol-1-yl)benzoic acid, Indole-3-propionic acid, m-Trifluoromethylbenzoic acid, 5-Methoxyindole-2-carboxylic acid, 4-Pentylbenzoic acid, Bz-b-Ala-OH, 4-Diethylaminobenzoic acid, 4-n-Butoxybenzoic acid, 3-Methyl-5-CF3-isoxazole-4-carboxylic acid, (3,4-Dimethoxyphenyl)acetic acid, 4-Biphenylcarboxylic acid, Pivaloyl-Pro-OH, Octanoyl-Gly-OH, (2-Naphthoxy)acetic acid, Indole-3-butyric acid, 4-(Trifluoromethyl)phenylacetic acid, 5-Methoxyindole-3-acetic acid, 4-(Trifluoromethoxy)benzoic acid, Ac-L-Phe-OH, 4-Pentyloxybenzoic acid, Z-Gly-OH, 4-Carboxy-N-(fur-2-ylmethyl)pyrrolidin-2-one, 3,4-Diethoxybenzoic acid, 2,4-Dimethyl-5-CO₂Et-pyrrole-3-carboxylic acid, N-(2-Fluorophenyl)succinamic acid, 3,4,5-Trimethoxybenzoic acid, N-Phenylanthranilic acid, 3-Phenoxybenzoic acid, Nonanoyl-Gly-OH, 2-Phenoxypyridine-3-carboxylic acid, 2,5-Dimethyl-1-phenylpyrrole-3-carboxylic acid, trans-4-(Trifluoromethyl)cinnamic acid, (5-Methyl-2-phenyloxazol-4-yl)acetic acid, 4-(2-Cyclohexenyloxy)benzoic acid, 5-Methoxy-2-methylindole-3-acetic acid, trans-4-Cotininecarboxylic acid, Bz-5-Aminovaleric acid, 4-Hexyloxybenzoic acid, N-(3-Methoxyphenyl)succinamic acid, Z-Sar-OH, 4-(3,4-Dimethoxyphenyl)butyric acid, Ac-o-Fluoro-DL-Phe-OH, N-(4-Fluorophenyl)glutaramic acid, 4'-Ethyl-4-biphenylcarboxylic acid, 1,2,3,4-Tetrahydroacridinecarboxylic acid, 3-Phenoxyphenylacetic acid, N-(2,4-Difluorophenyl)succinamic acid, N-Decanoyl-Gly-OH, (+)-6-Methoxy-a-methyl-2-naphthaleneacetic acid, 3-(Trifluoromethoxy)cinnamic acid, N-Formyl-DL-Trp-OH, (R)-(+)-a-Methoxy-a-(trifluoromethyl)phenylacetic acid, Bz-DL-Leu-OH, 4-(Trifluoromethoxy)phenoxyacetic acid, 4-Heptyloxybenzoic acid, 2,3,4-Trimethoxycinnamic acid, 2,6-Dimethoxybenzoyl-Gly-OH, 3-(3,4,5-Trimethoxyphenyl)propionic acid, 2,3,4,5,6-Pentafluorophenoxyacetic acid, N-(2,4-Difluorophenyl)glutaramic acid, N-Undecanoyl-Gly-OH, 2-(4-Fluorobenzoyl)benzoic acid, 5-Trifluoromethoxyindole-2-carboxylic acid, N-(2,4-Difluorophenyl)diglycolamic acid, Ac-L-Trp-OH, Tfa-L-Phenylglycine-OH, 3-Iodobenzoic acid, 3-(4-n-Pentylbenzoyl)propionic acid, 2-Phenyl4-quinolinecarboxylic acid, 4-Octyloxybenzoic acid, Bz-L-Met-OH, 3,4,5-Triethoxybenzoic acid, N-Lauroyl-Gly-OH, 3,5-Bis(trifluoromethyl)benzoic acid, Ac-5-Methyl-DL-Trp-OH, 2-Iodophenylacetic acid, 3-Iodo-4-methylbenzoic acid, 3-(4-n-Hexylbenzoyl)propionic acid, N-Hexanoyl-L-Phe-OH, 4-Nonyloxybenzoic acid, 4'-(Trifluoromethyl)-2-biphenylcarboxylic acid, Bz-L-Phe-OH, N-Tridecanoyl-Gly-OH, 3,5-Bis(trifluoromethyl)phenylacetic acid, 3-(4-n-Heptylbenzoyl)propionic acid, N-Hepytanoyl-L-Phe-OH, 4-Decyloxybenzoic acid, N-(a,a,a-trifluoro-m-tolyl)anthranilic acid, Niflumic acid, 4-(2-Hydroxyhexafluoroisopropyl)benzoic acid, N-Myristoyl-Gly-OH, 3-(4-n-Octylbenzoyl)propionic acid, N-Octanoyl-L-Phe-OH, 4-Undecyloxybenzoic acid, 3-(3,4,5-Trimethoxyphenyl)propionyl-Gly-OH, 8-Iodonaphthoic acid, N-Pentadecanoyl-Gly-OH, 4-Dodecyloxybenzoic acid, N-Palmitoyl-Gly-OH, and N-Stearoyl-Gly-OH. These organic acids are available from one or more of Advanced ChemTech, Louisville, KY; Bachem Bioscience Inc., Torrance, CA; Calbiochem-Novabiochem Corp., San Diego, CA; Farchan Laboratories Inc., Gainesville FL; Lancaster Synthesis, Windham NH; and MayBridge Chemical Company (c/o Ryan Scientific), Columbia, SC. The catalogs from these companies use the abreviations which are used above to identify the acids.

### f. Combinatorial Chemistry as a Means for Preparing Tags

Combinatorial chemistry is a type of synthetic strategy which leads to the production of large chemical libraries (see, for example, PCT Application Publication No. WO 94/08051). These combinatorial libraries can be used as tags for the identification of molecules of interest (MOIs). Combinatorial chemistry may be defined as the systematic and repetitive, covalent connection of a set of different "building blocks" of varying structures to each other to yield a large array of diverse molecular entities. Building blocks can take many forms, both naturally occurring and synthetic, such as nucleophiles, electrophiles, dienes, alkylating or acylating agents, diamines, nucleotides, amino acids, sugars, lipids, organic monomers, synthons, and combinations of the above. Chemical reactions used to connect the building blocks may involve alkylation, acylation, oxidation, reduction, hydrolysis, substitution, elimination, addition, cyclization, condensation, and the like. This process can produce libraries of compounds which are oligomeric, non-oligomeric, or combinations thereof. If oligomeric, the compounds can be branched, unbranched, or cyclic. Examples of oligomeric structures which can be prepared by combinatorial methods include oligopeptides, oligonucleotides, oligosaccharides, polylipids, polyesters, polyamides, polyurethanes, polyureas, polyethers, poly(phosphorus derivatives), *e.g.*, phosphates, phosphonates, phosphoramides, phosphonamides, phosphites, phosphinamides, etc., and poly(sulfur derivatives), *e.g.,* sulfones, sulfonates, sulfites, sulfonamides, sulfenamides, etc.

One common type of oligomeric combinatorial library is the peptide combinatorial library. Recent innovations in peptide chemistry and molecular biology have enabled libraries consisting of tens to hundreds of millions of different peptide sequences to be prepared and used. Such libraries can be divided into three broad categories. One category of libraries involves the chemical synthesis of soluble nonsupport-bound peptide libraries (*e.g.,* Houghten et al., Nature 354:84, 1991). A second category involves the chemical synthesis of support-bound peptide libraries, presented on solid supports such as plastic pins, resin beads, or cotton (Geysen et al., Mol. Immunol. 23:709, 1986; Lam et al., Nature 354:82, 1991; Eichler and Houghten, Biochemistry 32:11035, 1993). In these first two categories, the building blocks are typically L-amino acids, D-amino acids, unnatural amino acids, or some mixture or combination thereof. A third category uses molecular biology approaches to prepare peptides or proteins on the surface of filamentous phage particles or plasmids (Scott and Craig, Curr. Opinion Biotech. 5:40, 1994). Soluble, nonsupport-bound peptide libraries appear to be suitable for a number of applications, including use as tags. The available repertoire of chemical diversities in peptide libraries can be expanded by steps such as permethylation (Ostresh et al., Proc. Natl. Acad Sci., USA 91:11138, 1994).

Numerous variants of peptide combinatorial libraries are possible in which the peptide backbone is modified, and/or the amide bonds have been replaced by mimetic groups. Amide mimetic groups which may be used include ureas, urethanes, and carbonylmethylene groups. Restructuring the backbone such that sidechains emanate from the amide nitrogens of each amino acid, rather than the alpha-carbons, gives libraries of compounds known as peptoids (Simon et al., Proc. Natl. Acad. Sci., USA 89:9367, 1992).

Another common type of oligomeric combinatorial library is the oligonucleotide combinatorial library, where the building blocks are some form of naturally occurring or unnatural nucleotide or polysaccharide derivatives, including where various organic and inorganic groups may substitute for the phosphate linkage, and nitrogen or sulfur may substitute for oxygen in an ether linkage (Schneider et al., Biochem. 34:9599, 1995; Freier et al., J. Med. Chem. 38:344, 1995; Frank, J. Biotechnology 41:259, 1995; Schneider et al., Published PCT WO 942052; Ecker et al., Nucleic Acids Res. 21:1853, 1993).

More recently, the combinatorial production of collections of non-oligomeric, small molecule compounds has been described (DeWitt et al., Proc. Natl. Acad Sci., USA 90:690, 1993; Bunin et al., Proc. Natl. Acad. Sci., USA 91:4708, 1994). Structures suitable for elaboration into small-molecule libraries encompass a wide variety of organic molecules, for example heterocyclics, aromatics, alicyclics, aliphatics, steroids, antibiotics, enzyme inhibitors, ligands, hormones, drugs, alkaloids, opioids, terpenes, porphyrins, toxins, catalysts, as well as combinations thereof.

### g. Specific Methods for Combinatorial Synthesis of Tags

Two methods for the preparation and use of a diverse set of amine-containing MS tags are outlined below. In both methods, solid phase synthesis is employed to enable simultaneous parallel synthesis of a large number of tagged linkers, using the techniques of combinatorial chemistry. In the first method, the eventual cleavage of the tag from the oligonucleotide results in liberation of a carboxyl amide. In the second method, cleavage of the tag produces a carboxylic acid. The chemical components and linking elements used in these methods are abbreviated as follows:
- R =: resin
- FMOC =: fluorenylmethoxycarbonyl protecting group
- All =: allyl protecting group
- CO₂H =: carboxylic acid group
- CONH₂ =: carboxylic amide group
- NH₂ =: amino group
- OH =: hydroxyl group
- CONH =: amide linkage
- COO =: ester linkage
- NH₂ - Rink - CO₂H =: 4-[(a-amino)-2,4-dimethoxybenzyl]- phenoxybutyric acid (Rink linker)
- OH - 1MeO - CO₂H =: (4-hydroxymethyl)phenoxybutyric acid
- OH - 2MeO - CO₂H =: (4-hydroxymethyl-3-methoxy)phenoxyacetic acid
- NH₂-A-COOH =: amino acid with aliphatic or aromatic amine functionality in side chain
- X1....Xn-COOH =: set of n diverse carboxylic acids with unique molecular weights
- oligo1... oligo(n) =: set of n oligonucleotides
- HBTU =: O-benzotriazol-1-yl-N,N,N',N'-tetramethyluronium hexafluorophosphate

The sequence of steps in Method 1 is as follows:

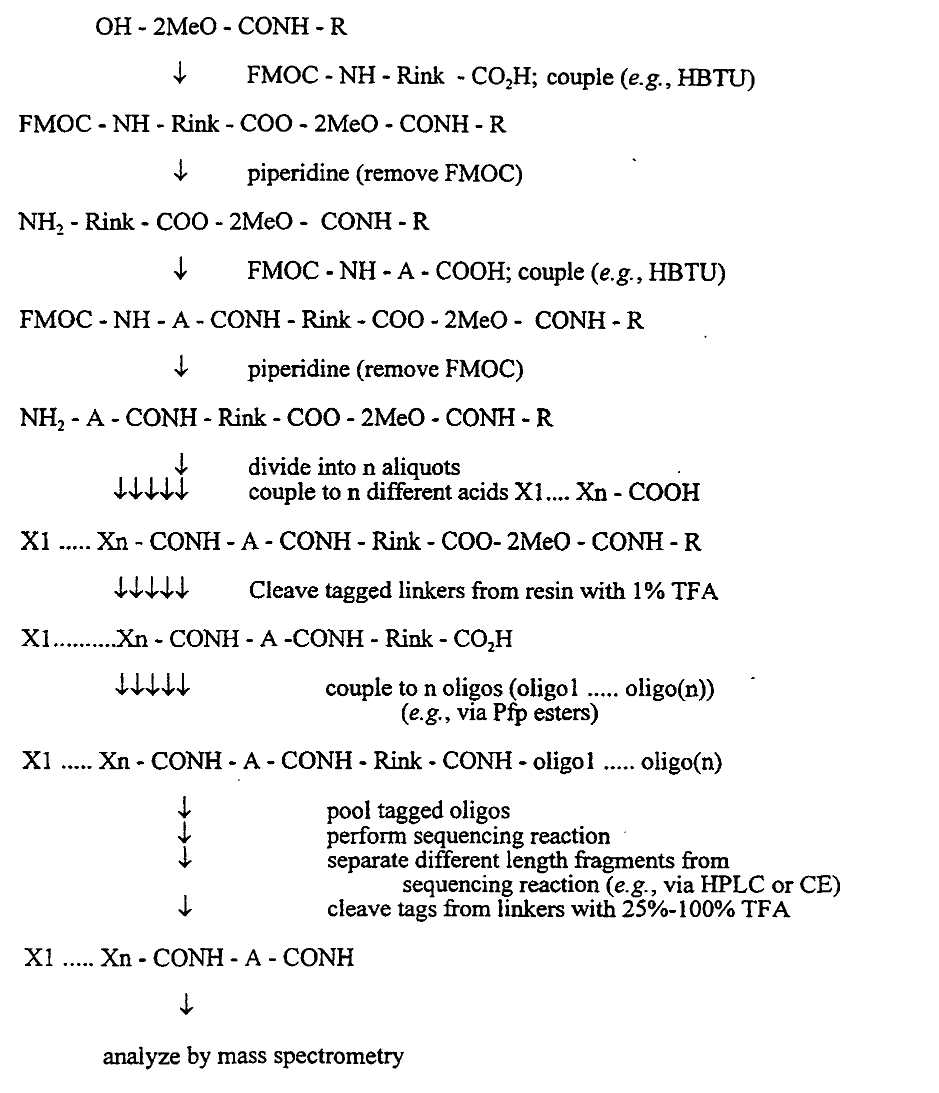

The sequence of steps in Method 2 is as follows:

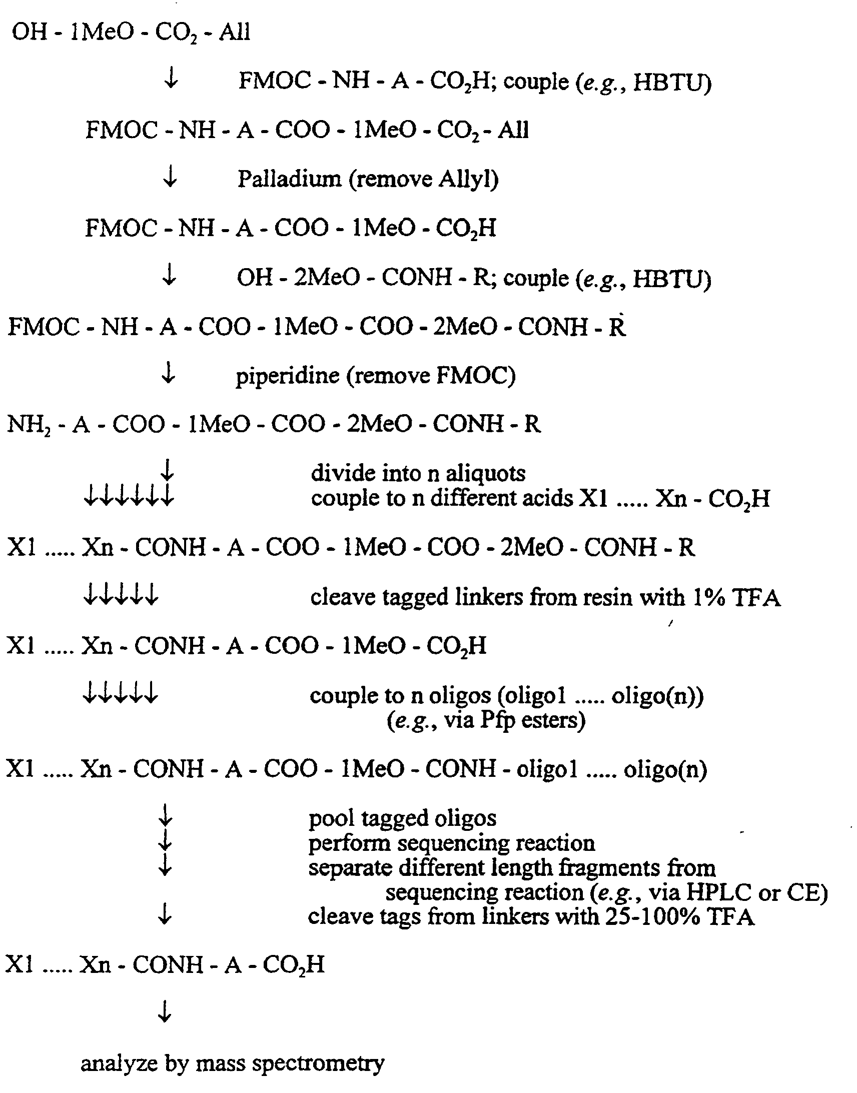

### 2. Linkers

A "linker" component (or L), as used herein, means either a direct covalent bond or an organic chemical group which is used to connect a "tag" (or T) to a "molecule of interest" (or MOI) through covalent chemical bonds. In addition, the direct bond itself, or one or more bonds within the linker component is cleavable under conditions which allows T to be released (in other words, cleaved) from the remainder of the T-L-X compound (including the MOI component). The tag variable component which is present within T should be stable to the cleavage conditions. Preferably, the cleavage can be accomplished rapidly; within a few minutes and preferably within about 15 seconds or less.

In general, a linker is used to connect each of a large set of tags to each of a similarly large set of MOIs. Typically, a single tag-linker combination is attached to each MOI (to give various T-L-MOI), but in some cases, more than one tag-linker combination may be attached to each individual MOI (to give various (T-L)n-MOI). In another embodiment of the present invention, two or more tags are bonded to a single linker through multiple, independent sites on the linker, and this multiple tag-linker combination is then bonded to an individual MOI (to give various (T)n-L-MOI).

After various manipulations of the set of tagged MOIs, special chemical and/or physical conditions are used to cleave one or more covalent bonds in the linker, resulting in the liberation of the tags from the MOIs. The cleavable bond(s) may or may not be some of the same bonds that were formed when the tag, linker, and MOI were connected together. The design of the linker will, in large part, determine the conditions under which cleavage may be accomplished. Accordingly, linkers may be identified by the cleavage conditions they are particularly susceptible too. When a linker is photolabile (*i.e.,* prone to cleavage by exposure to actinic radiation), the linker may be given the designation L^{hυ}. Likewise, the designations L^{acid}, L^{base}, L^{[O]}, L^{[R]}, L^{enz}**,** L^{elc}, L^{Δ} and L^{ss} may be used to refer to linkers that are particularly susceptible to cleavage by acid, base, chemical oxidation, chemical reduction, the catalytic activity of an enzyme (more simply "enzyme"), electrochemical oxidation or reduction, elevated temperature ("thermal") and thiol exchange, respectively.

Certain types of linker are labile to a single type of cleavage condition, whereas others are labile to several types of cleavage conditions. In addition, in linkers which are capable of bonding multiple tags (to give (T)n-L-MOI type structures), each of the tag-bonding sites may be labile to different cleavage conditions. For example, in a linker having two tags bonded to it, one of the tags may be labile only to base, and the other labile only to photolysis.

A linker which is useful in the present invention possesses several attributes:
1) The linker possesses a chemical handle (Lₕ) through which it can be attached to an MOI.
2) The linker possesses a second, separate chemical handle (Lₕ) through which the tag is attached to the linker. If multiple tags are attached to a single linker ((T)n-L-MOI type structures), then a separate handle exists for each tag.
3) The linker is stable toward all manipulations to which it is subjected, with the exception of the conditions which allow cleavage such that a T-containing moiety is released from the remainder of the compound, including the MOI. Thus, the linker is stable during attachment of the tag to the linker, attachment of the linker to the MOI, and any manipulations of the MOI while the tag and linker (T-L) are attached to it.
4) The linker does not significantly interfere with the manipulations performed on the MOI while the T-L is attached to it. For instance, if the T-L is attached to an oligonucleotide, the T-L must not significantly interfere with any hybridization or enzymatic reactions (*e.g.*, PCR) performed on the oligonucleotide. Similarly, if the T-L is attached to an antibody, it must not significantly interfere with antigen recognition by the antibody.
5) Cleavage of the tag from the remainder of the compound occurs in a highly controlled manner, using physical or chemical processes that do not adversely affect the detectability of the tag.

For any given linker, it is preferred that the linker be attachable to a wide variety of MOIs, and that a wide variety of tags be attachable to the linker. Such flexibility is advantageous because it allows a library of T-L conjugates, once prepared, to be used with several different sets of MOIs.

As explained above, a preferred linker has the formula

Lₕ-L¹-L²-L³-Lₕ

wherein each Lₕ is a reactive handle that can be used to link the linker to a tag reactant and a molecule of interest reactant. L² is an essential part of the linker, because L² imparts lability to the linker. L¹ and L³ are optional groups which effectively serve to separate L² from the handles Lₕ.

L¹ (which, by definition, is nearer to T than is L³), serves to separate T from the required labile moiety L². This separation may be useful when the cleavage reaction generates particularly reactive species (*e.g.*, free radicals) which may cause random changes in the structure of the T-containing moiety. As the cleavage site is further separated from the T-containing moiety, there is a reduced likelihood that reactive species formed at the cleavage site will disrupt the structure of the T-containing moiety. Also, as the atoms in L1 will typically be present in the T-containing moiety, these L¹ atoms may impart a desirable quality to the T-containing moiety. For example, where the T-containing moiety is a T^{ms}-containing moiety, and a hindered amine is desirably present as part of the structure of the T^{ms}-containing moiety (to serve, *e.g.,* as a MSSE), the hindered amine may be present in L¹ labile moiety.

In other instances, L¹ and/or L³ may be present in a linker component merely because the commercial supplier of a linker chooses to sell the linker in a form having such a L¹ and/or L³ group. In such an instance, there is no harm in using linkers having L¹ and/or L³ groups, (so long as these group do not inhibit the cleavage reaction) even though they may not contribute any particular performance advantage to the compounds that incorporate them. Thus, the present invention allows for L¹ and/or L³ groups to be present in the linker component.

L¹ and/or L³ groups may be a direct bond (in which case the group is effectively not present), a hydrocarbylene group (*e.g.,* alkylene, arylene, cycloalkylene, etc.), -O-hydrocarbylene (*e.g.*, -O-CH₂-, O-CH₂CH(CH₃)-, etc.) or hydrocarbylene-(O-hydrocarbylene)_{w}- wherein w is an integer ranging from 1 to about 10 (*e.g.,* -CH₂-O-Ar-, -CH₂-(O-CH₂CH₂)₄-, etc.).

With the advent of solid phase synthesis, a great body of literature has developed regarding linkers that are labile to specific reaction conditions. In typical solid phase synthesis, a solid support is bonded through a labile linker to a reactive site, and a molecule to be synthesized is generated at the reactive site. When the molecule has been completely synthesized, the solid support-linker-molecule construct is subjected to cleavage conditions which releases the molecule from the solid support. The labile linkers which have been developed for use in this context (or which may be used in this context) may also be readily used as the linker reactant in the present invention.

Lloyd-Williams, P., et al., "Convergent Solid-Phase Peptide Synthesis", Tetrahedron Report No. 347, 49(48):11065-11133 (1993) provides an extensive discussion of linkers which are labile to actinic radiation (*i.e.,* photolysis), as well as acid, base and other cleavage conditions. Additional sources of information about labile linkers may be readily obtained.

As described above, different linker designs will confer cleavability ("lability") under different specific physical or chemical conditions. Examples of conditions which serve to cleave various designs of linker include acid, base, oxidation, reduction, fluoride, thiol exchange, photolysis, and enzymatic conditions.

Examples of cleavable linkers that satisfy the general criteria for linkers listed above will be well known to those in the art and include those found in the catalog available from Pierce (Rockford, IL). Examples include:
- ethylene glycobis(succinimidylsuccinate) (EGS), an amine reactive cross-linking reagent which is cleavable by hydroxylamine (1 M at 37°C for 3-6 hours);
- disuccinimidyl tartarate (DST) and sulfo-DST, which are amine reactive cross-linking reagents, cleavable by 0.015 M sodium periodate;
- bis[2-(succinimidyloxycarbonyloxy)ethyl]sulfone (BSOCOES) and sulfo-BSOCOES, which are amine reactive cross-linking reagents, cleavable by base (pH 11.6);
- 1,4-di-[3'-(2'-pyridyldithio(propionamido))butane (DPDPB), a pyridyldithiol crosslinker which is cleavable by thiol exchange or reduction;
- N-[4-(p-azidosalicylamido)-butyl]-3'-(2'-pyridydithio)propionamide (APDP), a pyridyldithiol crosslinker which is cleavable by thiol exchange or reduction;
- bis-[beta-4-(azidosalicylamido)ethyl]-disulfide, a photoreactive crosslinker which is cleavable by thiol exchange or reduction;
- N-succinimidyl-(4-azidophenyl)-1,3'dithiopropionate (SADP), a photoreactive crosslinker which is cleavable by thiol exchange or reduction;
- sulfosuccinimidyl-2-(7-azido-4-methylcoumarin-3-acetamide)ethyl-1,3'-dithiopropionate (SAED), a photoreactive crosslinker which is cleavable by thiol exchange or reduction;
- sulfosuccinimidyl-2-(m-azido-o-nitrobenzamido)-ethyl-1,3'dithiopropionate (SAND), a photoreactive crosslinker which is cleavable by thiol exchange or reduction.

Other examples of cleavable linkers and the cleavage conditions that can be used to release tags are as follows. A silyl linking group can be cleaved by fluoride or under acidic conditions. A 3-, 4-, 5-, or 6-substituted-2-nitrobenzyloxy or 2-, 3-, 5-, or 6-substituted-4-nitrobenzyloxy linking group can be cleaved by a photon source (photolysis). A 3-, 4-, 5-, or 6-substituted-2-alkoxyphenoxy or 2-, 3-, 5-, or 6-substituted-4-alkoxyphenoxy linking group can be cleaved by Ce(NH₄)₂(NO₃)₆ (oxidation). A NCO₂ (urethane) linker can be cleaved by hydroxide (base), acid, or LiAlH₄ (reduction). A 3-pentenyl, 2-butenyl, or 1-butenyl linking group can be cleaved by O₃, O_{S}O₄/IO₄⁻, or KMnO₄ (oxidation). A 2-[3-, 4-, or 5-substituted-furyl]oxy linking group can be cleaved by O₂, Br₂, MeOH, or acid.

Conditions for the cleavage of other labile linking groups include: t-alkyloxy linking groups can be cleaved by acid; methyl(dialkyl)methoxy or 4-substituted-2-alkyl-1,3-dioxlane-2-yl linking groups can be cleaved by H₃O⁺; 2-silylethoxy linking groups can be cleaved by fluoride or acid; 2-(X)-ethoxy (where X = keto, ester amide, cyano, NO₂, sulfide, sulfoxide, sulfone) linking groups can be cleaved under alkaline conditions; 2-, 3-, 4-, 5-, or 6-substituted-benzyloxy linking groups can be cleaved by acid or under reductive conditions; 2-butenyloxy linking groups can be cleaved by (Ph₃P)₃RhCl(H), 3-, 4-, 5-, or 6-substituted-2-bromophenoxy linking groups can be cleaved by Li, Mg, or BuLi; methylthiomethoxy linking groups can be cleaved by Hg²⁺; 2-(X)-ethyloxy (where X = a halogen) linking groups can be cleaved by Zn or Mg; 2-hydroxyethyloxy linking groups can be cleaved by oxidation (*e.g.*, with Pb(OAc)₄).

Preferred linkers are those that are cleaved by acid or photolysis. Several of the acid-labile linkers that have been developed for solid phase peptide synthesis are useful for linking tags to MOIs. Some of these linkers are described in a recent review by Lloyd-Williams et al. (Tetrahedron 49:11065-11133, 1993). One useful type of linker is based upon p-alkoxybenzyl alcohols, of which two, 4-hydroxymethylphenoxyacetic acid and 4-(4-hydroxymethyl-3-methoxyphenoxy)butyric acid, are commercially available from Advanced ChemTech (Louisville, KY). Both linkers can be attached to a tag via an ester linkage to the benzylalcohol, and to an amine-containing MOI via an amide linkage to the carboxylic acid. Tags linked by these molecules are released from the MOI with varying concentrations of trifluoroacetic acid. The cleavage of these linkers results in the liberation of a carboxylic acid on the tag. Acid cleavage of tags attached through related linkers, such as 2,4-dimethoxy-4'-(carboxymethyloxy)-benzhydrylamine (available from Advanced ChemTech in FMOC-protected form), results in liberation of a carboxylic amide on the released tag.

The photolabile linkers useful for this application have also been for the most part developed for solid phase peptide synthesis (see Lloyd-Williams review). These linkers are usually based on 2-nitrobenzylesters or 2-nitrobenzylamides. Two examples of photolabile linkers that have recently been reported in the literature are 4-(4-(1-Fmoc-amino)ethyl)-2-methoxy-5-nitrophenoxy)butanoic acid (Holmes and Jones, J. Org. Chem. 60:2318-2319, 1995) and 3-(Fmoc-amino)-3-(2-nitrophenyl)propionic acid (Brown et al., Molecular Diversity 1:4-12, 1995). Both linkers can be attached via the carboxylic acid to an amine on the MOI. The attachment of the tag to the linker is made by forming an amide between a carboxylic acid on the tag and the amine on the linker. Cleavage of photolabile linkers is usually performed with UV light of 350 nm wavelength at intensities and times known to those in the art. Cleavage of the linkers results in liberation of a primary amide on the tag. Examples of photocleavable linkers include nitrophenyl glycine esters, exo- and endo-2-benzonorborneyl chlorides and methane sulfonates, and 3-amino-3(2-nitrophenyl) propionic acid. Examples of enzymatic cleavage include esterases which will cleave ester bonds, nucleases which will cleave phosphodiester bonds, proteases which cleave peptide bonds, etc.

A preferred linker component has an ortho-nitrobenzyl structure as shown below: 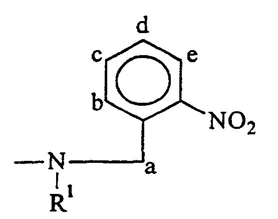 wherein one carbon atom at positions a, b, c, d or e is substituted with -L³-X, and L¹ (which is preferably a direct bond) is present to the left of N(R¹) in the above structure. Such a linker component is susceptible to selective photo-induced cleavage of the bond between the carbon labeled "a" and N(R¹). The identity of R¹ is not typically critical to the cleavage reaction, however R¹ is preferably selected from hydrogen and hydrocarbyl. The present invention provides that in the above structure, -N(R¹)- could be replaced with -O-. Also in the above structure, one or more of positions b, c, d or e may optionally be substituted with alkyl, alkoxy, fluoride, chloride, hydroxyl, carboxylate or amide, where these substituents are independently selected at each occurrence.

A further preferred linker component with a chemical handle Lₕ has the following structure: 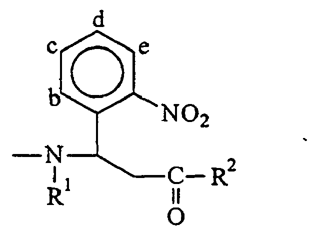 wherein one or more of positions b, c, d or e is substituted with hydrogen, alkyl, alkoxy, fluoride, chloride, hydroxyl, carboxylate or amide, R¹ is hydrogen or hydrocarbyl, and R² is -OH or a group that either protects or activates a carboxylic acid for coupling with another moiety. Fluorocarbon and hydrofluorocarbon groups are preferred groups that activate a carboxylic acid toward coupling with another moiety.

### 3. Molecule of Interest (MOI)

Examples of MOIs include nucleic acids or nucleic acid analogues (*e.g.,* PNA), fragments of nucleic acids (*i.e.,* nucleic acid fragments), synthetic nucleic acids or fragments, oligonucleotides (*e.g.,* DNA or RNA), proteins, peptides, antibodies or antibody fragments, receptors, receptor ligands, members of a ligand pair, cytokines, hormones, oligosaccharides, synthetic organic molecules, drugs, and combinations thereof. The MOIs of the present invention are nucleic acids.

Preferred MOIs include nucleic acid fragments. Preferred nucleic acid fragments are primer sequences that are complementary to sequences present in vectors, where the vectors are used for base sequencing. Preferably a nucleic acid fragment is attached directly or indirectly to a tag at other than the 3' end of the fragment; and most preferably at the 5' end of the fragment. Nucleic acid fragments may be purchased or prepared based upon genetic databases (e.g., Dib et al., Nature 380:152-154, 1996 and CEPH Genotype Database, http://www.cephb.fr) and commercial vendors (e.g., Promega, Madison, WI).

As used herein, MOI includes derivatives of an MOI that contain functionality useful in joining the MOI to a T-L-Lₕ compound. For example, a nucleic acid fragment that has a phosphodiester at the 5' end, where the phosphodiester is also bonded to an alkyleneamine, is an MOI. Such an MOI is described in, *e.g.,* U.S. Patent 4,762,779. A nucleic acid fragment with an internal modification is also an MOI. An exemplary internal modification of a nucleic acid fragment is where the base (*e.g.,* adenine, guanine, cytosine, thymidine, uracil) has been modified to add a reactive functional group. Such internally modified nucleic acid fragments are commercially available from, *e.g.,* Glen Research, Herndon, VA. Another exemplary internal modification of a nucleic acid fragment is where an abasic phosphoramidate is used to synthesize a modified phosphodiester which is interposed between a sugar and phosphate group of a nucleic acid fragment. The abasic phosphoramidate contains a reactive group which allows a nucleic acid fragment that contains this phosphoramidate-derived moiety to be joined to another moiety, *e.g.,* a T-L-Lₕ compound. Such abasic phosphoramidates are commercially available from, *e.g.,* Clonetech Laboratories, Inc., Palo Alto, CA.

### 4. Chemical Handles (Lₕ)

A chemical handle is a stable yet reactive atomic arrangement present as part of a first molecule, where the handle can undergo chemical reaction with a complementary chemical handle present as part of a second molecule, so as to form a covalent bond between the two molecules. For example, the chemical handle may be a hydroxyl group, and the complementary chemical handle may be a carboxylic acid group (or an activated derivative thereof, *e.g.,* a hydrofluroaryl ester), whereupon reaction between these two handles forms a covalent bond (specifically, an ester group) that joins the two molecules together.

Chemical handles may be used in a large number of covalent bond-forming reactions that are suitable for attaching tags to linkers, and linkers to MOIs. Such reactions include alkylation (*e.g.,* to form ethers, thioethers), acylation (*e.g.,* to form esters, amides, carbamates, ureas, thioureas), phosphorylation (*e.g.,* to form phosphates, phosphonates, phosphoramides, phosphonamides), sulfonylation (*e.g.,* to form sulfonates, sulfonamides), condensation (*e.g.,* to form imines, oximes, hydrazones), silylation, disulfide formation, and generation of reactive intermediates, such as nitrenes or carbenes, by photolysis. In general, handles and bond-forming reactions which are suitable for attaching tags to linkers are also suitable for attaching linkers to MOIs, and vice-versa. In some cases, the MOI may undergo prior modification or derivitization to provide the handle needed for attaching the linker.

One type of bond especially useful for attaching linkers to MOIs is the disulfide bond. Its formation requires the presence of a thiol group ("handle") on the linker, and another thiol group on the MOI. Mild oxidizing conditions then suffice to bond the two thiols together as a disulfide. Disulfide formation can also be induced by using an excess of an appropriate disulfide exchange reagent, *e.g.,* pyridyl disulfides. Because disulfide formation is readily reversible, the disulfide may also be used as the cleavable bond for liberating the tag, if desired. This is typically accomplished under similarly mild conditions, using an excess of an appropriate thiol exchange reagent, *e.g.,* dithiothreitol.

Of particular interest for linking tags (or tags with linkers) to oligonucleotides is the formation of amide bonds. Primary aliphatic amine handles can be readily introduced onto synthetic oligonucleotides with phosphoramidites such as 6-monomethoxytritylhexylcyanoethyl-N,N-diisopropyl phosphoramidite (available from Glenn Research, Sterling, VA). The amines found on natural nucleotides such as adenosine and guanosine are virtually unreactive when compared to the introduced primary amine. This difference in reactivity forms the basis of the ability to selectively form amides and related bonding groups (*e.g.,* ureas, thioureas, sulfonamides) with the introduced primary amine, and not the nucleotide amines.

As listed in the Molecular Probes catalog (Eugene, OR), a partial enumeration of amine-reactive functional groups includes activated carboxylic esters, isocyanates, isothiocyanates, sulfonyl halides, and dichlorotriazenes. Active esters are excellent reagents for amine modification since the amide products formed are very stable. Also, these reagents have good reactivity with aliphatic amines and low reactivity with the nucleotide amines of oligonucleotides. Examples of active esters include N-hydroxysuccinimide esters, pentafluorophenyl esters, tetrafluorophenyl esters, and p-nitrophenyl esters. Active esters are useful because they can be made from virtually any molecule that contains a carboxylic acid. Methods to make active esters are listed in Bodansky (Principles of Peptide Chemistry (2d ed.), Springer Verlag, London, 1993).

### 5. Linker Attachment

Typically, a single type of linker is used to connect a particular set or family of tags to a particular set or family of MOIs. In a preferred embodiment of the invention, a single, uniform procedure may be followed to create all the various T-L-MOI structures. This is especially advantageous when the set of T-L-MOI structures is large, because it allows the set to be prepared using the methods of combinatorial chemistry or other parallel processing technology. In a similar manner, the use of a single type of linker allows a single, uniform procedure to be employed for cleaving all the various T-L-MOI structures. Again, this is advantageous for a large set of T-L-MOI structures, because the set may be processed in a parallel, repetitive, and/or automated manner.

There are, however, other embodiment of the present invention, wherein two or more types of linker are used to connect different subsets of tags to corresponding subsets of MOIs. In this case, selective cleavage conditions may be used to cleave each of the linkers independently, without cleaving the linkers present on other subsets of MOIs.

A large number of covalent bond-forming reactions are suitable for attaching tags to linkers, and linkers to MOIs. Such reactions include alkylation (*e.g.,* to form ethers, thioethers), acylation (*e.g.,* to form esters, amides, carbamates, ureas, thioureas), phosphorylation (*e.g.,* to form phosphates, phosphonates, phosphoramides, phosphonamides), sulfonylation (*e.g.,* to form sulfonates, sulfonamides), condensation (*e.g.,* to form imines, oximes, hydrazones), silylation, disulfide formation, and generation of reactive intermediates, such as nitrenes or carbenes, by photolysis. In general, handles and bond-forming reactions which are suitable for attaching tags to linkers are also suitable for attaching linkers to MOIs, and vice-versa. In some cases, the MOI may undergo prior modification or derivitization to provide the handle needed for attaching the linker.

One type of bond especially useful for attaching linkers to MOIs is the disulfide bond. Its formation requires the presence of a thiol group ("handle") on the linker, and another thiol group on the MOI. Mild oxidizing conditions then suffice to bond the two thiols together as a disulfide. Disulfide formation can also be induced by using an excess of an appropriate disulfide exchange reagent, *e.g*., pyridyl disulfides. Because disulfide formation is readily reversible, the disulfide may also be used as the cleavable bond for liberating the tag, if desired. This is typically accomplished under similarly mild conditions, using an excess of an appropriate thiol exchange reagent, *e.g.,* dithiothreitol.

Of particular interest for linking tags to oligonucleotides is the formation of amide bonds. Primary aliphatic amine handles can be readily introduced onto synthetic oligonucleotides with phosphoramidites such as 6-monomethoxytritylhexylcyanoethyl-N,N-diisopropyl phosphoramidite (available from Glenn Research, Sterling, VA). The amines found on natural nucleotides such as adenosine and guanosine are virtually unreactive when compared to the introduced primary amine. This difference in reactivity forms the basis of the ability to selectively form amides and related bonding groups (*e.g.,* ureas, thioureas, sulfonamides) with the introduced primary amine, and not the nucleotide amines.

As listed in the Molecular Probes catalog (Eugene, OR), a partial enumeration of amine-reactive functional groups includes activated carboxylic esters, isocyanates, isothiocyanates, sulfonyl halides, and dichlorotriazenes. Active esters are excellent reagents for amine modification since the amide products formed are very stable. Also, these reagents have good reactivity with aliphatic amines and low reactivity with the nucleotide amines of oligonucleotides. Examples of active esters include N-hydroxysuccinimide esters, pentafluorophenyl esters, tetrafluorophenyl esters, and p-nitrophenyl esters. Active esters are useful because they can be made from virtually any molecule that contains a carboxylic acid. Methods to make active esters are listed in Bodansky (Principles of Peptide Chemistry (2d ed.), Springer Verlag, London, 1993).

Numerous commercial cross-linking reagents exist which can serve as linkers (*e.g*., see Pierce Cross-linkers, Pierce Chemical Co., Rockford, IL). Among these are homobifunctional amine-reactive cross-linking reagents which are exemplified by homobifunctional imidoesters and N-hydroxysuccinimidyl (NHS) esters. There also exist heterobifunctional cross-linking reagents possess two or more different reactive groups that allows for sequential reactions. Imidoesters react rapidly with amines at alkaline pH. NHS-esters give stable products when reacted with primary or secondary amines. Maleimides, alkyl and aryl halides, alpha-haloacyls and pyridyl disulfides are thiol reactive. Maleimides are specific for thiol (sulfhydryl) group in the pH range of 6.5 to 7.5, and at alkaline pH can become amine reactive. The thioether linkage is stable under physiological conditions. Alpha-haloacetyl cross-linking reagents contain the iodoacetyl group and are reactive towards sulfhydryls. Imidazoles can react with the iodoacetyl moiety, but the reaction is very slow. Pyridyl disulfides react with thiol groups to form a disulfide bond. Carbodiimides couple carboxyls to primary amines of hydrazides which give rises to the formation of an acyl-hydrazine bond. The arylazides are photoaffinity reagents which are chemically inert until exposed to UV or visible light. When such compounds are photolyzed at 250-460 nm, a reactive aryl nitrene is formed. The reactive aryl nitrene is relatively non-specific. Glyoxals are reactive towards guanidinyl portion of arginine.

In one typical embodiment of the present invention, a tag is first bonded to a linker, then the combination of tag and linker is bonded to a MOI, to create the structure T-L-MOI. Alternatively, the same structure is formed by first bonding a linker to a MOI, and then bonding the combination of linker and MOI to a tag. An example is where the MOI is a DNA primer or oligonucleotide. In that case, the tag is typically first bonded to a linker, then the T-L is bonded to a DNA primer or oligonucleotide, which is then used, for example, in a sequencing reaction.

One useful form in which a tag could be reversibly attached to an MOI (*e.g.,* an oligonucleotide or DNA sequencing primer) is through a chemically labile linker. One preferred design for the linker allows the linker to be cleaved when exposed to a volatile organic acid, for example, trifluoroacetic acid (TFA). TFA in particular is compatible with most methods of MS ionization, including electrospray.

As described in detail below, the invention provides methodology for genotyping. A composition which is useful in the genotyping method comprises a purality of compounds of the formula:

T^{ms}-L-MOI

wherein,
T^{ms} is an organic group detectable by mass spectrometry, comprising carbon, at least one of hydrogen and fluoride, and optional atoms selected from oxygen, nitrogen, sulfur, phosphorus and iodine. In the formula, L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid. In the formula, MOI is a nucleic acid fragment wherein L is conjugated to the MOI at a location other than the 3' end of the MOI. In the composition, at least two compounds have the same T^{ms} but the MOI groups of those molecules have non-identical nucleotide lengths.

Another composition that is useful in the genotyping method comprises a plurality of compounds of the formula:

T^{ms}-L-MOI

wherein T^{ms} is an organic group detectable by mass spectrometry, comprising carbon, at least one of hydrogen and fluoride, and optional atoms selected from oxygen, nitrogen, sulfur, phosphorus and iodine. In the formula, L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid. In the formula, MOI is a nucleic acid fragment wherein L is conjugated to the MOI at a location other than the 3' end of the MOI. In the composition, at least two compounds have the same T^{ms} but those compounds have non-identical elution times by column chromatography.

Another composition that may be used in the genotyping method comprises a plurality of compounds of the formula:

T^{ms}-L-MOI

wherein T^{ms} is an organic group detectable by mass spectrometry, comprising carbon, at least one of hydrogen and fluoride, and optional atoms selected from oxygen, nitrogen, sulfur, phosphorus and iodine. In the formula, L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid. In the formula, MOI is a nucleic acid fragment wherein L is conjugated to the MOI at a location other than the 3' end of the MOI. In the composition, no two compounds which have the same MOI nucleotide length also have the same T^{ms}.

In the above composition, the plurality is preferably greater than 2, and preferably greater than 4. Also, the nucleic acid fragment in the MOI have a sequence complementary to a portion of a vector, wherein the fragment is capable of priming polynucleotide synthesis. Preferably, the T^{ms} groups of members of the plurality differ by at least 2 amu, and may differ by at least 4 amu.

Also described is a composition comprising a plurality of sets of compounds, each set of compounds having the formula:

T^{ms}-L-MOI

wherein T^{ms} is an organic group detectable by mass spectrometry, comprising carbon, at least one of hydrogen and fluoride, and optional atoms selected from oxygen, nitrogen, sulfur, phosphorus and iodine. In the formula, L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid. Also, in the formula, MOI is a nucleic acid fragment wherein L is conjugated to the MOI at a location other than the 3' end of the MOI. In the composition, members within a first set of compounds have identical Tms groups, however have non-identical MOI groups with differing numbers of nucleotides in the MOI and there are at least ten members within the first set, wherein between sets, the T^{ms} groups differ by at least 2 amu. The plurality is preferably at least 3, and more preferably at least 5.

Also described is a composition comprising a plurality of sets of compounds, each set of compounds having the formula

T^{ms}-L-MOI

wherein, T^{ms} is an organic group detectable by mass spectrometry, comprising carbon, at least one of hydrogen and fluoride, and optional atoms selected from oxygen, nitrogen, sulfur, phosphorus and iodine. In the formula, L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid. In the formula, MOI is a nucleic acid fragment wherein L is conjugated to the MOI at a location other than the 3' end of the MOI. In the composition, the compounds within a set have the same elution time but non-identical T^{ms} groups.

In addition, herein described is a kit for genotyping. The kit comprises a plurality of amplification primer pairs, wherein at least one of the primers has the formula:

T^{ms}-L-MOI

wherein T^{ms} is an organic group detectable by mass spectrometry, comprising carbon, at least one of hydrogen and fluoride, and optional atoms selected from oxygen, nitrogen, sulfur, phosphorus and iodine. In the formula, L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid. In the formula, MOI is a nucleic acid fragment wherein L is conjugated to the MOI at a location other than the 3' end of the MOI; and each primer pair associates with a different loci. In the kit, the pluality is preferably at least 3, and more preferably at least 5.

As noted above, herein described are compositions and methods for determining the sequence of nucleic acid molecules. Briefly, such methods generally comprise the steps of (a) generating tagged nucleic acid fragments which are complementary to a selected nucleic acid molecule (e.g., tagged fragments) from a first terminus to a second terminus of a nucleic acid molecule), wherein a tag is correlative with a particular or selected nucleotide, and may be detected by any of a variety of methods, (b) separating the tagged fragments by sequential length, (c) cleaving a tag from a tagged fragment, and (d) detecting the tags, and thereby determining the sequence of the nucleic acid molecule. Each of the aspects will be discussed in more detail below.

### B. DIAGNOSTIC METHODS

### 1. Introduction

As noted above, the present by described are a wide variety of methods wherein the above-described tags and/or linkers may be utilized in place of traditional labels (*e.g.,* radioactive or enzymatic), in order to enhance the specificity, sensitivity, or number of samples that may be simultaneously analyzed, within a given method. Representative examples of such methods which may be enhanced include, for example, RNA amplification (*see* Lizardi et al., Bio/Technology 6:1197-1202, 1988; Kramer et al., Nature 339:401-402, 1989; Lomeli et al., Clinical Chem. 35(9):1826-1831, 1989; U.S. Patent No. 4,786,600), and DNA amplification utilizing LCR or polymerase chain reaction ("PCR") (*see,* U.S. Patent Nos. 4,683,195, 4,683,202, and 4,800,159).

Methods are described for
1) determining the identity of a nucleic acid molecule or fragment (or for detecting the presence of a selected nucleic acid molecule or fragment), comprising the steps of (a) generating tagged nucleic acid molecules from one or more selected target nucleic acid molecules, wherein a tag is correlative with a particular nucleic acid molecule and detectable by non-fluorescent spectrometry or potentiometry, (b) separating the tagged molecules by size, (c) cleaving the tags from the tagged molecules, and (d) detecting the tags by non-fluorescent spectrometry or potentiometry, and therefrom determining the identity of the nucleic acid molecules.
2) detecting a selected nucleic acid molecule, comprising the steps of (a) combining tagged nucleic acid probes with target nucleic acid molecules under conditions and for a time sufficient to permit hybridization of a tagged nucleic acid probe to a complementary selected target nucleic acid sequence, wherein a tagged nucleic acid probe is detectable by non-fluroescent spectrometry or potentiometry, (b) altering the size of hybridized tagged probes, unhybridized probes or target molecules, or the probe:target hybrids, (c) separating the tagged probes by size, (d) cleaving tags from the tagged probes, and (e) detecting tags by non-fluorescent spectrometry or potentiometry, and therefrom detecting the selected nucleic acid molecule. These, other related techniques are discussed in more detail below.

### 2. PCR

PCR can amplify a desired DNA sequence of any origin (virus, bacteria, plant, or human) hundreds of millions of times in a matter of hours. PCR is especially valuable because the reaction is highly specific, easily automated, and capable of amplifying minute amounts of sample. For these reasons, PCR has had a major impact on clinical medicine, genetic disease diagnostics, forensic science and evolutionary biology.

Briefly, PCR is a process based on a specialized polymerase, which can synthesize a complementary strand to a given DNA strand in a mixture containing the 4 DNA bases and 2 DNA fragments (primers, each about 20 bases long) flanking the target sequence. The mixture is heated to separate the strands of double- stranded DNA containing the target sequence and then cooled to allow (1) the primers to find and bind to their complementary sequences on the separated strands and (2) the polymerase to extend the primers into new complementary strands. Repeated heating and cooling cycles multiply the target DNA exponentially, since each new double strand separates to become two templates for further synthesis. In about 1 hour, 20 PCR cycles can amplify the target by a millionfold.

Methods are described for determining the identity of a nucleic acid molecule, or for detecting the selected nucleic acid molecule in, for example, a biological sample, utilizing the technique of PCR. Briefly, such methods comprise the steps of generating a series of tagged nucleic acid fragments or molecules during the PCR and separating the resulting fragments are by size. The size separation step can be accomplished utilizing any of the techniques described herein, including for example gel electrophoresis (*e.g*., polyacrylamide gel electrophoresis) or preferably HPLC. The tags are then cleaved from the separated fragments and detected by the respective detection technology. Examples of such technologies have been described herein, and include for example mass spectrometry, infra-red spectrometry, potentiostatic amperometry or UV spectrometry.

### 3. RNA Fingerprinting and Differential Display

When the template is RNA, the first step in fingerprinting is reverse transcription. Liang and Pardee (Science 257:967, 1992) were the first to describe an RNA fingerprinting protocol, using a primer for reverse transcription based on oligo (dT) but with an 'anchor' of two bases at the 5' end (*e.g.,* oligo 5'-(dT₁₁)CA-3'. Priming occurs mainly at the 5' end of the poly(rA) tail and mainly in sequences that end 5'-UpG-poly(rA)-3', with a selectivity approaching one out of 12 polyadenylated RNAs. After reverse transcription and denaturation, arbitrary priming is performed on the resulting first strand of cDNA. PCR can now be used to generate a fingerprint of products that best matches the primers and that are derived from the 3' end of the mRNAs and polyadenylated heterogeneous RNAs. This protocol has been named 'differential display'.

Alternatively, an arbitrary primer can be used in the first step of reverse transcription, selecting those regions internal to the RNA that have 6-8 base matches with the 3' end of the primer. This is followed by arbitrary priming of the resulting first strand of cDNA with the same or a different arbitrary primer and then PCR. This particular protocol samples anywhere in the RNA, including open reading frames (Welsh et al., Nuc. Acids. Res. 20:4965, 1992). In addition, it can be used on RNAs that are not polyadenylated, such as many bacterial RNAs. This variant of RNA fingerprinting by arbitrarily primed PCR has been called RAP-PCR.

If arbitrarily primed PCR fingerprinting of RNA is performed on samples derived from cells, tissues or other biological material that have been subjected to different experimental treatments or have different developmental histories, differences in gene expression between the samples can be detected. For each reaction, it is assumed that the same number of effective PCR doubling events occur and any differences in the initial concentrations of cDNA products are preserved as a ratio of intensities in the final fingerprint. There are no meaningful relationships between the intensities of bands within a single lane on a gel, which are a function of match and abundance. However, the ratio between lanes is preserved for each sampled RNA, allowing differentially expressed RNAs to be detected. The ratio of starting materials between samples is maintained even when the number of cycles is sufficient to allow the PCR reaction to saturate. This is because the number of doublings needed to reach saturation are almost completely controlled by the invariant products that make up the majority of the fingerprint. In this regard, PCR fingerprinting is different from conventional PCR of a single product in which the ratio of starting materials between samples is not preserved unless products are sampled in the exponential phase of amplification.

Methods are also described for determining the identity of a nucleic acid molecule, or for detecting a selecting nucleic acid molecule, in, for example a biological sample, utilizing the technique of RNA fingerprinting. Briefly, such methods generally comprise the steps of generating a series of tagged nucleic acid fragments. The fragments generated by PCR or similar amplification schemes and are then subsequently separated by size. The size separation step can be, for example, any of the techniques described herein, including for example gel electrophoresis (*e.g.,* polyacrylamide gel electrophoresis) or preferably HPLC. The tags are then cleaved from the separated fragments, and then the tags are detected by the respective detection technology. Representative examples of suitable technologies include mass spectrometry, infra-red spectrometry, potentiostatic amperometry or UV spectrometry. The relative quantities of any given nucleic acid fragments are not important, but the size of the band is informative when referenced to a control sample.

### 4. Fluorescence-Based PCR Single-Strand Conformation Polymorphism (PCR-SSCP)

A number of methods in addition to the RFLP approach are available for analyzing base substitution polymorphisms. Orita, *et al.* have devised a way of analyzing these polymorphisms on the basis of conformational differences in denatured DNA. Briefly, restriction enzyme digestion or PCR is used to produce relatively small DNA fragments which are then denatured and resolved by electrophoresis on non-denaturing polyacrylamide gels. Conformational differences in the single-stranded DNA fragments resulting from base substitutions are detected by electrophoretic mobility shifts. Intra-strand base pairing creates single strand conformations that are highly sequence-specific and distinctive in electrophoretic mobility. However, detection rates in different studies using conventional SSCP range from 35% to nearly 100% with the highest detection rates most often requiring several different conditions. In principle, the method could also be used to analyze polymorphisms based on short insertions or deletions. This method is one of the most powerful tools for identifying point mutations and deletions in DNA (SSCP-PCR, Dean et al., Cell 61:863, 1990).

Methods are described for determining the identity of a nucleic acid molecule, or for detecting a selecting nucleic acid molecule, in, for example a biological sample, utilizing the technique of PCR-SSP. Briefly, such methods generally comprise the steps of generating a series of tagged nucleic acid fragments. The fragments generated by PCR are then separated by size. Preferably, the size separation step is non-denaturing and the nucleic acid fragments are denatured prior to the separation methodology. The size separation step can be accomplished, for example gel electrophoresis (*e.g.,* polyacrylamide gel electrophoresis) or preferably HPLC. The tags are then cleaved from the separated fragments, and then the tags are detected by the respective detection technology *(e.g.,* mass spectrometry, infra-red spectrometry, potentiostatic amperometry or UV spectrometry).

### 5. Dideoxy Fingerprinting (ddF)

Another method has been described (ddF, Sarkar et al., Genomics 13:441, 1992) that detected 100% of single-base changes in the human factor IX gene when tested in a retrospective and prospective manner. In total, 84 of 84 different sequence changes were detected when genomic DNA was analyzed from patients with hemophilia B.

Briefly, in the applications of tags for genotyping or other purposes, one method that can be used is dideoxy-fingerprinting. This method utilizes a dideoxy terminator in a Sanger sequencing reation. The principle of the method is as follows: a target nucleic acid that is to be sequenced is placed in a reaction which possesses a dideoxy-terminator complementary to the base known to be mutated in the target nucleic acid. For example, if the mutation results in a A->G change, the reaction would be carried out in a C dideoxy-terminator reaction. PCR primers are used to locate and amplify the target sequence of interest. If the hypothetical target sequence contains the A->G change, the size of a population of sequences is changed due to the incorporation of a dideoxy-terminator in the amplified sequences. In this particular application of tags, a fragment would be generated which would possess a predictable size in the case of a mutation. The tags would be attached to the 5'-end of the PCR primers and provide a "map" to sample type and dideoxy-terminator type. A PCR amplification reaction would take place, the resulting fragments would be separated by size by for example HPLC or PAGE. At the end of the separation procedure, the DNA fragments are collected in a temporal reference frame, the tags are cleaved and the presence or absence of mutation is determined by the chain length due to premature chain terminator by the incorporation of a given dideoxy-terminator.

It is important to note that ddf results in the gain or loss of a dideoxy-termination segment and or a shift in the mobility of at least one of the termination segments or products. Therefore, in this method, a search is made of the shift of one fragment mobility in a high background of other molecular weight fragments. One advantage is the foreknowledge of the length of fragment associated with a given mutation.

Further described are methods for determining the identity of a nucleic acid molecule, or for detecting a selecting nucleic acid molecule, in, for example a biological sample, utilizing the technique of ddF. Briefly, such methods generally comprise the steps of generating a series of tagged nucleic acid fragments, followed by separation based upon size. Preferably, the size separation step is non-denaturing and the nucleic acid fragments are denatured prior to the separation methodology. The size separation step can be accomplished, for example gel electrophoresis (*e.g.,* polyacrylamide gel electrophoresis) or preferably HPLC. The tags are then cleaved from the separated fragments, and then the tags are detected by the respective detection technology (*e.g.,* mass spectrometry, infra-red spectrometry, potentiostatic amperometry or UV spectrometry).

### 6. Restriction Maps and RFLPs

Restriction endonucleases recognize short DNA sequences and cut DNA molecules at those specific sites. Some restriction enzymes (rare-cutters) cut DNA very infrequently, generating a small number of very large fragments (several thousand to a million bp). Most enzymes cut DNA more frequently, thus generating a large number of small fragments (less than a hundred to more than a thousand bp). On average, restriction enzymes with 4-base recognition sites will yield pieces 256 bases long, 6-base recognition sites will yield pieces 4000 bases long, and 8-base recognition sites will yield pieces 64,000 bases long. Since hundreds of different restriction enzymes have been characterized, DNA can be cut into many different small fragments.

A wide variety of techniques have been developed for the analysis of DNA polymorphisms. The most widely used method, the restriction fragment length polymorphism (RFPL) approach, combines restriction enzyme digestion, gel electrophoresis, blotting to a membrane and hybridization to a cloned DNA probe. Polymorphisms are detected as variations in the lengths of the labeled fragments on the blots. The RFLP approach can be used to analyze base substitutions when the sequence change falls within a restriction enzyme site or to analyze minisatellites/VNTRs by choosing restriction enzymes that cut outside the repeat units. The agarose gels do not usually afford the resolution necessary to distinguish minisatellite/VNTR alleles differing by a single repeat unit, but many of the minisatellites/VNTRs are so variable that highly informative markers can still be obtained.

Methods are described for determining the identity of a nucleic acid molecule, or for detecting a selecting nucleic acid molecule, in, for example a biological sample, utilizing the technique of restriction mapping or RFLPs. Briefly, such methods generally comprise the steps of generating a series of tagged nucleic acid fragments in which the fragments generated are digested with restriction enzymes. The tagged fragments are generated by conducting a hybridization step of the tagged probes with the digested target nucleic acid. The hybridization step can take place prior to or after the restriction nuclease digestion. The resulting digested nucleic acid fragments are then separated by size. The size separation step can be accomplished, for example gel electrophoresis (*e.g.,* polyacrylamide gel electrophoresis) or preferably HPLC. The tags are then cleaved from the separated fragments, and then the tags are detected by the respective detection technology (*e.g.,* mass spectrometry, infra-red spectrometry, potentiostatic amperometry or UV spectrometry).

### 7. DNA Fingerprinting

DNA fingerprinting involves the display of a set of DNA fragments from a specific DNA sample. A variety of DNA fingerprinting techniques are presently available (Jeffries et al., Nature 314:67, 1985; Welsh and McClelland, Nuc. Acids. Res. 19:861, 1991), most of which use PCR to generate fragments. The choice of which fingerprinting technique to use, is dependent on the application, *e.g.,* DNA typing, DNA marker mapping and the organisms under investigation, *e.g.,* prokaryotes, plants, animals, humans. A number of fingerprinting methods which meet these requirements have been developed over the past few years, including random amplified polymorphic DNA (RAPD). DNA amplification fingerprinting (DAF) and arbitrarily primed PCR (AP-PCR). These methods are all based on the amplification of random genomic DNA fragments by arbitrarily selected PCR primers. DNA fragment patterns may be generated of any DNA without prior sequence knowledge. The patterns generated depend on the sequence of the PCR primers and the nature of the template DNA. PCR is performed at low annealing temperatures to allow the primers to anneal to multiple loci on the DNA. DNA fragments are generated when primer binding sites are within a distance that allows amplification. In principle, a single primer is sufficient for generating band patterns.

A new technique for DNA fingerprinting has been described, named AFLP (Vos et al., Nuc. Acids Res. 23:4407, 1995). The AFLP technique is based on the detection of genomic restriction fragments by PCR amplification, and can be used for DNAs of any origin or complexity. Briefly, fingerprints are produced without prior sequence knowledge using a limited set of generic primers. The number of fragments detected in a single reaction can be "tunes" by selection of specific primer sets. The AFLP technique is robust and reliable because stringent reaction conditions are used for primer annealing: the reliability of the RFLP technique is combined with the power of the PCR technique.

Methods are also described for determining the identity of a nucleic acid molecule, or for detecting a selecting nucleic acid molecule, in, for example a biological sample, utilizing the technique of DNA fingerprinting. Briefly, such methods generally comprise the steps of generating a series of tagged nucleic acid fragments, followed by separation of the fragments by size. The size separation step can be accomplished, for example gel electrophoresis (*e.g.,* polyacrylamide gel electrophoresis) or preferably HPLC. The tags are then cleaved from the separated fragments, and then the tags are detected by the respective detection technology (*e.g*., mass spectrometry, infra-red spectrometry, potentiostatic amperometry or UV spectrometry).

### 8. Application of Cleavable Tags to Genotyping and Polymorphism Detection

### a. Introduction

Although a few known human DNA polymorphisms are based upon insertions, deletions or other rearrangements of non-repeated sequences, the vast majority are based either upon single base substitutions or upon variations in the number of tandem repeats. Base substitutions are very abundant in the human genome, occurring on average once every 200-500 bp. Length variations in blocks of tandem repeats are also common in the genome, with at least tens of thousands of interspersed polymorphic sites (termed loci). Repeat lengths for tandem repeat polymorphisms range from 1 bp in (dA)ₙ(dT)ₙ sequences to at least 170 bp in α-satellite DNA. Tandem repeat polymorphisms can be divided into two major groups which consist of minisatellites/variable number of tandem repeats (VNTRs), with typical repeat lengths of tens of base pairs and with tens to thousands of total repeat units, and microsatellites, with repeat lengths of up to 6 bp and with maximum total lengths of about 70 bp. Most of the microsatellite polymorphisms identified to date have been based on (dC-dA)ₙ or (dG-dT)ₙ dinucleotide repeat sequences. Analysis of microsatellite polymorphisms involves amplification by the polymerase chain reaction (PCR) of a small fragment of DNA containing a block of repeats followed by electrophoresis of the amplified DNA on denaturing polyacrylamide gel. The PCR primers are complementary to unique sequences that flank the blocks of repeats. Polyacrylamide gels, rather than agarose gels, are traditionally used for microsatellites because the alleles often only differ in size by a single repeat.

Thus, methods are described for genotyping a selected organism, comprising the steps of (a) generating tagged nucleic acid molecules from a selected target molecule, wherein a tag is correlative with a particular fragment and may be detected by non-fluorescent spectrometry or potentiometry, (b) separating the tagged molecules by sequential length, (c) cleaving the tag from the tagged molecule, and (d) detecting the tag by non-fluorescent spectrometry or potentiometry, and therefrom determining the genotype of the organism.

Within another aspect, methods are described for genotyping a selected organism, comprising the steps of (a) combining a tagged nucleic acid molecule with a selected target molecule under conditions and for a time sufficient to permit hybridization of the tagged molecule to the target molecule, wherein a tag is correlative with a particular fragment and may be detected by non-fluorescent spectrometry or potentiometry, (b) separating the tagged fragments by sequential length, (c) cleaving the tag from the tagged fragment, and (d) detecting the tag by non-fluorescent spectrometry or potentiometry, and therefrom determining the genotype of the organism.

### b. Application of cleavable tags to genotyping.

A PCR approach to identify restriction fragment length polymorphism (RFPL) combines gel electrophoresis and detection of tags assoicated with specific PCR primers. In general, one PCR primer will possess one specific tag. The tag will therefore represent one set of PCR primers and therefore a pre-determined DNA fragment length. Polymorphisms are detected as variations in the lengths of the labeled fragments in a gel or eluting from a gel. Polyacrylamide gel electrophoresis will usually afford the resolution necessary to distinguish minisatellite/VNTR alleles differing by a single repeat unit. Analysis of microsatellite polymorphisms involves amplification by the polymerase chain reaction (PCR) of a small fragment of DNA containing a block of repeats followed by electrophoresis of the amplified DNA on denaturing polyacrylamide gel or followed by separation of DNA fragments by HPLC The amplified DNA will be labeled using primers that have cleavable tags at the 5' end of the primer. The primers are incorporated into the newly synthesized strands by chain extension. The PCR primers are complementary to unique sequences that flank the blocks of repeats. Minisatellite/VNTR polymorphisms can also be amplified, much as with the microsatellites described above.

Descriptions of many types of DNA sequence polymorphisms have provided the fundamental basis for the understanding of the structure of the human genome (Botstein et al., Am. J. Human Genetics 32:p314, 1980; Donis-Keller, Cell 51:319, 1987; Weissenbach et al., Nature 359:794). The construction of extensive framework linkage maps has been facilitated by the use of these DNA polymorphisms and has provided a practical means for localization of disease genes by linkage. Microsatellite dinucleotide markers are proving to be very powerful tools in the identification of human genes which have been shown to contain mutations and in some instances cause disease. Genomic dinucleotide repeats are highly polymorphic (Weber, 1990, Genomic Analysis, Vol 1, pp 159-181, Cold Spring Laboratory Press, Cold Spring Harbor, NY; Weber and Wong, 1993, Hum. Mol. Genetics, 2, p1123) and may possess up to 24 alleles. Microsatellite dinucleotide repeats can be amplified using primers complementary to the unique regions surrounding the dinucleotide repeat by PCR. Following amplification, several amplified loci and be combined (multiplexed) prior to a size separation step. The process of applying the amplified microsatellite fragments to a size separation step and then identifying the size and therefore the allele is known as genotyping. Chromosome specific markers which permit a high level of multiplexing have been reported for performing whole genome scans for linkage analysis (Davies et al., 1994, Nature, 371, p130).

Tags can be used to great effect in genotyping with microsatellites. Briefly, the PCR primers are constructed to carry tags and used in a carefully chosen PC reaction to amplify di-, tri-, or tetra- nucleotide repeats. The amplification products are then separated according to size by methods such as HPLC or PAGE. The DNA fragments are then collected in a temporal fashion, the tags cleaved from their respective DNA fragments and length deduced from comparison to internal standards in the size separation step. Allele identification is made from reference to size of the amplified products.

With cleavable tags approach to genotyping, it is possible to combine multiple samples on a single separation step. There are two general ways in which this can performed. The first general method for high through-put screening is the detection of a single polymorphism in a large group of individuals. In this senario a single or nested set of PCR primers is used and each amplification is done with one DNA sample type per reaction. The number of samples that can be combined in the separation step is proportional to the number of cleavable tags that can be generated per detection technology (*i.e.,* 400-600 for mass spectrometer tags). It is therefore possible to identify 1 to several polymorphisms in a large group of individuals simultaneously. The second approach is to use multiple sets of PCR primers which can identify numerous polymorphisms on a single DNA sample (genotyping an individual for example). In this approach PCR primers are combined in a single amplification reaction which generate PCR products of different length. Each primer pair or nested set is encoded by a specific cleavable Tag which implies each PCR fragment will be encoded witha specific tag. The reaction is run on a single separation step (see below). The number of samples that can be combined in the separation step is proportional to the number of cleavable tags that can be generated per detection technology (*i.e.,* 400-600 for mass spectrometer tags).

### c. Enzymatic detection of mutation and the applications of tags.

In this particular application or method, mismatches in heteroduplexes are detected by enzymatic cleavage of mismatched base pairs in a given nucleic acid duplex. DNA sequences to be tested for the presence of a mutation are amplified by PCR using a specific set of primers, the amplified products are denatured and mixed with denatured reference fragments and hybridized which result in the formation of heteroduplexes. The heteroduplexes are then treated with enzymes which recognize and cleave the duplex if a mismatch is present. Such enzymes are nuclease S1, Mung bean nuclease, "resolvases", T4 endonuclease IV, etc. Essentially any enzyme can be used which recognizes mismatches *in vitro* and cleave the resulting mismatch. The treatment with the appropriate enzyme, the DNA duplexes are separated by size, by, for example HPLC or PAGE. The DNA fragments are collected temporally. Tags are cleaved and detected. The presence of a mutation is detected by the shift in mobility of a fragments relative to a wild-type reference fragment.

### d. Applications of tags to the oligonucleotide ligation assay (OLA).

The oligonucleotide ligation assay as originally described by Landegren et al. (Landegen et al., Science 241:487, 1988) is a useful technique for the identification of sequences (known) in very large and complex genomes. The principle of the OLA reaction is based on the ability of ligase to covalently join two diagnostic oligonucleotides as they hybridize adjacent to one another on a given DNA target. If the sequences at the probe junctions are not perfectly based-paired, the probes will not be joined by the ligase. The ability of a thermostable ligase to discriminate potential single base-pair differences when positioned at the 3' end of the "upstream" probe provides the opportunity for single base-pair resolution (Barony, PNAS USA 88:189, 1991). In the application of tags, the tags would be attached the probe which is ligated to the amplified product. After completion of the OLR, the fragments are separated on the basis of size, the tags cleaved and detected by mass spectrometry.

### e. Sequence specific amplification.

PCR primers with a 3' end complementary either to a mutant or normal oligonucleotide sequence can be used to selectively amplify one or the other allele (Newton et al., Nuc. Acids Res., 17, p2503; et al., 1989, Genomics, 5, p535; Okayama et al., 1989, J. Lab. Clin. Med., 114, p105; Sommer et al., 1989, Mayo Clin.Proc., 64, 1361; Wu et al., PNAS USA, 86, p2757). Usually the PCR products are visualized after amplification by PAGE, but the principle of sequence specific amplification can be applied to solid phase formats.

### f. Potential application of tags to some amplification based assays.

Genotyping of viruses: A potential application of tags is the genotyping or identification of viruses by hybridization with tagged probes. For example, F+ RNA coliphages may be useful candidates as indicators for enteric virus contamination. Genotyping by nucleic acid hybridization methods is a reliable, rapid, simple, and inexpensive alternative to serotyping (Kafatos et. al., Nucleic Acids Res. 7:1541, 1979). Amplification techniques and nucleic aid hybridization techniques have been successfully used to classify a variety of microorganisms including E. coli (Feng, Mol. Cell Probes 7:151. 1993), rotavirus (Sethabutr et. al., J. Med Virol. 37:192, 1992), hepatitis C virus (Stuyver et. al., J. Gen Virol. 74:1093, 1993), and herpes simplex virus (Matsumoto et. al., J. Virol. Methods 40:119, 1992).

Prognostic applications of mutational analysis in cancers: Genetic alterations have been described in a variety of experimental mammalian and human neoplasms and represent the morphological basis for the sequence of morphological alterations observed in carcinogenesis (Vogelstein et al., NEJM 319:525, 1988). In recent years with the advent of molecular biology techniques, allelic losses on certain chromosomes or mutation of tumor suppressor genes as well as mutations in several oncogenes (*e.g*., c-myc, c-jun, and the ras family) have been the most studied entities. Previous work (Finkelstein et al., Arch Surg. 128:526, 1993) has identified a correlation between specific types of point mutations in the K-ras oncogene and the stage at diagnosis in colorectal carcinoma. The results suggested that mutational analysis could provide important information of tumor aggressiveness, including the pattern and spread of metastasis. The prognostic value of TP53 and K-ras-2 mutational analysis in stage III carconoma of the colon has more recently been demonstrated (Pricolo et al., Am. J. Surg. 171:41, 1996). It is therefore apparent that genotyping of tumors and pre-cancerous cells, and specific mutation detection will become increasingly important in the treatment of cancers in humans.

### C. SEPARATION OF NUCLEIC ACID FRAGMENTS

A sample that requires analysis is often a mixture of many components in a complex matrix. For samples containing unknown compounds, the components must be separated from each other so that each individual component can be identified by other analytical methods. The separation properties of the components in a mixture are constant under constant conditions, and therefore once determined they can be used to identify and quantify each of the components. Such procedures are typical in chromatographic and electrophoretic analytical separations.

### 1. High-Performance Liquid Chromatography (HPLC)

High-Performance liquid chromatography (HPLC) is a chromatographic separations technique to separate compounds that are dissolved in solution. HPLC instruments consist of a reservoir of mobile phase, a pump, an injector, a separation column, and a detector. Compounds are separated by injecting an aliquot of the sample mixture onto the column. The different components in the mixture pass through the column at different rates due to differences in their partitioning behavior between the mobile liquid phase and the stationary phase.

Recently, IP-RO-HPLC on non-porous PS/DVB particles with chemically bonded alkyl chains have been shown to be rapid alternatives to capillary electrophoresis in the analysis of both single and double-strand nucleic acids providing similair degrees of resolution (Huber et al, Anal. Biochem. 212:351, 1993; Huber et al., 1993, Nuc. Acids Res. 21:1061; Huber et al., Biotechniques 16:898, 1993). In contrast to ion-excahnge chromoatrography, which does not always retain double-strand DNA as a function of strand length (Since AT base pairs intereact with the positively charged stationary phase, more strongly than GC base-pairs), IP-RP-HPLC enables a strictly size-dependent separation.

A method has been developed using 100 mM triethylammonium acetate as ion-pairing reagent, phosphodiester oligonucleotides could be successfully separated on alkylated non-porous 2.3 µM poly(styrene-divinylbenzene) particles by means of high performance liquid chromatography (Oefner et al., Anal. Biochem. 223:39, 1994). The technique described allowed the separation of PCR products differing only 4 to 8 base pairs in length within a size range of 50 to 200 nucleotides.

### 2. Electrophoresis

Electrophoresis is a separations technique that is based on the mobility of ions (or DNA as is the case described herein) in an electric field. Negatively charged DNA charged migrate towards a positive electrode and positively-charged ions migrate toward a negative electrode. For safety reasons one electrode is usually at ground and the other is biased positively or negatively. Charged species have different migration rates depending on their total charge, size, and shape, and can therefore be separated. An electrode apparatus consists of a high-voltage power supply, electrodes, buffer, and a support for the buffer such as a polyacrylamide gel, or a capillary tube. Open capillary tubes are used for many types of samples and the other gel supports are usually used for biological samples such as protein mixtures or DNA fragments.

### 3. Capillary Electrophoresis (CE)

Capillary electrophoresis (CE) in its various manifestations (free solution, isotachophoresis, isoelectric focusing, polyacrylamide gel, micellar electrokinetic "chromatography") is developing as a method for rapid high resolution separations of very small sample volumes of complex mixtures. In combination with the inherent sensitivity and selectivity of MS, CE-MS is a potential powerful technique for bioanalysis. In the novel application disclosed herein, the interfacing of these two methods will lead to superior DNA sequencing methods that eclipse the current rate methods of sequencing by several orders of magnitude.

The correspondence between CE and electrospray ionization (ESI) flow rates and the fact that both are facilitated by (and primarily used for) ionic species in solution provide the basis for an extremely attractive combination. The combination of both capillary zone electrophoresis (CZE) and capillary isotachophoresis with quadrapole mass spectrometers based upon ESI have been described (Olivares et al., Anal. Chem. 59:1230, 1987; Smith et al., Anal. Chem. 60:436, 1988; Loo et al., Anal. Chem. 179:404, 1989; Edmonds et al., J. Chroma. 474:21, 1989; Loo et al., J. Microcolumn Sep. 1:223, 1989; Lee et al., J. Chromatog. 458:313, 1988; Smith et al., J. Chromatog. 480:211, 1989; Grese et al., J. Am. Chem. Soc. 111:2835, 1989). Small peptides are easily amenable to CZE analysis with good (femtomole) sensitivity.

The most powerful separation method for DNA fragments is polyacrylamide gel electrophoresis (PAGE), generally in a slab gel format. However, the major limitation of the current technology is the relatively long time required to perform the gel electrophoresis of DNA fragments produced in the sequencing reactions. An increase magnitude (10-fold) can be achieved with the use of capillary electrophoresis which utilize ultrathin gels. In free solution to a first approximation all DNA migrate with the same mobility as the addition of a base results in the compensation of mass and charge. In polyacrylamide gels, DNA fragments sieve and migrate as a function of length and this approach has now been applied to CE. Remarkable plate number per meter has now been achieved with cross-linked polyacrylamide (10⁺⁷ plates per meter, Cohen et al., Proc. Natl. Acad Sci., USA 85:9660, 1988). Such CE columns as described can be employed for DNA sequencing. The method of CE is in principle 25 times faster than slab gel electrophoresis in a standard sequencer. For example, about 300 bases can be read per hour. The separation speed is limited in slab gel electrophoresis by the magnitude of the electric field which can be applied to the gel without excessive heat production. Therefore, the greater speed of CE is achieved through the use of higher field strengths (300 V/cm in CE versus 10 V/cm in slab gel electrophoresis). The capillary format reduces the amperage and thus power and the resultant heat generation.

Smith and others (Smith et al., Nuc. Acids. Res. 18:4417, 1990) have suggested employing multiple capillaries in parallel to increase throughput. Likewise, Mathies and Huang (Mathies and Huang, Nature 359:167, 1992) have introduced capillary electrophoresis in which separations are performed on a parallel array of capillaries and demonstrated high through-put sequencing (Huang et al., Anal. Chem. 64:967, 1992, Huang et al., Anal. Chem. 64:2149, 1992). The major disadvantage of capillary electrophoresis is the limited amount of sample that can be loaded onto the capillary. By concentrating a large amount of sample at the beginning of the capillary, prior to separation, loadability is increased, and detection levels can be lowered several orders of magnitude. The most popular method of preconcentration in CE is sample stacking. Sample stacking has recently been reviewed (Chien and Burgi, Anal. Chem. 64:489A, 1992). Sample stacking depends of the matrix difference, (pH, ionic strength) between the sample buffer and the capillary buffer, so that the electric field across the sample zone is more than in the capillary region. In sample stacking, a large volume of sample in a low concentration buffer is introduced for preconcentration at the head of the capillary column. The capillary is filled with a buffer of the same composition, but at higher concentration. When the sample ions reach the capillary buffer and the lower electric field, they stack into a concentrated zone. Sample stacking has increased detectabilities 1-3 orders of magnitude.

Another method of preconcentration is to apply isotachophoresis (ITP) prior to the free zone CE separation of analytes. ITP is an electrophoretic technique which allows microliter volumes of sample to be loaded on to the capillary, in contrast to the low nL injection volumes typically associated with CE. The technique relies on inserting the sample between two buffers (leading and trailing electrolytes) of higher and lower mobility respectively, than the analyte. The technique is inherently a concentration technique, where the analytes concentrate into pure zones migrating with the same speed. The technique is currently less popular than the stacking methods described above because of the need for several choices of leading and trailing electrolytes, and the ability to separate only cationic or anionic species during a separation process.

The heart of the DNA sequencing process is the remarkably selective electrophoretic separation of DNA or oligonucleotide fragments. It is remarkable because each fragment is resolved and differs by only nucleotide. Separations of up to 1000 fragments (1000 bp) have been obtained. A further advantage of sequencing with cleavable tags is as follows. There is no requirement to use a slab gel format when DNA fragments are separated by polyacrylamide gel electrophoresis when cleavable tags are employed. Since numerous samples are combined (4 to 2000) there is no need to run samples in parallel as is the case with current dye-primer or dye-terminator methods (*i.e.,* ABI373 sequencer). Since there is no reason to run parallel lanes, there is no reason to use a slab gel. Therefore, one can employ a tube gel format for the electrophoretic separation method. Grossman (Grossman et al., Genet. Anal. Tech. Appl. 9:9, 1992) have shown that considerable advantage is gained when a tube gel format is used in place of a slab gel format. This is due to the greater ability to dissipate Joule heat in a tube format compared to a slab gel which results in faster run times (by 50%), and much higher resolution of high molecular weight DNA fragments (greater than 1000 nt). Long reads are critical in genomic sequencing. Therefore, the use of cleavable tags in sequencing has the additional advantage of allowing the user to employ the most efficient and sensitive DNA separation method which also possesses the highest resolution.

### 4. Microfabricated Devices

Capillary electrophoresis (CE) is a powerful method for DNA sequencing, forensic analysis, PCR product analysis and restriction fragment sizing. CE is far faster than traditional slab PAGE since with capillary gels a far higher potential field can be applied. However, CE has the drawback of allowing only one sample to be processed per gel. The method combines the faster separations times of CE with the ability to analyze multiple samples in parallel. The underlying concept behind the use of microfabricated devices is the ability to increase the information density in electrophoresis by miniaturizing the lane dimension to about 100 micrometers. The electronics industry routinely uses microfabrication to make circuits with features of less than one micron in size. The current density of capillary arrays is limited the outside diameter of the capillary tube. Microfabrication of channels produces a higher density of arrays. Microfabrication also permits physical assemblies not possible with glass fibers and links the channels directly to other devices on a chip. Few devices have been constructed on microchips for separation technologies. A gas chromatograph (Terry et al., IEEE Trans. Electron Device, ED-26:1880, 1979) and a liquid chromatograph (Manz et al., Sens. Actuators B1:249, 1990) have been fabricated on silicon chips, but these devices have not been widely used. Several groups have reported separating fluorescent dyes and amino acids on microfabricated devices (Manz et al., J. Chromatography 593:253, 1992, Effenhauser et al., Anal. Chem. 65:2637, 1993). Recently Woolley and Mathies (Woolley and Mathies, Proc. Natl. Acad. Sci. 91:11348, 1994) have shown that photolithography and chemical etching can be used to make large numbers of separation channels on glass substrates. The channels are filled with hydroxyethyl cellulose (HEC) separation matrices. It was shown that DNA restriction fragments could be separated in as little as two minutes.

### D. CLEAVAGE OF TAGS

As described above, different linker designs will confer cleavability ("lability") under different specific physical or chemical conditions. Examples of conditions which serve to cleave various designs of linker include acid, base, oxidation, reduction, fluoride, thiol exchange, photolysis, and enzymatic conditions.

Examples of cleavable linkers that satisfy the general criteria for linkers listed above will be well known to those in the art and include those found in the catalog available from Pierce (Rockford, IL). Examples include:
- ethylene glycobis(succinimidylsuccinate) (EGS), an amine reactive cross-linking reagent which is cleavable by hydroxylamine (1 M at 37°C for 3-6 hours);
- disuccinimidyl tartarate (DST) and sulfo-DST, which are amine reactive cross-linking reagents, cleavable by 0.015 M sodium periodate;
- bis[2-(succinimidyloxycarbonyloxy)ethyl]sulfone(BSOCOES) and sulfo-BSOCOES, which are amine reactive cross-linking reagents, cleavable by base (pH 11.6);
- 1,4-di-[3'-(2'-pyridyldithio(propionamido))butane (DPDPB), a pyridyldithiol crosslinker which is cleavable by thiol exchange or reduction;
- N-[4-(p-azidosalicylamido)-butyl]-3'-(2'-pyridydithio)propionamide (APDP), a pyridyldithiol crosslinker which is cleavable by thiol exchange or reduction;
- bis-[beta-4-(azidosalicylamido)ethyl]-disulfide, a photoreactive crosslinker which is cleavable by thiol exchange or reduction;
- N-succinimidyl-(4-azidophenyl)-1,3'dithiopropionate (SADP), a photoreactive crosslinker which is cleavable by thiol exchange or reduction;
- sulfosuccinimidyl-2-(7-azido-4-methylcoumarin-3-acetamide)ethyl-1,3'-dithiopropionate (SAED), a photoreactive crosslinker which is cleavable by thiol exchange or reduction;
- sulfosuccinimidyl-2-(m-azido-o-nitrobenzamido)-ethyl-1,3'dithiopropionate (SAND), a photoreactive crosslinker which is cleavable by thiol exchange or reduction.

Other examples of cleavable linkers and the cleavage conditions that can be used to release tags are as follows. A silyl linking group can be cleaved by fluoride or under acidic conditions. A 3-, 4-, 5-, or 6-substituted-2-nitrobenzyloxy or 2-, 3-, 5-, or 6-substituted-4-nitrobenzyloxy linking group can be cleaved by a photon source (photolysis). A 3-, 4-, 5-, or 6-substituted-2-alkoxyphenoxy or 2-, 3-, 5-, or 6-substituted-4-alkoxyphenoxy linking group can be cleaved by Ce(NH₄)₂(NO₃)₆ (oxidation). A NCO₂ (urethane) linker can be cleaved by hydroxide (base), acid, or LiAlH₄ (reduction). A 3-pentenyl, 2-butenyl, or 1-butenyl linking group can be cleaved by O₃, O_{S}O₄/IO₄⁻, or KMnO₄ (oxidation). A 2-[3-, 4-, or 5-substituted-furyl]oxy linking group can be cleaved by O₂, Br₂, MeOH, or acid.

Conditions for the cleavage of other labile linking groups include: t-alkyloxy linking groups can be cleaved by acid; methyl(dialkyl)methoxy or 4-substituted-2-alkyl-1,3-dioxlane-2-yl linking groups can be cleaved by H₃O⁺; 2-silylethoxy linking groups can be cleaved by fluoride or acid; 2-(X)-ethoxy (where X = keto, ester amide, cyano, NO₂, sulfide, sulfoxide, sulfone) linking groups can be cleaved under alkaline conditions; 2-, 3-, 4-, 5-, or 6-substituted-benzyloxy linking groups can be cleaved by acid or under reductive conditions; 2-butenyloxy linking groups can be cleaved by (Ph₃P)₃RhCl(H), 3-, 4-, 5-, or 6-substituted-2-bromophenoxy linking groups can be cleaved by Li, Mg, or BuLi; methylthiomethoxy linking groups can be cleaved by Hg²⁺; 2-(X)-ethyloxy (where X = a halogen) linking groups can be cleaved by Zn or Mg; 2-hydroxyethyloxy linking groups can be cleaved by oxidation (*e.g.,* with Pb(OAc)₄).

Preferred linkers are those that are cleaved by acid or photolysis. Several of the acid-labile linkers that have been developed for solid phase peptide synthesis are useful for linking tags to MOIs. Some of these linkers are described in a recent review by Lloyd-Williams et al. (Tetrahedron 49:11065-11133, 1993). One useful type of linker is based upon p-alkoxybenzyl alcohols, of which two, 4-hydroxymethylphenoxyacetic acid and 4-(4-hydroxymethyl-3-methoxyphenoxy)butyric acid, are commercially available from Advanced ChemTech (Louisville, KY). Both linkers can be attached to a tag via an ester linkage to the benzylalcohol, and to an amine-containing MOI via an amide linkage to the carboxylic acid. Tags linked by these molecules are released from the MOI with varying concentrations of trifluoroacetic acid. The cleavage of these linkers results in the liberation of a carboxylic acid on the tag. Acid cleavage of tags attached through related linkers, such as 2,4-dimethoxy-4'-(carboxymethyloxy)-benzhydrylamine (available from Advanced ChemTech in FMOC-protected form), results in liberation of a carboxylic amide on the released tag.

The photolabile linkers useful for this application have also been for the most part developed for solid phase peptide synthesis (see Lloyd-Williams review). These linkers are usually based on 2-nitrobenzylesters or 2-nitrobenzylamides. Two examples of photolabile linkers that have recently been reported in the literature are 4-(4-(1-Fmoc-amino)ethyl)-2-methoxy-5-nitrophenoxy)butanoic acid (Holmes and Jones, J. Org. Chem. 60:2318-2319, 1995) and 3-(Fmoc-amino)-3-(2-nitrophenyl)propionic acid (Brown et al., Molecular Diversity 1:4-12, 1995). Both linkers can be attached via the carboxylic acid to an amine on the MOI. The attachment of the tag to the linker is made by forming an amide between a carboxylic acid on the tag and the amine on the linker. Cleavage of photolabile linkers is usually performed with UV light of 350 nm wavelength at intensities and times known to those in the art. Examples of commercial sources of instruments for photochemical cleavage are Aura Industries Inc. (Staten Island, NY) and Agrenetics (Wilmington, MA). Cleavage of the linkers results in liberation of a primary amide on the tag. Examples of photocleavable linkers include nitrophenyl glycine esters, exo- and endo-2-benzonorborneyl chlorides and methane sulfonates, and 3-amino-3(2-nitrophenyl) propionic acid. Examples of enzymatic cleavage include esterases which will cleave ester bonds, nucleases which will cleave phosphodiester bonds, proteases which cleave peptide bonds, etc.

### E. DETECTION OF TAGS

Detection methods typically rely on the absorption and emission in some type of spectral field. When atoms or molecules absorb light, the incoming energy excites a quantized structure to a higher energy level. The type of excitation depends on the wavelength of the light. Electrons are promoted to higher orbitals by ultraviolet or visible light, molecular vibrations are excited by infrared light, and rotations are excited by microwaves. An absorption spectrum is the absorption of light as a function of wavelength. The spectrum of an atom or molecule depends on its energy level structure. Absorption spectra are useful for identification of compounds. Specific absorption spectroscopic methods include atomic absorption spectroscopy (AA), infrared spectroscopy (IR), and UV-vis spectroscopy (uv-vis).

Atoms or molecules that are excited to high energy levels can decay to lower levels by emitting radiation. This light emission is called fluorescence if the transition is between states of the same spin, and phosphorescence if the transition occurs between states of different spin. The emission intensity of an analyte is linearly proportional to concentration (at low concentrations), and is useful for quantifying the emitting species. Specific emission spectroscopic methods include atomic emission spectroscopy (AES), atomic fluorescence spectroscopy (AFS), molecular laser-induced fluorescence (LIF), and X-ray fluorescence (XRF).

When electromagnetic radiation passes through matter, most of the radiation continues in its original direction but a small fraction is scattered in other directions. Light that is scattered at the same wavelength as the incoming light is called Rayleigh scattering. Light that is scattered in transparent solids due to vibrations (phonons) is called Brillouin scattering. Brillouin scattering is typically shifted by 0.1 to 1 wave number from the incident light. Light that is scattered due to vibrations in molecules or optical phonons in opaque solids is called Raman scattering. Raman scattered light is shifted by as much as 4000 wavenumbers from the incident light. Specific scattering spectroscopic methods include Raman spectroscopy.

IR spectroscopy is the measurement of the wavelength and intensity of the absorption of mid-infrared light by a sample. Mid-infrared light (2.5 - 50 µm, 4000 - 200 cm⁻¹) is energetic enough to excite molecular vibrations to higher energy levels. The wavelength of IR absorption bands are characteristic of specific types of chemical bonds and IR spectroscopy is generally most useful for identification of organic and organometallic molecules.

Near-infrared absorption spectroscopy (NIR) is the measurement of the wavelength and intensity of the absorption of near-infrared light by a sample. Near-infrared light spans the 800 nm - 2.5 µm (12,500 - 4000 cm⁻¹) range and is energetic enough to excite overtones and combinations of molecular vibrations to higher energy levels. NIR spectroscopy is typically used for quantitative measurement of organic functional groups, especially O-H, N-H, and C=O. The components and design of NIR instrumentation are similar to uv-vis absorption spectrometers. The light source is usually a tungsten lamp and the detector is usually a PbS solid-state detector. Sample holders can be glass or quartz and typical solvents are CCl₄ and CS₂. The convenient instrumentation of NIR spectroscopy makes it suitable for on-line monitoring and process control.

Ultraviolet and Visible Absorption Spectroscopy (uv-vis) spectroscopy is the measurement of the wavelength and intensity of absorption of near-ultraviolet and visible light by a sample. Absorption in the vacuum UV occurs at 100-200 nm; (10⁵-50,000 cm⁻¹) quartz UV at 200-350 nm; (50,000-28,570 cm⁻¹) and visible at 350-800 nm; (28,570-12,500 cm⁻¹) and is described by the Beer-Lambert-Bouguet law. Ultraviolet and visible light are energetic enough to promote outer electrons to higher energy levels. UV-vis spectroscopy can be usually applied to molecules and inorganic ions or complexes in solution. The uv-vis spectra are limited by the broad features of the spectra. The light source is usually a hydrogen or deuterium lamp for uv measurements and a tungsten lamp for visible measurements. The wavelengths of these continuous light sources are selected with a wavelength separator such as a prism or grating monochromator. Spectra are obtained by scanning the wavelength separator and quantitative measurements can be made from a spectrum or at a single wavelength.

Mass spectrometers use the difference in the mass-to-charge ratio (m/z) of ionized atoms or molecules to separate them from each other. Mass spectrometry is therefore useful for quantitation of atoms or molecules and also for determining chemical and structural information about molecules. Molecules have distinctive fragmentation patterns that provide structural information to identify compounds. The general operations of a mass spectrometer are as follows. Gas-phase ions are created, the ions are separated in space or time based on their mass-to-charge ratio, and the quantity of ions of each mass-to-charge ratio is measured. The ion separation power of a mass spectrometer is described by the resolution, which is defined as R = m / delta m, where m is the ion mass and delta m is the difference in mass between two resolvable peaks in a mass spectrum. For example, a mass spectrometer with a resolution of 1000 can resolve an ion with a m/z of 100.0 from an ion with a m/z of 100.1.

In general, a mass spectrometer (MS) consists of an ion source, a mass-selective analyzer, and an ion detector. The magnetic-sector, quadrupole, and time-of-flight designs also require extraction and acceleration ion optics to transfer ions from the source region into the mass analyzer. The details of several mass analyzer designs (for magnetic-sector MS, quadrupole MS or time-of-flight MS) are discussed below. Single Focusing analyzers for magnetic-sector MS utilize a particle beam path of 180, 90, or 60 degrees. The various forces influencing the particle separate ions with different mass-to-charge ratios. With double-focusing analyzers, an electrostatic analyzer is added in this type of instrument to separate particles with difference in kinetic energies.

A quadrupole mass filter for quadrupole MS consists of four metal rods arranged in parallel. The applied voltages affect the trajectory of ions traveling down the flight path centered between the four rods. For given DC and AC voltages, only ions of a certain mass-to-charge ratio pass through the quadrupole filter and all other ions are thrown out of their original path. A mass spectrum is obtained by monitoring the ions passing through the quadrupole filter as the voltages on the rods are varied.

A time-of-flight mass spectrometer uses the differences in transit time through a "drift region" to separate ions of different masses. It operates in a pulsed mode so ions must be produced in pulses and/or extracted in pulses. A pulsed electric field accelerates all ions into a field-free drift region with a kinetic energy of qV, where q is the ion charge and V is the applied voltage. Since the ion kinetic energy is 0.5 mV², lighter ions have a higher velocity than heavier ions and reach the detector at the end of the drift region sooner. The output of an ion detector is displayed on an oscilloscope as a function of time to produce the mass spectrum.

The ion formation process is the starting point for mass spectrometric analyses. Chemical ionization is a method that employs a reagent ion to react with the analyte molecules (tags) to form ions by either a proton or hydride transfer. The reagent ions are produced by introducing a large excess of methane (relative to the tag) into an electron impact (EI) ion source. Electron collisions produce CH₄⁺ and CH₃⁺ which further react with methane to form CH₅⁺ and C₂H₅⁺. Another method to ionize tags is by plasma and glow discharge. Plasma is a hot, partially-ionized gas that effectively excites and ionizes atoms. A glow discharge is a low-pressure plasma maintained between two electrodes. Electron impact ionization employs an electron beam, usually generated from a tungsten filament, to ionize gas-phase atoms or molecules. An electron from the beam knocks an electron off analyte atoms or molecules to create ions. Electrospray ionization utilizes a very fine needle and a series of skimmers. A sample solution is sprayed into the source chamber to form droplets. The droplets carry charge when the exit the capillary and as the solvent vaporizes the droplets disappear leaving highly charged analyte molecules. ESI is particularly useful for large biological molecules that are difficult to vaporize or ionize. Fast-atom bombardment (FAB) utilizes a high-energy beam of neutral atoms, typically Xe or Ar, that strikes a solid sample causing desorption and ionization. It is used for large biological molecules that are difficult to get into the gas phase. FAB causes little fragmentation and usually gives a large molecular ion peak, making it useful for molecular weight determination. The atomic beam is produced by accelerating ions from an ion source though a charge-exchange cell. The ions pick up an electron in collisions with neutral atoms to form a beam of high energy atoms. Laser ionization (LIMS) is a method in which a laser pulse ablates material from the surface of a sample and creates a microplasma that ionizes some of the sample constituents. Matrix-assisted laser desorption ionization (MALDI) is a LIMS method of vaporizing and ionizing large biological molecules such as proteins or DNA fragments. The biological molecules are dispersed in a solid matrix such as nicotinic acid. A UV laser pulse ablates the matrix which carries some of the large molecules into the gas phase in an ionized form so they can be extracted into a mass spectrometer. Plasma-desorption ionization (PD) utilizes the decay of ²⁵²Cf which produces two fission fragments that travel in opposite directions. One fragment strikes the sample knocking out 1-10 analyte ions. The other fragment strikes a detector and triggers the start of data acquisition. This ionization method is especially useful for large biological molecules. Resonance ionization (RIMS) is a method in which one or more laser beams are tuned in resonance to transitions of a gas-phase atom or molecule to promote it in a stepwise fashion above its ionization potential to create an ion. Secondary ionization (SIMS) utilizes an ion beam; such as ³He⁺, ¹⁶O+, or ⁴⁰Ar⁺; is focused onto the surface of a sample and sputters material into the gas phase. Spark source is a method which ionizes analytes in solid samples by pulsing an electric current across two electrodes.

A tag may become charged prior to, during or after cleavage from the molecule to which it is attached. Ionization methods based on ion "desorption", the direct formation or emission of ions from solid or liquid surfaces have allowed increasing application to nonvolatile and thermally labile compounds. These methods eliminate the need for neutral molecule volatilization prior to ionization and generally minimize thermal degradation of the molecular species. These methods include field desorption (Becky, Principles of Field Ionization and Field Desorption Mass Spectrometry, Pergamon, Oxford, 1977), plasma desorption (Sundqvist and Macfarlane, Mass Spectrom. Rev. 4:421, 1985), laser desorption (Karas and Hillenkamp, Anal. Chem. 60:2299. 1988; Karas et al., Angew. Chem. 101:805, 1989), fast particle bombardment (*e.g.,* fast atom bombardment, FAB, and secondary ion mass spectrometry, SIMS, Barber et al., Anal. Chem. 54:645A, 1982), and thermospray (TS) ionization (Vestal, Mass Spectrom. Rev. 2:447, 1983). Thermospray is broadly applied for the on-line combination with liquid chromatography. The continuous flow FAB methods (Caprioli et al., Anal. Chem. 58:2949, 1986) have also shown significant potential. A more complete listing of ionization/mass spectrometry combinations is ion-trap mass spectrometry, electrospray ionization mass spectrometry, ion-spray mass spectrometry, liquid ionization mass spectrometry, atmospheric pressure ionization mass spectrometry, electron ionization mass spectrometry, metastable atom bombardment ionization mass spectrometry, fast atom bombard ionization mass spectrometry, MALDI mass spectrometry, , photo-ionization time-of-flight mass spectrometry, laser droplet mass spectrometry, MALDI-TOF mass spectrometry, APCI mass spectrometry, nano-spray mass spectrometry, nebulised spray ionization mass spectrometry, chemical ionization mass spectrometry, resonance ionization mass spectrometry, secondary ionization mass spectrometry, thermospray mass spectrometry.

The ionization methods amenable to nonvolatile biological compounds have overlapping ranges of applicability. Ionization efficiencies are highly dependent on matrix composition and compound type. Currently available results indicate that the upper molecular mass for TS is about 8000 daltons (Jones and Krolik, Rapid Comm. Mass Spectrom. 1:67, 1987). Since TS is practiced mainly with quadrapole mass spectrometers, sensitivity typically suffers disporportionately at higher mass-to-charge ratios (m/z). Time-of-flight (TOF) mass spectrometers are commercially available and possess the advantage that the m/z range is limited only by detector efficiency. Recently, two additional ionization methods have been introduced. These two methods are now referred to as matrix-assisted laser desorption (MALDI, Karas and Hillenkamp, Anal. Chem. 60:2299, 1988; Karas et al., Angew. Chem. 101:805, 1989) and electrospray ionization (ESI). Both methodologies have very high ionization efficiency (*i.e.,* very high [molecular ions produced]/[molecules consumed]). Sensitivity, which defines the ultimate potential of the technique, is dependent on sample size, quantity of ions, flow rate, detection efficiency and actual ionization efficiency.

Electrospray-MS is based on an idea first proposed in the 1960s (Dole et al., J. Chem. Phys. 49:2240, 1968). Electrospray ionization (ESI) is one means to produce charged molecules for analysis by mass spectroscopy. Briefly, electrospray ionization produces highly charged droplets by nebulizing liquids in a strong electrostatic field. The highly charged droplets, generally formed in a dry bath gas at atmospheric pressure, shrink by evaporation of neutral solvent until the charge repulsion overcomes the cohesive forces, leading to a "Coulombic explosion". The exact mechanism of ionization is controversial and several groups have put forth hypotheses (Blades et al., Anal. Chem. 63:2109-14, 1991; Kebarle et al., Anal. Chem. 65:A972-86, 1993; Fenn, J. Am. Soc. Mass. Spectrom. 4:524-35, 1993). Regardless of the ultimate process of ion formation, ESI produces charged molecules from solution under mild conditions.

The ability to obtain useful mass spectral data on small amounts of an organic molecule relies on the efficient production of ions. The efficiency of ionization for ESI is related to the extent of positive charge associated with the molecule. Improving ionization experimentally has usually involved using acidic conditions. Another method to improve ionization has been to use quaternary amines when possible (see Aebersold et al., Protein Science 1:494-503, 1992; Smith et al., Anal. Chem. 60:436-41, 1988).

Electrospray ionization is described in more detail as follows. Electrospray ion production requires two steps: dispersal of highly charged droplets at near atmospheric pressure, followed by conditions to induce evaporation. A solution of analyte molecules is passed through a needle that is kept at high electric potential. At the end of the needle, the solution disperses into a mist of small highly charged droplets containing the analyte molecules. The small droplets evaporate quickly and by a process of field desorption or residual evaporation, protonated protein molecules are released into the gas phase. An electrospray is generally produced by application of a high electric field to a small flow of liquid (generally 1-10 uL/min) from a capillary tube. A potential difference of 3-6 kV is typically applied between the capillary and counter electrode located 0.2-2 cm away (where ions, charged clusters, and even charged droplets, depending on the extent of desolvation, may be sampled by the MS through a small orifice). The electric field results in charge accumulation on the liquid surface at the capillary terminus; thus the liquid flow rate, resistivity, and surface tension are important factors in droplet production. The high electric field results in disruption of the liquid surface and formation of highly charged liquid droplets. Positively or negatively charged droplets can be produced depending upon the capillary bias. The negative ion mode requires the presence of an electron scavenger such as oxygen to inhibit electrical discharge.

A wide range of liquids can be sprayed electrostatically into a vacuum, or with the aid of a nebulizing agent. The use of only electric fields for nebulization leads to some practical restrictions on the range of liquid conductivity and dielectric constant. Solution conductivity of less than 10⁻⁵ ohms is required at room temperature for a stable electrospray at useful liquid flow rates corresponding to an aqueous electrolyte solution of < 10⁻⁴ M. In the mode found most useful for ESI-MS, an appropriate liquid flow rate results in dispersion of the liquid as a fine mist. A short distance from the capillary the droplet diameter is often quite uniform and on the order of 1 µm. Of particular importance is that the total electrospray ion current increases only slightly for higher liquid flow rates. There is evidence that heating is useful for manipulating the electrospray. For example, slight heating allows aqueous solutions to be readily electrosprayed, presumably due to the decreased viscosity and surface tension. Both thermally-assisted and gas-nebulization-assisted electrosprays allow higher liquid flow rates to be used, but decrease the extent of droplet charging. The formation of molecular ions requires conditions effecting evaporation of the initial droplet population. This can be accomplished at higher pressures by a flow of dry gas at moderate temperatures (<60°C), by heating during transport through the interface, and (particularly in the case of ion trapping methods) by energetic collisions at relatively low pressure.

Although the detailed processes underlying ESI remain uncertain, the very small droplets produced by ESI appear to allow almost any species carrying a net charge in solution to be transferred to the gas phase after evaporation of residual solvent. Mass spectrometric detection then requires that ions have a tractable m/z range (<4000 daltons for quadrupole instruments) after desolvation, as well as to be produced and transmitted with sufficient efficiency. The wide range of solutes already found to be amenable to ESI-MS, and the lack of substantial dependence of ionization efficiency upon molecular weight, suggest a highly non-discriminating and broadly applicable ionization process.

The electrospray ion "source" functions at near atmospheric pressure. The electrospray "source" is typically a metal or glass capillary incorporating a method for electrically biasing the liquid solution relative to a counter electrode. Solutions, typically water-methanol mixtures containing the analyte and often other additives such as acetic acid, flow to the capillary terminus. An ESI source has been described (Smith et al., Anal. Chem. 62:885, 1990) which can accommodate essentially any solvent system. Typical flow rates for ESI are 1-10 uL/min. The principal requirement of an ESI-MS interface is to sample and transport ions from the high pressure region into the MS as efficiently as possible.

The efficiency of ESI can be very high, providing the basis for extremely sensitive measurements, which is useful for the invention described herein. Current instrumental performance can provide a total ion current at the detector of about 2 x 10⁻¹² A or about 10⁷ counts/s for singly charged species. On the basis of the instrumental performance, concentrations of as low as 10⁻¹⁰ M or about 10⁻¹⁸ mol/s of a singly charged species will give detectable ion current (about 10 counts/s) if the analyte is completely ionized. For example, low attomole detection limits have been obtained for quaternary ammonium ions using an ESI interface with capillary zone electrophoresis (Smith et al., Anal. Chem. 59:1230, 1988). For a compound of molecular weight of 1000, the average number of charges is 1, the approximate number of charge states is 1, peak width (m/z) is 1 and the maximum intensity (ion/s) is 1 x 10¹².

Remarkably little sample is actually consumed in obtaining an ESI mass spectrum (Smith et al., Anal. Chem. 60:1948, 1988). Substantial gains might be also obtained by the use of array detectors with sector instruments, allowing simultaneous detection of portions of the spectrum. Since currently only about 10⁻⁵ of all ions formed by ESI are detected, attention to the factors limiting instrument performance may provide a basis for improved sensitivity. It will be evident to those in the art that the present invention contemplates and accommodates for improvements in ionization and detection methodologies.

An interface is preferably placed between the separation instrumentation (*e.g.,* gel)and the detector (*e.g.,* mass spectrometer). The interface preferably has the following properties: (1) the ability to collect the DNA fragments at discreet time intervals, (2) concentrate the DNA fragments, (3) remove the DNA fragments from the electrophoresis buffers and milieu, (4) cleave the tag from the DNA fragment, (5) separate the tag from the DNA fragment, (6) dispose of the DNA fragment, (7) place the tag in a volatile solution, (8) volatilize and ionize the tag, and (9) place or transport the tag to an electrospray device that introduces the tag into mass spectrometer.

The interface also has the capability of "collecting" DNA fragments as they elute from the bottom of a gel. The gel may be composed of a slab gel, a tubular gel, a capillary, etc. The DNA fragments can be collected by several methods. The first method is that of use of an electric field wherein DNA fragments are collected onto or near an electrode. A second method is that wherein the DNA fragments are collected by flowing a stream of liquid past the bottom of a gel. Aspects of both methods can be combined wherein DNA collected into a flowing stream which can be later concentrated by use of an electric field. The end result is that DNA fragments are removed from the milieu under which the separation method was performed. That is, DNA fragments can be "dragged" from one solution type to another by use of an electric field.

Once the DNA fragments are in the appropriate solution (compatible with electrospray and mass spectrometry) the tag can be cleaved from the DNA fragment. The DNA fragment (or remnants thereof) can then be separated from the tag by the application of an electric field (preferably, the tag is of opposite charge of that of the DNA tag). The tag is then introduced into the electrospray device by the use of an electric field or a flowing liquid.

Fluorescent tags can be identified and quantitated most directly by their absorption and fluorescence emission wavelengths and intensities.

While a conventional spectrofluorometer is extremely flexible, providing continuous ranges of excitation and emission wavelengths (1_{EX}, 1_{S1}, 1_{S2}), more specialized instruments such as flow cytometers and laser-scanning microscopes require probes that are excitable at a single fixed wavelength. In contemporary instruments, this is usually the 488-nm line of the argon laser.

Fluorescence intensity per probe molecule is proportional to the product of e and QY. The range of these parameters among fluorophores of current practical importance is approximately 10,000 to 100,000 cm⁻¹M⁻¹ for ε and 0.1 to 1.0 for QY. When absorption is driven toward saturation by high-intensity illumination, the irreversible destruction of the excited fluorophore (photobleaching) becomes the factor limiting fluorescence detectability. The practical impact of photobleaching depends on the fluorescent detection technique in question.

It will be evident to one in the art that a device (an interface) may be interposed between the separation and detection steps to permit the continuous operation of size separation and tag detection (in real time). This unites the separation methodology and instrumentation with the detection methodology and instrumentation forming a single device. For example, an interface is interposed between a separation technique and detection by mass spectrometry or potentiostatic amperometry.

The function of the interface is primarily the release of the (*e.g.,* mass spectrometry) tag from analyte. There are several representative implementations of the interface. The design of the interface is dependent on the choice of cleavable linkers. In the case of light or photo-cleavable linkers, an energy or photon source is required. In the case of an acid-labile linker, a base-labile linker, or a disulfide linker, reagent addition is required within the interface. In the case of heat-labile linkers, an energy heat source is required. Enzyme addition is required for an enzyme-sensitive linker such as a specific protease and a peptide linker, a nuclease and a DNA or RNA linker, a glycosylase, HRP or phosphatase and a linker which is unstable after cleavage (*e.g.,* similiar to chemiluminescent substrates). Other characteristics of the interface include minimal band broadening, separation of DNA from tags before injection into a mass spectrometer. Separation techniques include those based on electrophoretic methods and techniques, affinity techniques, size retention (dialysis), filtration and the like.

It is also possible to concentrate the tags (or nucleic acid-linker-tag construct), capture electrophoretically, and then release into alternate reagent stream which is compatible with the particular type of ionization method selected. The interface may also be capable of capturing the tags (or nucleic acid-linker-tag construct) on microbeads, shooting the bead(s) into chamber and then preforming laser desorption/vaporization. Also it is possible to extract in flow into alternate buffer (*e.g.,* from capillary electrophoresis buffer into hydrophobic buffer across a permeable membrane). It may also be desirable in some uses to deliver tags into the mass spectrometer intermittently which would comprise a further function of the interface. Another function of the interface is to deliver tags from multiple columns into a mass spectrometer, with a rotating time slot for each column. Also, it is possible to deliver tags from a single column into multiple MS detectors, separated by time, collect each set of tags for a few milliseconds, and then deliver to a mass spectrometer.

The following is a list of representative vendors for separation and detection technologies which may be used in the present invention. Hoefer Scientific Instruments (San Francisco, CA) manufactures electrophoresis equipment (Two Step™, Poker Face™ II) for sequencing applications. Pharmacia Biotech (Piscataway, NJ) manufactures electrophoresis equipment for DNA separations and sequencing (PhastSystem for PCR-SSCP analysis, MacroPhor System for DNA sequencing). Perkin Elmer/Applied Biosystems Division (ABI, Foster City, CA) manufactures semi-automated sequencers based on fluorescent-dyes (ABI373 and ABI377). Analytical Spectral Devices (Boulder, CO) manufactures UV spectrometers. Hitachi Instruments (Tokyo, Japan) manufactures Atomic Absorption spectrometers, Fluorescence spectrometers, LC and GC Mass Spectrometers, NMR spectrometers, and UV-VIS Spectrometers. PerSeptive Biosystems (Framingham, MA) produces Mass Spectrometers (Voyager^{™} Elite). Bruker Instruments Inc. (Manning Park, MA) manufactures FTIR Spectrometers (Vector 22), FT-Raman Spectrometers, Time of Flight Mass Spectrometers (Reflex II^{™}), Ion Trap Mass Spectrometer (Esquire^{™}) and a Maldi Mass Spectrometer. Analytical Technology Inc. (ATI, Boston, MA) makes Capillary Gel Electrophoresis units, UV detectors, and Diode Array Detectors. Teledyne Electronic Technologies (Mountain View, CA) manufactures an Ion Trap Mass Spectrometer (3DQ Discovery^{™} and the 3DQ Apogee^{™}). Perkin Elmer/Applied Biosystems Division (Foster City, CA) manufactures a Sciex Mass Spectrometer (triple quadrupole LC/MS/MS, the API 100/300) which is compatible with electrospray. Hewlett-Packard (Santa Clara, CA) produces Mass Selective Detectors (HP 5972A), MALDI-TOF Mass Spectrometers (HP G2025A), Diode Array Detectors, CE units, HPLC units (HP1090) as well as UV Spectrometers. Finnigan Corporation (San Jose, CA) manufactures mass spectrometers (magnetic sector (MAT 95 S™), quadrapole spectrometers (MAT 95 SQ™) and four other related mass spectrometers). Rainin (Emeryville, CA) manufactures HPLC instruments.

The methods and compositions described herein permit the use of cleaved tags to serve as maps to particular sample type and nucleotide identity. At the beginning of each sequencing method, a particular (selected) primer is assigned a particular unique tag. The tags map to either a sample type, a dideoxy terminator type (in the case of a Sanger sequencing reaction) or preferably both. Specifically, the tag maps to a primer type which in turn maps to a vector type which in turn maps to a sample identity. The tag may also may map to a dideoxy terminator type (ddTTP, ddCTP, ddGTP, ddATP) by reference into which dideoxynucleotide reaction the tagged primer is placed. The sequencing reaction is then performed and the resulting fragments are sequentially separated by size in time.

The tags are cleaved from the fragments in a temporal frame and measured and recorded in a temporal frame. The sequence is constructed by comparing the tag map to the temporal frame. That is, all tag identities are recorded in time after the sizing step and related become related to one another in a temporal frame. The sizing step separates the nucleic acid fragments by a one nucleotide increment and hence the related tag identities are separated by a one nucleotide increment. By foreknowledge of the dideoxy-terminator or nucleotide map and sample type, the sequence is readily deduced in a linear fashion.

The following examples are offered by way of illustration, and not by way of limitation.

Unless otherwise stated, chemicals as used in the examples may be obtained from Aldrich Chemical Company, Milwaukee, WI. The following abbreviations, with the indicated meanings, are used herein:
ANP = 3-(Fmoc-amino)-3-(2-nitrophenyl)propionic acid
NBA = 4-(Fmoc-aminomethyl)-3-nitrobenzoic acid
HATU = O-7-azabenzotriazol-1-yl-N,N,N',N'-tetramethyluronium hexafluorophosphate
DIEA = diisopropylethylamine
MCT = monochlorotriazine
NMM = 4-methylmorpholine
NMP = N-methylpyrrolidone
ACT357 = ACT357 peptide synthesizer from Advanced ChemTech, Inc., Louisville, KY
ACT = Advanced ChemTech, Inc., Louisville, KY
NovaBiochem = CalBiochem-NovaBiochem International, San Diego, CA
TFA = Trifluoroacetic acid
Tfa = Trifluoroacetyl
iNIP = N-Methylisonipecotic acid
Tfp = Tetrafluorophenyl
DIAEA = 2-(Diisopropylamino)ethylamine
MCT = monochlorotriazene
5'-AH-ODN = 5'-aminohexyl-tailed oligodeoxynucleotide

### EXAMPLES

### EXAMPLE 1

### PREPARATION OF ACID LABILE LINKERS FOR USE IN CLEAVABLE-MW-IDENTIFIER SEQUENCING

### A. Synthesis of Pentafluorophenyl Esters of Chemically Cleavable Mass Spectroscopy Tags, to Liberate Tags with Carboxyl Amide Termini

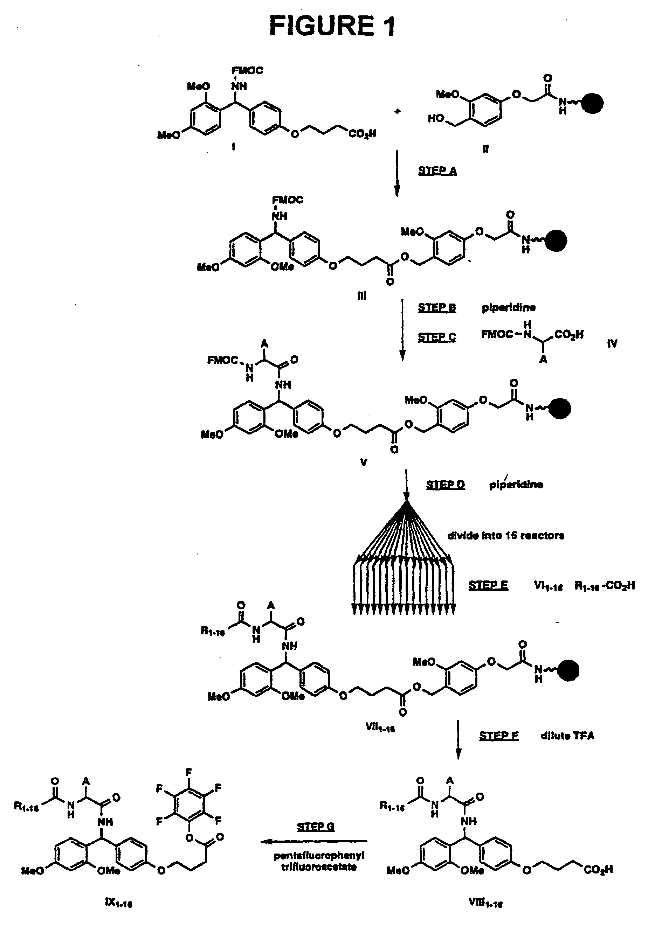

Figure 1 shows the reaction scheme.
Step A. TentaGel S AC resin (compound II; available from ACT; 1 eq.) is suspended with DMF in the collection vessel of the ACT357 peptide synthesizer (ACT). Compound I (3 eq.), HATU (3 eq.) and DIEA (7.5 eq.) in DMF are added and the collection vessel shaken for 1 hr. The solvent is removed and the resin washed with NMP (2X), MeOH (2X), and DMF (2X). The coupling of I to the resin and the wash steps are repeated, to give compound III.
Step B. The resin (compound III) is mixed with 25% piperidine in DMF and shaken for 5 min. The resin is filtered, then mixed with 25% piperidine in DMF and shaken for 10 min. The solvent is removed, the resin washed with NMP (2X), MeOH (2X), and DMF (2X), and used directly in step C.
Step C. The deprotected resin from step B is suspended in DMF and to it is added an FMOC-protected amino acid, containing amine functionality in its side chain (compound IV, *e.g.* alpha-N-FMOC-3-(3-pyridyl)-alanine, available from Synthetech, Albany, OR; 3 eq.), HATU (3 eq.), and DIEA (7.5 eq.) in DMF. The vessel is shaken for 1 hr. The solvent is removed and the resin washed with NMP (2X), MeOH (2X), and DMF (2X). The coupling of IV to the resin and the wash steps are repeated, to give compound V.
Step D. The resin (compound V) is treated with piperidine as described in step B to remove the FMOC group. The deprotected resin is then divided equally by the ACT357 from the collection vessel into 16 reaction vessels.
Step E. The 16 aliquots of deprotected resin from step D are suspended in DMF. To each reaction vessel is added the appropriate carboxylic acid VI₁₋₁₆ (R₁₋₁₆CO₂H; 3 eq.), HATU (3 eq.), and DIEA (7.5 eq.) in DMF. The vessels are shaken for 1 hr. The solvent is removed and the aliquots of resin washed with NMP (2X), MeOH (2X), and DMF (2X). The coupling of VI₁₋₁₆ to the aliquots of resin and the wash steps are repeated, to give compounds VII₁₋₁₆.
Step F. The aliquots of resin (compounds VII₁₋₁₆) are washed with CH₂Cl₂ (3X). To each of the reaction vessels is added 1% TFA in CH₂Cl₂ and the vessels shaken for 30 min. The solvent is filtered from the reaction vessels into individual tubes. The aliquots of resin are washed with CH₂Cl₂ (2X) and MeOH (2X) and the filtrates combined into the individual tubes. The individual tubes are evaporated *in vacuo,* providing compounds VIII₁₋₁₆.
Step G. Each of the free carboxylic acids VIII₁₋₁₆ is dissolved in DMF. To each solution is added pyridine (1.05 eq.), followed by pentafluorophenyl trifluoroacetate (1.1 eq.). The mixtures are stirred for 45 min. at room temperature. The solutions are diluted with EtOAc, washed with 1 M aq. citric acid (3X) and 5% aq. NaHCO₃ (3X), dried over Na₂SO₄, filtered, and evaporated *in vacuo,* providing compounds IX₁₋₁₆,

### B. Synthesis of Pentafluorophenyl Esters of Chemically Cleavable Mass Spectroscopy Tags, to Liberate Tags with Carboxyl Acid Termini

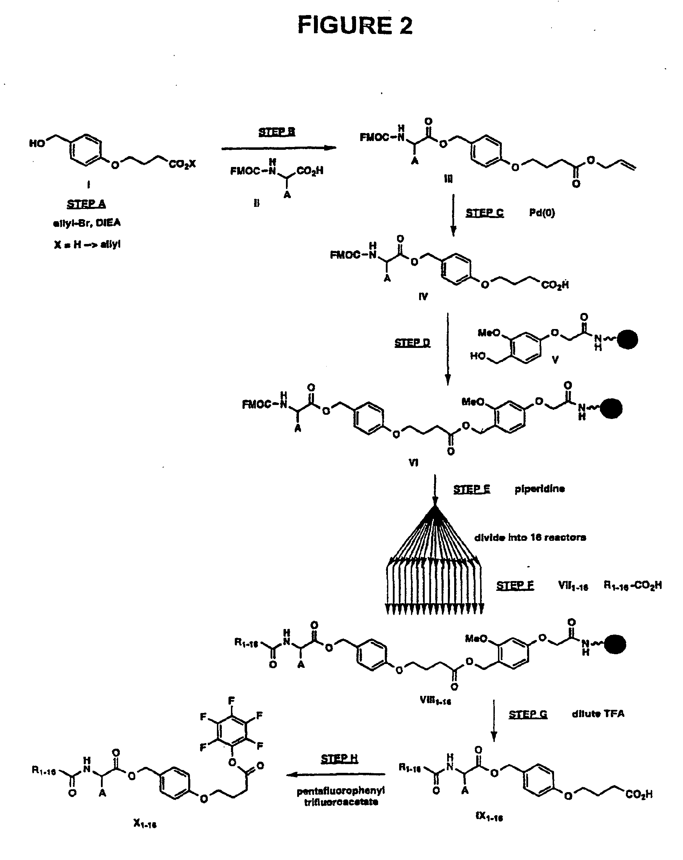

Figure 2 shows the reaction scheme.
Step A. 4-(Hydroxymethyl)phenoxybutyric acid (compound I; 1 eq.) is combined with DIEA (2.1 eq.) and allyl bromide (2.1 eq.) in CHCl₃ and heated to reflux for 2 hr. The mixture is diluted with EtOAc, washed with 1 N HCI (2X), pH 9.5 carbonate buffer (2X), and brine (1X), dried over Na₂SO₄, and evaporated *in vacuo* to give the allyl ester of compound I.
Step B. The allyl ester of compound I from step A (1.75 eq.) is combined in CH₂Cl₂ with an FMOC-protected amino acid containing amine functionality in its side chain (compound II, *e.g.* alpha-N-FMOC-3-(3-pyridyl)-alanine, available from Synthetech, Albany, OR; 1 eq.), N-methylmorpholine (2.5 eq.), and HATU (1.1 eq.), and stirred at room temperature for 4 hr. The mixture is diluted with CH₂Cl₂, washed with 1 M aq. citric acid (2X), water (1X), and 5% aq. NaHCO₃ (2X), dried over Na₂SO₄, and evaporated *in vacuo.* Compound III is isolated by flash chromatography (CH₂Cl₂-> EtOAc).
Step C. Compound III is dissolved in CH₂Cl₂, Pd(PPh₃)₄ (0.07 eq.) and N-methylaniline (2 eq.) are added, and the mixture stirred at room temperature for 4 hr. The mixture is diluted with CH₂Cl₂, washed with 1 M aq. citric acid (2X) and water (1X), dried over Na₂SO₄, and evaporated *in vacuo.* Compound IV is isolated by flash chromatography (CH₂Cl₂--> EtOAc + HOAc).
Step D. TentaGel S AC resin (compound V; 1 eq.) is suspended with DMF in the collection vessel of the ACT357 peptide synthesizer (Advanced ChemTech Inc. (ACT), Louisville, KY). Compound IV (3 eq.), HATU (3 eq.) and DIEA (7.5 eq.) in DMF are added and the collection vessel shaken for 1 hr. The solvent is removed and the resin washed with NMP (2X), MeOH (2X), and DMF (2X). The coupling of IV to the resin and the wash steps are repeated, to give compound VI.
Step E. The resin (compound VI) is mixed with 25% piperidine in DMF and shaken for 5 min. The resin is filtered, then mixed with 25% piperidine in DMF and shaken for 10 min. The solvent is removed and the resin washed with NMP (2X), MeOH (2X), and DMF (2X). The deprotected resin is then divided equally by the ACT357 from the collection vessel into 16 reaction vessels.
Step F. The 16 aliquots of deprotected resin from step E are suspended in DMF. To each reaction vessel is added the appropriate carboxylic acid VII₁₋₁₆ (R₁₋₁₆CO₂H; 3 eq.), HATU (3 eq.), and DIEA (7.5 eq.) in DMF. The vessels are shaken for 1 hr. The solvent is removed and the aliquots of resin washed with NMP (2X), MeOH (2X), and DMF (2X). The coupling of VII₁₋₁₆ to the aliquots of resin and the wash steps are repeated, to give compounds VIII₁₋₁₆.
Step G. The aliquots of resin (compounds VIII₁₋₁₆) are washed with CH₂Cl₂ (3X). To each of the reaction vessels is added 1% TFA in CH₂Cl₂ and the vessels shaken for 30 min. The solvent is filtered from the reaction vessels into individual tubes. The aliquots of resin are washed with CH₂Cl₂ (2X) and MeOH (2X) and the filtrates combined into the individual tubes. The individual tubes are evaporated *in vacuo,* providing compounds IX₁₋₁₆.
Steps H. Each of the free carboxylic acids IX₁₋₁₆ is dissolved in DMF. To each solution is added pyridine (1.05 eq.), followed by pentafluorophenyl trifluoroacetate (1.1 eq.). The mixtures are stirred for 45 min. at room temperature. The solutions are diluted with EtOAc, washed with 1 M aq. citric acid (3X) and 5% aq. NaHCO₃ (3X), dried over Na₂SO₄, filtered, and evaporated *in vacuo,* providing compounds X₁₋₁₆.

### EXAMPLE 2

### DEMONSTRATION OF PHOTOLYTIC CLEAVAGE OF T-L-X

A T-L-X compound as prepared in Example 11 was irradiated with near-UV light for 7 min at room temperature. A Rayonett fluorescence UV lamp (Southern New England Ultraviolet Co., Middletown, CT) with an emission peak at 350 nm is used as a source of UV light. The lamp is placed at a 15-cm distance from the Petri dishes with samples. SDS gel electrophoresis shows that >85% of the conjugate is cleaved under these conditions.

### EXAMPLE 3

### PREPARATION OF FLUORESCENT LABELED PRIMERS AND DEMONSTRATION OF CLEAVAGE OF FLUOROPHORE

### Synthesis and Purification of Oligonucleotides

The oligonucleotides (ODNs) are prepared on automated DNA synthesizers using the standard phosphoramidite chemistry supplied by the vendor, or the H-phosphonate chemistry (Glenn Research Sterling, VA). Appropriately blocked dA, dG, dC, and T phosphoramidites are commercially available in these forms, and synthetic nucleosides may readily be converted to the appropriate form. The oligonucleotides are prepared using the standard phosphoramidite supplied by the vendor, or the H-phosphonate chemistry. Oligonucleotides are purified by adaptations of standard methods. Oligonucleotides with 5'-trityl groups are chromatographed on HPLC using a 12 micrometer, 300 # Rainin (Emeryville, CA) Dynamax C-8 4.2x250 mm reverse phase column using a gradient of 15% to 55% MeCN in 0.1 N Et₃NH⁺OAc⁻, pH 7.0, over 20 min. When detritylation is performed, the oligonucleotides are further purified by gel exclusion chromatography. Analytical checks for the quality of the oligonucleotides are conducted with a PRP-column (Alltech, Deerfield, IL) at alkaline pH and by PAGE.

Preparation of 2,4,6-trichlorotriazine derived oligonucleotides: 10 to 1000 µg of 5'-terminal amine linked oligonucleotide are reacted with an excess recrystallized cyanuric chloride in 10% n-methyl-pyrrolidone in alkaline (pH 8.3 to 8.5 preferably) buffer at 19°C to 25°C for 30 to 120 minutes. The final reaction conditions consist of 0.15 M sodium borate at pH 8.3, 2 mg/ml recrystallized cyanuric chloride and 500 ug/ml respective oligonucleotide. The unreacted cyanuric chloride is removed by size exclusion chromatography on a G-50 Sephadex (Pharmacia, Piscataway, NJ) column.

The activated purified oligonucleotide is then reacted with a 100-fold molar excess of cystamine in 0.15 M sodium borate at pH 8.3 for 1 hour at room temperature. The unreacted cystamine is removed by size exclusion chromatography on a G-50 Sephadex column. The derived ODNs are then reacted with amine-reactive fluorochromes. The derived ODN preparation is divided into 3 portions and each portion is reacted with either (a) 20-fold molar excess of Texas Red sulfonyl chloride (Molecular Probes, Eugene, OR), with (b) 20-fold molar excess of Lissamine sulfonyl chloride (Molecular Probes, Eugene, OR), (c) 20-fold molar excess of fluorescein isothiocyanate. The final reaction conditions consist of 0.15 M sodium borate at pH 8.3 for 1 hour at room temperature. The unreacted fluorochromes are removed by size exclusion chromatography on a G-50 Sephadex column.

To cleave the fluorochrome from the oligonucleotide, the ODNs are adjusted to 1 x 10⁻⁵ molar and then dilutions are made (12, 3-fold dilutions) in TE (TE is 0.01 M Tris, pH 7.0, 5 mM EDTA). To 100 µl volumes of ODNs 25 µl of 0.01 M dithiothreitol (DTT) is added. To an identical set of controls no DDT is added. The mixture is incubated for 15 minutes at room temperature. Fluorescence is measured in a black microtiter plate. The solution is removed from the incubation tubes (150 microliters) and placed in a black microtiter plate (Dynatek Laboratories, Chantilly, VA). The plates are then read directly using a Fluoroskan II fluorometer (Flow Laboratories, McLean, VA) using an excitation wavelength of 495 nm and monitoring emission at 520 nm for fluorescein, using an excitation wavelength of 591 nm and monitoring emission at 612 nm for Texas Red, and using an excitation wavelength of 570 nm and monitoring emission at 590 nm for lissamine.

| Moles of Fluorochrome | RFU non-cleaved | RFU cleaved | RFU free |
|---|---|---|---|
| 1.0 x 10⁵M | 6.4 | 1200 | 1345 |
| 3.3 x 10⁶M | 2.4 | 451 | 456 |
| 1.1 x 10⁶M | 0.9 | 135 | 130 |
| 3.7 x 10⁷M | 0.3 | 44 | 48 |
| 1.2 x 10⁷M | 0.12 | 15.3 | 16.0 |
| 4.1 x 10⁷M | 0.14 | 4.9 | 5.1 |
| 1.4 x 10⁸M | 0.13 | 2.5 | 2.8 |
| 4.5 x 10⁹M | 0.12 | 0.8 | 0.9 |

The data indicate that there is about a 200-fold increase in relative fluorescence when the fluorochrome is cleaved from the ODN.

### EXAMPLE 4

### PREPARATION OF TAGGED M13 SEQUENCE PRIMERS AND DEMONSTRATION OF CLEAVAGE OF TAGS

Preparation of 2,4,6-trichlorotriazine derived oligonucleotides: 1000 µg of 5'-terminal amine linked oligonucleotide (5'-hexylamine-TGTAAAACGACGGCCAGT-3") (Seq. ID No. 1) are reacted with an excess recrystallized cyanuric chloride in 10% n-methyl-pyrrolidone alkaline (pH 8.3 to 8.5 preferably) buffer at 19 to 25- C for 30 to 120 minutes. The final reaction conditions consist of 0.15 M sodium borate at pH 8.3, 2 mg/ml recrystallized cyanuric chloride and 500 ug/ml respective oligonucleotide. The unreacted cyanuric chloride is removed by size exclusion chromatography on a G-50 Sephadex column.

The activated purified oligonucleotide is then reacted with a 100-fold molar excess of cystamine in 0.15 M sodium borate at pH 8.3 for 1 hour at room temperature. The unreacted cystamine is removed by size exclusion chromatography on a G-50 Sephadex column. The derived ODNs are then reacted with a variety of amides. The derived ODN preparation is divided into 12 portions and each portion is reacted (25 molar excess) with the pentafluorophenyl-esters of either: (1) 4-methoxybenzoic acid, (2) 4-fluorobenzoic acid, (3) toluic acid, (4) benzoic acid, (5) indole-3-acetic acid, (6) 2,6-difluorobenzoic acid, (7) nicotinic acid N-oxide, (8) 2-nitrobenzoic acid, (9) 5-acetylsalicylic acid, (10) 4-ethoxybenzoic acid, (11) cinnamic acid, (12) 3-aminonicotinic acid. The reaction is for 2 hours at 37°C in 0.2 M NaBorate pH 8.3. The derived ODNs are purified by gel exclusion chromatography on G-50 Sephadex.

To cleave the tag from the oligonucleotide, the ODNs are adjusted to I x 10⁻⁵ molar and then dilutions are made (12, 3-fold dilutions) in TE (TE is 0.01 M Tris, pH 7.0, 5 mM EDTA) with 50% EtOH (V/V). To 100 µl volumes of ODNs 25 µl of 0.01 M dithiothreitol (DTT) is added. To an identical set of controls no DDT is added. Incubation is for 30 minutes at room temperature. NaCI is then added to 0.1 M and 2 volumes of EtOH is added to precipitate the ODNs. The ODNs are removed from solution by centrifugation at 14,000 x G at 4°C for 15 minutes. The supernatants are reserved, dried to completeness. The pellet is then dissolved in 25 µl MeOH. The pellet is then tested by mass spectrometry for the presence of tags.

The mass spectrometer used in this work is an external ion source Fourier-transform mass spectrometer (FTMS). Samples prepared for MALDI analysis are deposited on the tip of a direct probe and inserted into the ion source. When the sample is irradiated with a laser pulse, ions are extracted from the source and passed into a long quadrupole ion guide that focuses and transports them to an FTMS analyzer cell located inside the bore of a superconducting magnet.

The spectra yield the following information. Peaks varying in intensity from 25 to 100 relative intensity units at the following molecular weights: (1) 212.1 amu indicating 4-methoxybenzoic acid derivative, (2) 200.1 indicating 4-fluorobenzoic acid derivative, (3) 196.1 amu indicating toluic acid derivative, (4) 182.1 amu indicating benzoic acid derivative, (5) 235.2 amu indicating indole-3-acetic acid derivative, (6) 218.1 amu indicating 2,6-difluorobenzoic derivative, (7) 199.1 amu indicating nicotinic acid N-oxide derivative, (8) 227.1 amu indicating 2-nitrobenzamide, (9) 179.18 amu indicating 5-acetylsalicylic acid derivative, (10) 226.1 amu indicating 4-ethoxybenzoic acid derivative, (11) 209.1 amu indicating cinnamic acid derivative, (12) 198.1 amu indicating 3-aminonicotinic acid derivative.

The results indicate that the MW-identifiers are cleaved from the primers and are detectable by mass spectrometry.

### EXAMPLE 5

### PREPARATION OF A SET OF COMPOUNDS OF THE FORMULA R₁₋₃₆-LYS(ε-INIP)-ANP-TFP

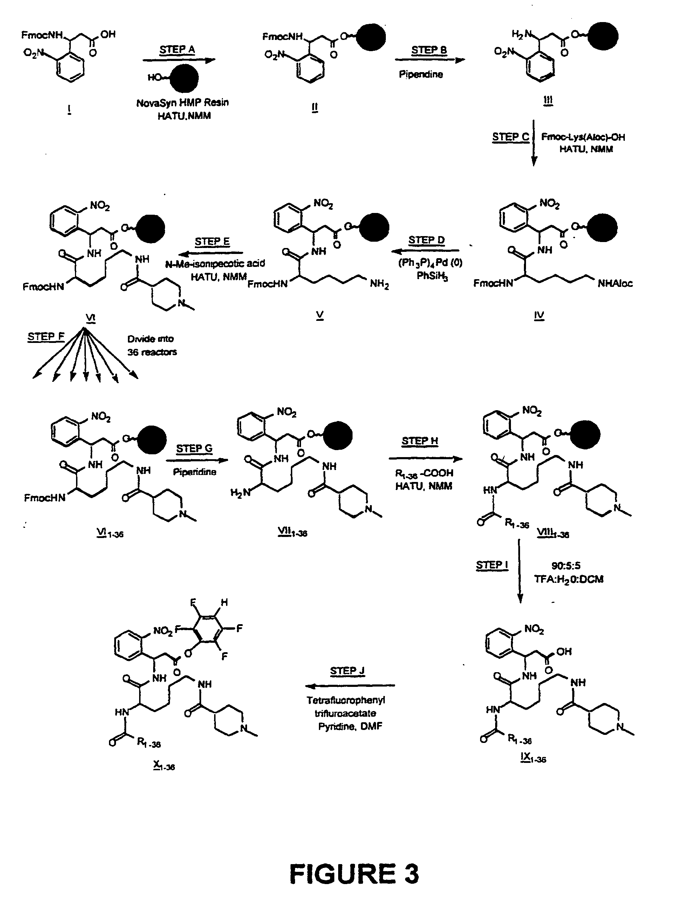

Figure 3 illustrates the parallel synthesis of a set of 36 T-L-X compounds (X = Lₕ), where Lₕ is an activated ester (specifically, tetrafluorophenyl ester), L² is an ortho-nitrobenzylamine group with L³ being a methylene group that links Lₕ and L², T has a modular structure wherein the carboxylic acid group of lysine has been joined to the nitrogen atom of the L² benzylamine group to form an amide bond, and a variable weight component R₁₋₃₆, (where these R groups correspond to T² as defined herein, and may be introduced via any of the specific carboxylic acids listed herein) is bonded through the α-amino group of the lysine, while a mass spec sensitivity enhancer group (introduced via N-methylisonipecotic acid) is bonded through the ε-amino group of the lysine.

Referring to Figure 3:
Step A. NovaSyn HMP Resin (available from NovaBiochem; 1 eq.) is suspended with DMF in the collection vessel of the ACT357. Compound I (ANP available from ACT; 3 eq.), HATU (3 eq.) and NMM (7.5 eq.) in DMF are added and the collection vessel shaken for 1 hr. The solvent is removed and the resin washed with NMP (2X), MeOH (2X), and DMF (2X). The coupling of I to the resin and the wash steps are repeated, to give compound II.
Step B. The resin (compound II) is mixed with 25% piperidine in DMF and shaken for 5 min. The resin is filtered, then mixed with 25% piperidine in DMF and shaken for 10 min. The solvent is removed, the resin washed with NMP (2X), MeOH (2X), and DMF (2X), and used directly in step C.
Step C. The deprotected resin from step B is suspended in DMF and to it is added an FMOC-protected amino acid, containing a protected amine functionality in its side chain (Fmoc-Lysine(Aloc)-OH, available from PerSeptive Biosystems; 3 eq.), HATU (3 eq.), and NMM (7.5 eq.) in DMF. The vessel is shaken for I hr. The solvent is removed and the resin washed with NMP (2X), MeOH (2X), and DMF (2X). The coupling of Fmoc-Lys(Aloc)-OH to the resin and the wash steps are repeated, to give compound IV.
Step D. The resin (compound IV) is washed with CH₂Cl₂(2X), and then suspended in a solution of (PPh₃)₄Pd (0) (0.3 eq.) and PhSiH₃ (10 eq.) in CH₂Cl₂. The mixture is shaken for 1 hr. The solvent is removed and the resin is washed with CH₂Cl₂ (2X). The palladium step is repeated. The solvent is removed and the resin is washed with CH₂Cl₂ (2X), N,N-diisopropylethylammonium diethyldithiocarbamate in DMF (2X), DMF (2X) to give compound V.
Step E. The deprotected resin from step D is coupled with N-methylisonipecotic acid as described in step C to give compound VI.
Step F. The Fmoc protected resin VI is divided equally by the ACT357 from the collection vessel into 36 reaction vessels to give compounds VI₁₋₃₆.
Step G. The resin (compounds VI₁₋₃₆) is treated with piperidine as described in step B to remove the FMOC group.
Step H. The 36 aliquots of deprotected resin from step G are suspended in DMF. To each reaction vessel is added the appropriate carboxylic acid (R₁₋₃₆CO₂H; 3 eq.), HATU (3 eq.), and NMM (7.5 eq.) in DMF. The vessels are shaken for 1 hr. The solvent is removed and the aliquots of resin washed with NMP (2X), MeOH (2X), and DMF (2X). The coupling of R₁₋₃₆CO₂H to the aliquots of resin and the wash steps are repeated, to give compounds VIII₁₋₃₆.
Step I. The aliquots of resin (compounds VIII₁₋₃₆) are washed with CH₂Cl₂ (3X). To each of the reaction vessels is added 90:5:5 TFA:H20:CH₂Cl₂ and the vessels shaken for 120 min. The solvent is filtered from the reaction vessels into individual tubes. The aliquots of resin are washed with CH₂Cl₂ (2X) and MeOH (2X) and the filtrates combined into the individual tubes. The individual tubes are evaporated *in vacuo,* providing compounds IX₁₋₃₆.
Step J. Each of the free carboxylic acids IX₁₋₃₆ is dissolved in DMF. To each solution is added pyridine (1.05 eq.), followed by tetrafluorophenyl trifluoroacetate (1.1 eq.). The mixtures are stirred for 45 min. at room temperature. The solutions are diluted with EtOAc, washed with 5% aq. NaHCO₃ (3X), dried over Na₂SO₄, filtered, and evaporated *in vacuo,* providing compounds X₁₋₃₆.

### EXAMPLE 6

### PREPARATION OF A SET OF COMPOUNDS OF THE FORMULA R₁₋₃₆-LYS(ε-INIP)-NBA-TFP

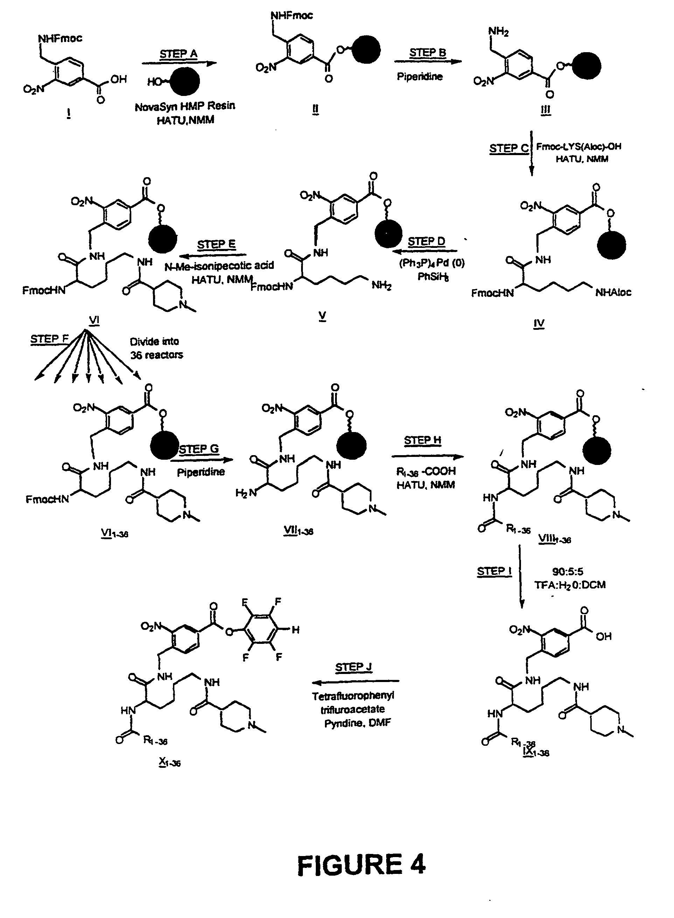

Figure 4 illustrates the parallel synthesis of a set of 36 T-L-X compounds (X = Lₕ), where Lₕ is an activated ester (specifically, tetrafluorophenyl ester), L² is an ortho-nitrobenzylamine group with L³ being a direct bond between Lₕ and L², where Lₕ is joined directly to the aromatic ring of the L² group, T has a modular structure wherein the carboxylic acid group of lysine has been joined to the nitrogen atom of the L² benzylamine group to form an amide bond, and a variable weight component R₁₋₃₆, (where these R groups correspond to T² as defined herein, and may be introduced via any of the specific carboxylic acids listed herein) is bonded through the α-amino group of the lysine, while a mass spec enhancer group (introduced via N-methylisonipecotic acid) is bonded through the ε-amino group of the lysine.

Referring to Figure 4
Step A. NovaSyn HMP Resin is coupled with compound I (NBA prepared according to the procedure of Brown et al., Molecular Diversity, 1, 4 (1995)) according to the procedure described in step A of Example 5, to give compound II.
Steps B-J. The resin (compound II) is treated as described in steps B-J of Example 5 to give compounds X₁₋₃₆.

### EXAMPLE 7

### PREPARATION OF A SET OF COMPOUNDS OF THE FORMULA INIP-LYS (ε-R₁₋₃₆)-ANP-TFP

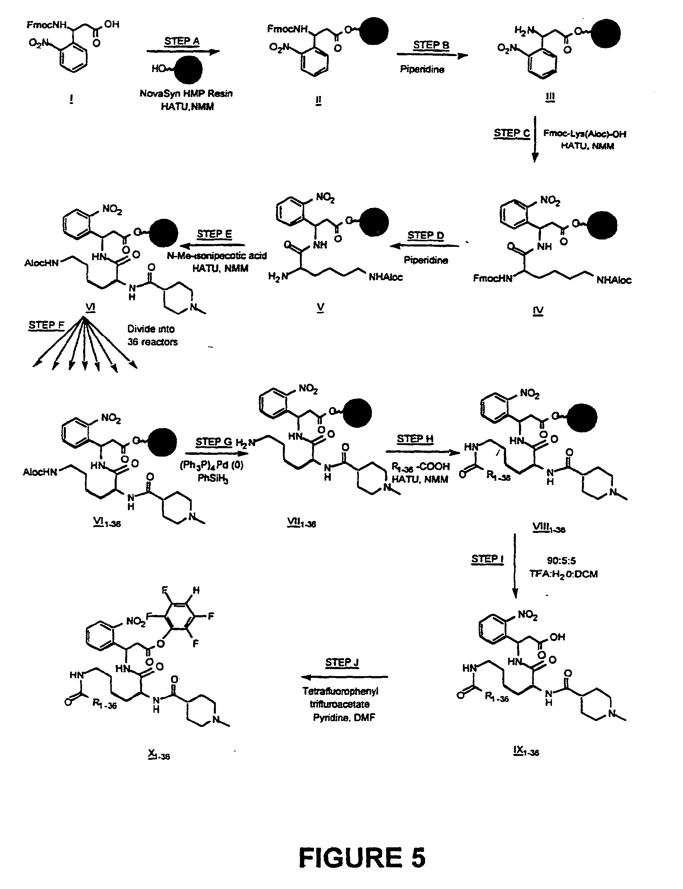

Figure 5 illustrates the parallel synthesis of a set of 36 T-L-X compounds (X = Lₕ), where Lₕ is an activated ester (specifically, tetrafluorophenyl ester), L² is an ortho-nitrobenzylamine group with L³ being a methylene group that links Lₕ and L², T has a modular structure wherein the carboxylic acid group of lysine has been joined to the nitrogen atom of the L² benzylamine group to form an amide bond, and a variable weight component R₁₁₆, (where these R groups correspond to T² as defined herein, and may be introduced via any of the specific carboxylic acids listed herein) is bonded through the ε-amino group of the lysine, while a mass spec sensitivity enhancer group (introduced via N-methylisonipecotic acid) is bonded through the α-amino group of the lysine.

Referring to Figure 5:
Steps A-C. Same as in Example 5.
Step D. The resin (compound IV) is treated with piperidine as described in step B of Example 5 to remove the FMOC group.
Step E. The deprotected α-amine on the resin in step D is coupled with N-methylisonipecotic acid as described in step C of Example 5 to give compound V.
Step F. Same as in Example 5.
Step G. The resin (compounds VI₁₋₃₆) are treated with palladium as described in step D of Example 5 to remove the Aloc group.
Steps H-J. The compounds X₁₋₃₆ are prepared in the same manner as in Example 5.

### EXAMPLE 8

### PREPARATION OF A SET OF COMPOUNDS OF THE FORMULA R₁₋₃₆-GLU(γ-DIAEA)-ANP-TFP

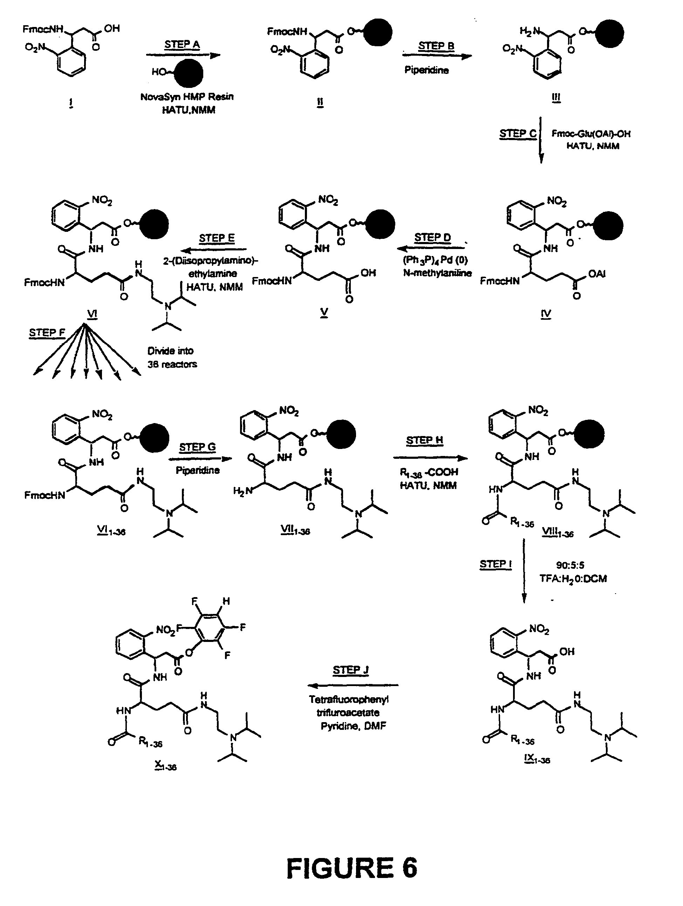

Figure 6 illustrates the parallel synthesis of a set of 36 T-L-X compounds (X = Lₕ), where Lₕ is an activated ester (specifically, tetrafluorophenyl ester), L² is an ortho-nitrobenzylamine group with L³ being a methylene group that links Lₕ and L², T has a modular structure wherein the α-carboxylic acid group of glutamatic acid has been joined to the nitrogen atom of the L² benzylamine group to form an amide bond, and a variable weight component R₁₋₃₆, (where these R groups correspond to T² as defined herein, and may be introduced via any of the specific carboxylic acids listed herein) is bonded through the aa-amino group of the glutamic acid, while a mass spec sensitivity enhancer group (introduced via 2-(diisopropylamino)ethylamine) is bonded through the γ-carboxylic acid of the glutamic acid.

Referring to Figure 6:
Steps A-B. Same as in Example 5.
Step C. The deprotected resin (compound III) is coupled to Fmoc-Glu-(OAI)-OH using the coupling method described in step C of Example 5 to give compound IV.
Step D. The allyl ester on the resin (compound IV) is washed with CH₂Cl₂ (2X) and mixed with a solution of (PPh₃)₄Pd (0) (0.3 eq.) and N-methylaniline (3 eq.) in CH₂Cl₂. The mixture is shaken for 1 hr. The solvent is removed and the resin is washed with CH₂Cl₂ (2X). The palladium step is repeated. The solvent is removed and the resin is washed with CH₂Cl₂ (2X), N,N-diisopropylethylammonium diethyldithiocarbamate in DMF (2X), DMF (2X) to give compound V.
Step E. The deprotected resin from step D is suspended in DMF and activated by mixing HATU (3 eq.), and NMM (7.5 eq.). The vessels are shaken for 15 minutes. The solvent is removed and the resin washed with NMP (1X). The resin is mixed with 2-(diisopropylamino)ethylamine (3 eq.) and NMM (7.5 eq.). The vessels are shaken for 1 hour. The coupling of 2-(diisopropylamino)ethylamine to the resin and the wash steps are repeated, to give compound VI.
Steps F-J. Same as in Example 5.

### EXAMPLE 9

### PREPARATION OF A SET OF COMPOUNDS OF THE FORMULA R₁₋₃₆-LYS(ε-INIP)-ANP-LYS(ε-NH₂)-NH₂

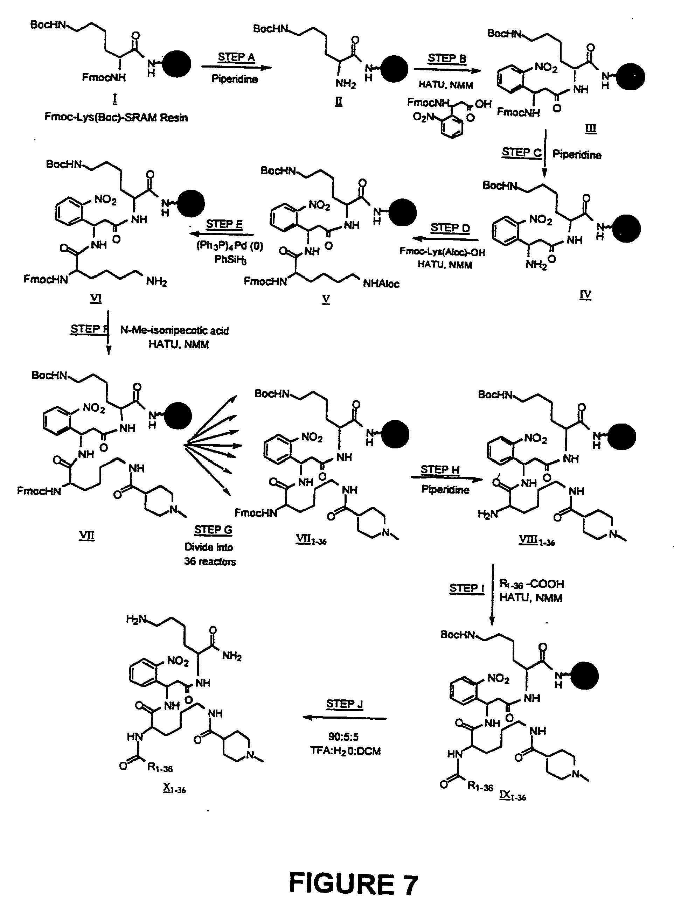

Figure 7 illustrates the parallel synthesis of a set of 36 T-L-X compounds (X = Lₕ), where Lₕ is an amine (specifically, the ε-amino group of a lysine-derived moiety), L² is an ortho-nitrobenzylamine group with L³ being a carboxamido-substituted alkyleneaminoacylalkylene group that links Lₕ and L², T has a modular structure wherein the carboxylic acid group of lysine has been joined to the nitrogen atom of the L² benzylamine group to form an amide bond, and a variable weight component R₁₋₃₆, (where these R groups correspond to T² as defined herein, and may be introduced via any of the specific carboxylic acids listed herein) is bonded through the α-amino group of the lysine, while a mass spec sensitivity enhancer group (introduced via N-methylisonipecotic acid) is bonded through the ε-amino group of the lysine.

Referring to Figure 7:
Step A. Fmoc-Lys(Boc)-SRAM Resin (available from ACT; compound I) is mixed with 25% piperidine in DMF and shaken for 5 min. The resin is filtered, then mixed with 25% piperidine in DMF and shaken for 10 min. The solvent is removed, the resin washed with NMP (2X), MeOH (2X), and DMF (2X), and used directly in step B.
Step B. The resin (compound II), ANP (available from ACT; 3 eq.), HATU (3 eq.) and NMM (7.5 eq.) in DMF are added and the collection vessel shaken for 1 hr. The solvent is removed and the resin washed with NMP (2X), MeOH (2X), and DMF (2X). The coupling of I to the resin and the wash steps are repeated, to give compound III.
Steps C-J. The resin (compound III) is treated as in steps B-I in Example 5 to give compounds X₁₋₃₆.

### EXAMPLE 10

### PREPARATION OF A SET OF COMPOUNDS OF THE FORMULA R₁₋₃₆-LYS(ε-TFA)-LYS(ε-IINP)-ANP-TFP

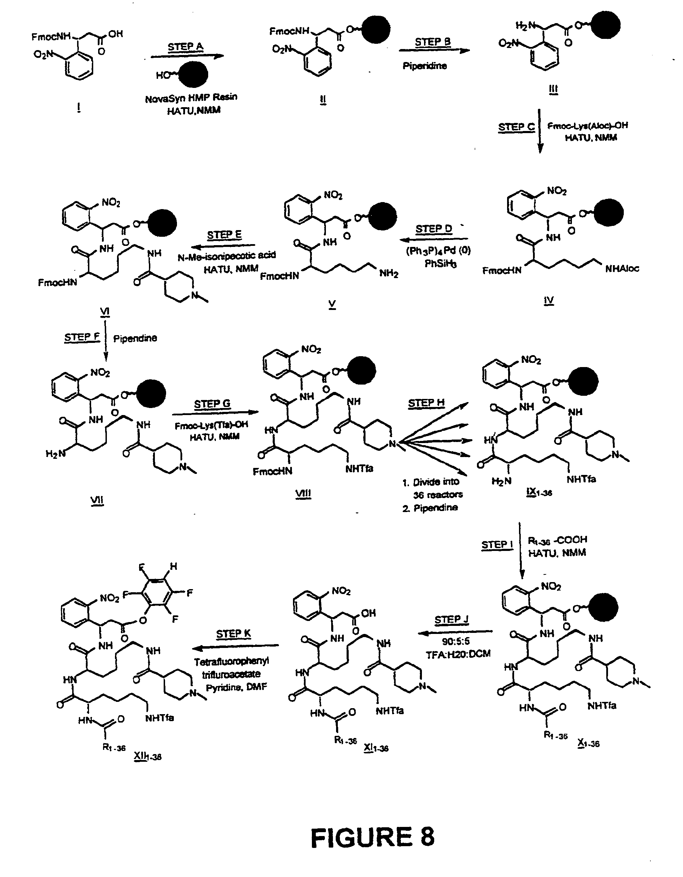

Figure 8 illustrates the parallel synthesis of a set of 36 T-L-X compounds (X = Lₕ), where Lₕ is an activated ester (specifically, tetrafluorophenyl ester), L² is an ortho-nitrobenzylamine group with L³ being a methylene group that links Lₕ and L², T has a modular structure wherein the carboxylic acid group of a first lysine has been joined to the nitrogen atom of the L² benzylamine group to form an amide bond, a mass spec sensitivity enhancer group (introduced via N-methylisonipecotic acid) is bonded through the ε-amino group of the first lysine, a second lysine molecle has been joined to the first lysine through the α-amino group of the first lysine, a molecular weight adjuster group (having a trifluoroacetyl structure) is bonded through the ε-amino group of the second lysine, and a variable weight component R₁₋₃₆, (where these R groups correspond to T² as defined herein, and may be introduced via any of the specific carboxylic acids listed herein) is bonded through the α-amino group of the second lysine. Referring to Figure 8:
Steps A-E. These steps are identical to steps A-E in Example 5.
Step F. The resin (compound VI) is treated with piperidine as described in step B in Example 5 to remove the FMOC group.
Step G. The deprotected resin (compound VII) is coupled to Fmoc-Lys(Tfa)-OH using the coupling method described in step C of Example 5 to give compound VIII.
Steps H-K. The resin (compound VIII) is treated as in steps F-J in Example 5 to give compounds XI₁₋₃₆.

### EXAMPLE 11

### PREPARATION OF A SET OF COMPOUNDS OF THE FORMULA R₁₋₃₆-LYS(ε-INIP)-ANP-5'-AH-ODN

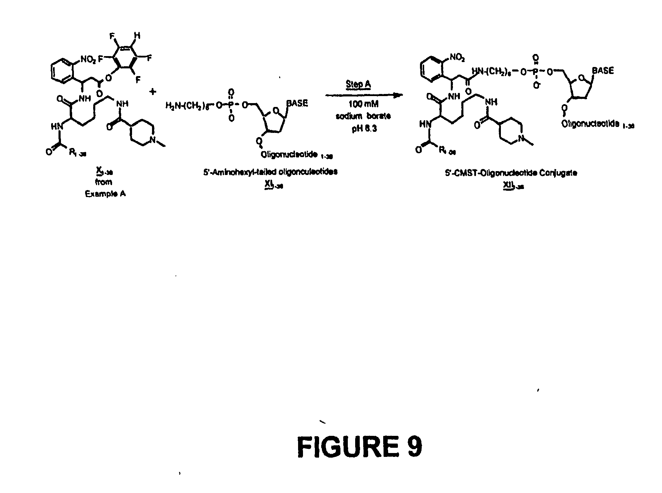

Figure 9 illustrates the parallel synthesis of a set of 36 T-L-X compounds (X = MOI, where MOI is a nucleic acid fragment, ODN) derived from the esters of Example 5 (the same procedure could be used with other T-L-X compounds wherein X is an activated ester). The MOI is conjugated to T-L through the 5' end of the MOI, via a phosphodiester - alkyleneamine group.

Referring to Figure 9:
Step A. Compounds XII₁₋₃₆ are prepared according to a modified biotinylation procedure in Van Ness et al., Nucleic Acids Res., 19, 3345 (1991). To a solution of one of the 5'-aminohexyl oligonucleotides (compounds XI₁₋₃₆,1 mg) in 200 mM sodium borate (pH 8.3, 250 mL) is added one of the Tetrafluorophenyl esters (compounds X₁₋₃₆ from Example A, 100-fold molar excess in 250 mL of NMP). The reaction is incubated overnight at ambient temperature. The unreacted and hydrolyzed tetrafluorophenyl esters are removed from the compounds XII₁₋₃₆ by Sephadex G-50 chromatography.

### EXAMPLE 12

### PREPARATION OF A SET OF COMPOUNDS OF THE FORMULA R₁₋₃₆-LYS(ε-INIP)-ANP-LYS(ε-(MCT-5'-AH-ODN))-NH₂

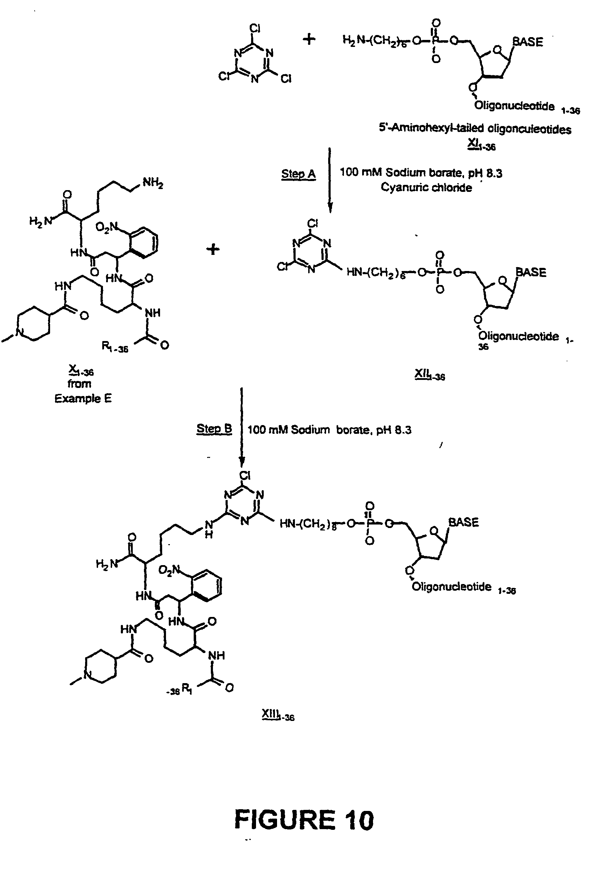

Figure 10 illustrates the parallel synthesis of a set of 36 T-L-X compounds (X = MOI, where MOI is a nucleic acid fragment, ODN) derived from the amines of Example 11 (the same procedure could be used with other T-L-X compounds wherein X is an amine). The MOI is conjugated to T-L through the 5' end of the MOI, via a phosphodiester - alkyleneamine group.

Referring to Figure 10:
Step A. The 5'-[6-(4,6-dichloro-1,3,5-triazin-2-ylamino)hexyl]oligonucleotides XII₁₋₃₆ are prepared as described in Van Ness et al., Nucleic Acids Res., 19, 3345 (1991).
Step B. To a solution of one of the 5'-[6-(4,6-dichloro- 1,3,5-triazin-2-ylamino)hexyl]oligonucleotides (compounds XII₁₋₃₆) at a concentration of 1 mg/ml in 100 mM sodium borate (pH 8.3) was added a 100-fold molar excess of a primary amine selected from R₁₋₃₆-Lys(e-iNIP)-ANP-Lys(e-NH₂)-NH₂ (compounds X₁₋₃₆ from Example 11). The solution is mixed overnight at ambient temperature. The unreacted amine is removed by ultrafiltration through a 3000 MW cutoff membrane (Amicon, Beverly, MA) using H₂O as the wash solution (3 X). The compounds XIII₁₋₃₆ are isolated by reduction of the volume to 100 mL.

### EXAMPLE 13

### DEMONSTRATION OF THE SIMULTANEOUS DETECTION OF MULTIPLE TAGS BY MASS SPECTROMETRY

This example provides a description of the ability to simultaneously detect multiple compounds (tags) by mass spectrometry. In this particular example, 31 compounds are mixed with a matrix, deposited and dried on to a solid support and then desorbed with a laser. The resultant ions are then introduced in a mass spectrometer.

The following compounds (purchased from Aldrich, Milwaukee, WI) are mixed together on an equal molar basis to a final concentration of 0.002 M (on a per compound) basis: benzamide (121.14), nicotinamide (122.13), pyrazinamide (123.12), 3-amino-4-pyrazolecarboxylic acid (127.10), 2-thiophenecarboxamide (127.17), 4-aminobenzamide (135.15), tolumide (135.17), 6-methylnicotinamide (136.15), 3-aminonicotinamide (137.14), nicotinamide N-oxide (138.12), 3-hydropicolinamide (138.13), 4-fluorobenzamide (139.13), cinnamamide (147.18), 4-methoxybenzamide (151.17), 2,6-difluorbenzamide (157.12), 4-amino-5-imidazole-carboxyamide (162.58), 3,4-pyridine-dicarboxyamide (165.16), 4-ethoxybenzamide (165.19), 2,3-pyrazinedicarboxamide (166.14), 2-nitrobenzamide (166.14), 3-fluoro-4-methoxybenzoic acid (170.4), indole-3-acetamide (174.2), 5-acetylsalicylamide (179.18), 3,5-dimethoxybenzamide (181.19), 1-naphthaleneacetamide (185.23), 8-chloro-3,5-diamino-2-pyrazinecarboxyamide (187.59), 4-trifluoromethyl-benzamide (189.00), 5-amino-5-phenyl-4-pyrazole-carboxamide (202.22), 1-methyl-2-benzyl-malonamate (207.33), 4-amino-2,3,5,6-tetrafluorobenzamide (208.11), 2,3-napthlenedicarboxylic acid (212.22). The compounds are placed in DMSO at the concentration described above. One µl of the material is then mixed with alpha-cyano-4-hydroxy cinnamic acid matrix (after a 1:10,000 dilution) and deposited on to a solid stainless steel support.

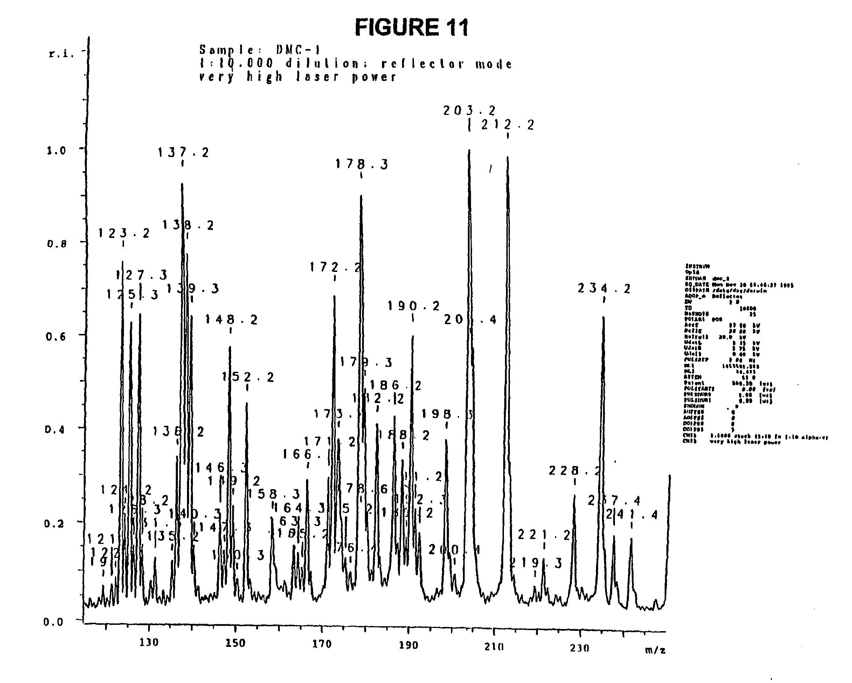

The material is then desorbed by a laser using the Protein TOF Mass Spectrometer (Bruker, Manning Park, MA) and the resulting ions are measured in both the linear and reflectron modes of operation. The following m/z values are observed (Figure 11):
- 121.1---->: benzamide (121.14)
- 122.1---->: nicotinamide (122.13)
- 123.1---->: pyrazinamide (123.12)
- 124.1:
- 125.2:
- 127.3---->: 3-amino-4-pyrazolecarboxylic acid (127.10)
- 127.2---->: 2-thiophenecarboxamide (127.17)
- 135.1---->: 4-aminobenzamide (135.15)
- 135.1---->: tolumide (135.17)
- 136.2---->: 6-methylnicotinamide (136.15)
- 137.1---->: 3-aminonicotinamide (137.14)
- 138,2---->: nicotinamide N-oxide (138.12)
- 138.2---->: 3-hydropicolinamide (138.13)
- 139.2---->: 4-fluorobenzamide (139.13)
- 140.2:
- 147.3---->: cinnamamide (147.18)
- 148.2:
- 149.2: 4-methoxybenzamide (151.17)
- 152.2: 2,6-difluorbenzamide (157.12)
- 158.3: 4-amino-5-imidazole-carboxyamide (162.58)
- 163.3:
- 165.2---->: 3,4-pyridine-dicarboxyamide (165.16)
- 165.2---->: 4-ethoxybenzamide (165.19)
- 166.2---->: 2,3-pyrazinedicarboxamide (166.14)
- 166.2---->: 2-nitrobenzamide (166.14) 3-fluoro-4-methoxybenzoic acid (170.4)
- 171.1:
- 172.2:
- 173.4: indole-3-acetamide (174.2)
- 178.3:
- 179.3---->: 5-acetylsalicylamide (179.18)
- 181.2---->: 3,5-dimethoxybenzamide (181.19)
- 182.2---->: 1-naphthaleneacetamide (185.23)
- 186.2: 8-chloro-3,5-diamino-2-pyrazinecarboxyamide (187.59)
- 188.2:
- 189.2---->: 4-trifluoromethyl-benzamide (189.00)
- 190.2:
- 191.2:
- 192.3: 5-amino-5-phenyl-4-pyrazole-carboxamide (202.22)
- 203.2:
- 203.4: 1-methyl-2-benzyl-malonamate (207.33) 4-amino-2,3,5,6-tetrafluorobenzamide (208.11)
- 212.2---->: 2,3-napthlenedicarboxylic acid (212.22).
- 219.3:
- 221.2:
- 228.2:
- 234.2:
- 237.4:
- 241.4:

The data indicate that 22 of 31 compounds appeared in the spectrum with the anticipated mass, 9 of 31 compounds appeared in the spectrum with a n + H mass (1 atomic mass unit, amu) over the anticipated mass. The latter phenomenon is probably due to the protonation of an amine within the compounds. Therefore 31 of 31 compounds are detected by MALDI Mass Spectroscopy. More importantly, the example demonstrates that multiple tags can be detected simultaneously by a spectroscopic method.

The alpha-cyano matrix alone (Figure 11) gave peaks at 146.2, 164.1, 172.1, 173.1, 189.1, 190.1, 191.1, 192.1, 212.1, 224.1, 228.0, 234.3. Other identified masses in the spectrum are due to contaminants in the purchased compounds as no effort was made to further purify the compounds.

### EXAMPLE 14

### MICROSATELLITE MARKERS: PCR AMPLIFICATIONS.

The microsatellite markers are amplified utilizing the following standard PCR conditions. Briefly, PCR reactions are performed in a total volume of 50 µl, containing 40 ng of genomic DNA, 50 pmol of each primer, 0.125 mM dNTPs and 1 unit of *Taq* polymerase. 1X amplification buffer contains 10 mM Tris base, pH 9, 50 mM KCl, 1.5 mM MgCl₂, 0.1% Triton X-100 and 0.01% gelatin. The reactions are performed using a "hot-start" procedure: *Taq* polymerase is added only after a first denaturation step of 5 minutes at 96°C. Amplification is carried out for 35 cycles: denaturation (94°C for 40 sec) and annealing (55°C for 30 sec). An elongation step (72°C for 2 minutes) ends the process after the last annealing. Since the amplification products to be obtained are short (90 to 350 base pairs long) and the time interval to raise the temperature from 55°C to 94°C (obtained with a ramping rate of 1°C/second) is long enough, completion of DNA elongation can be achieved without a step at 72°C.

## Claims

1. A method for high through-put screening for a polymorphism in multiple individuals or multiple polymorphisms in an individual, comprising:
(a) forming a plurality of tagged nucleic acids using tagged PCR primers, where a tag is correlative with a particular individual of the multiple individuals or a particular polymorphism of the multiple polymorphisms;
(b) subjecting the plurality to a separation procedure, where the separation procedure separates tagged nucleic acids based on size;
(c) cleaving the tags from the tagged nucleic acids, and
(c) detecting the cleaved tags by non-fluorescent spectrometry or potentiometry.

2. The method of claim 1 wherein the nucleic acids within the plurality of tagged nucleic acids are derived from more than one individual.

3. The method of claim 1 wherein the nucleic acids within the plurality of tagged nucleic acids are all derived from a single individual.

4. The method of any one of claims 1-3 wherein the detection of the tag is by mass spectrometry, infrared spectrometry, ultraviolet spectrometry, or potentiostatic amperometry.

5. The method of any one of claims 1-4 wherein the separation of tagged nucleic acids is by a method selected from the group consisting of gel electrophoresis, capillary electrophoresis, micro-channel electrophoresis, HPLC, size exclusion chromatography, and filtration.

6. The method of claim 5 wherein the tagged nucleic acids are cleaved by a method selected from the group consisting of oxidation, reduction, acid-labile, base labile, enzymatic, electrochemical, thermal and photolabile methods.

7. The method according to any one of claims 1-6 wherein the cleaved tag is detected by time-of-flight mass spectrometry, quadrupole mass spectrometry, magnetic sector mass spectrometry and electric sector mass spectrometry.

8. The method according to any one of claim 1-6 wherein the cleaved tag is detected by potentiostatic amperometry utilizing detectors selected from the group consisting of coutometric detectors and amperometric detectors.

9. The method according to any one of claims 1-8 wherein the steps of separating, cleaving and detecting are performed in a continuous manner.

10. The method according to any one of claims 1-9 wherein the steps of separating, cleaving and detecting are performed in a continuous manner on a single device.

11. The method according to any one of claims 1-10 wherein the steps of separating, cleaving and detecting are automated.

12. The method according to any one of claims 1-11 wherein the tagged nucleic acids are generated from non 3'-tagged oligonucleotide primers.

13. The method according to any one of claims 1-11 wherein the tagged nucleic acids are generated from tagged dideoxynucleotide terminators.

14. The method according to any one of the preceding claims wherein the tagged nucleic acids have the formula:
T^{ms}-L-MOI
wherein,
T^{ms} is an organic group detectable by mass spectrometry, comprising carbon, at least one of hydrogen and fluoride, and optional atoms selected from oxygen, nitrogen, sulfur, phosphorus and iodine;
L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid; and
MOI (molecule of interest) is a nucleic acid fragment,
wherein L is conjugated to the MOI at a location other than the 3' end of the MOI, and wherein at least two tagged nucleic acids have the same T^{ms} but non-identical nucleotide lengths.

15. The method according to any one of the preceding claims wherein the tagged nucleic acids have the formula:
T^{ms}-L-MOI
wherein,
T^{ms} is an organic group detectable by mass spectrometry, comprising carbon, at least one of hydrogen and fluoride, and optional atoms selected from oxygen, nitrogen, sulfur, phosphorus and iodine;
L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid; and
MOI (molecule of interest) is a nucleic acid fragment,
wherein L is conjugated to the MOI at a location other than the 3' end of the MOI, and wherein at least two tagged nucleic acids have the same T^{ms} but non-identical elution times as measured by column chromatography.

16. The method according to any one of the preceding claims wherein the tagged nucleic acids have the formula:
T^{ms}-L-MOI
wherein,
T^{ms} is an organic group detectable by mass spectrometry, comprising carbon, at least one of hydrogen and fluoride, and optional atoms selected from oxygen, nitrogen, sulfur, phosphorus and iodine;
L is an organic group which allows a T^{ms}-containing moiety to be cleaved from the remainder of the compound, wherein the T^{ms}-containing moiety comprises a functional group which supports a single ionized charge state when the compound is subjected to mass spectrometry and is selected from tertiary amine, quaternary amine and organic acid; and
MOI (molecule of interest) is a nucleic acid fragment,
wherein L is conjugated to the MOI at a location other than the 3' end of the MOI, and wherein no two tagged nucleic acids that have the same nucleotide length also have the same T^{ms}.

## Patentansprüche

1. Verfahren zum Hochdurchsatz-Screenen auf einen Polymorphismus in mehreren Individuen oder mehrere Polymorphismen in einem Individuum, umfassend:
(a) Bilden einer Vielzahl markierter Nukleinsäuren unter Verwendung markierter PCR Primer, wobei eine Markierung korrelierend ist mit einem bestimmten Individuum der mehreren Individuen oder mit einem bestimmten Polymorphismus der mehreren Polymorphismen;
(b) Unterziehen der Vielzahl einem Trennverfahren, wobei das Trennverfahren die markierten Nukleinsäuren aufgrund der Größe trennt;
(c) Abspalten der Markierungen von den markierten Nukleinsäuren, und
(d) Nachweisen der abgetrennten Markierungen durch Nicht-Fluoreszenz-Spektrometrie oder Potentiometrie.

2. Verfahren nach Anspruch 1, wobei die Nukleinsäuren innerhalb der Vielzahl markierter Nukleinsäuren von mehr als einem Individuum abgeleitet sind.

3. Verfahren nach Anspruch 1, wobei die Nukleinsäuren innerhalb der Vielzahl der markierten Nukleinsäuren alle von einem einzelnen Individuum abgeleitet sind.

4. Verfahren nach einem der Ansprüche 1-3, wobei der Nachweis der Markierung durch Massenspektrometrie, Infrarotspektrometrie, Ultravioletspektrometrie oder potentiostatische Amperometrie erfolgt.

5. Verfahren nach einem der Ansprüche 1-4, wobei die Trennung der markierten Nukleinsäuren durch ein Verfahren erfolgt ausgewählt aus der Gruppe bestehend aus Gelelektrophorese, Kapillarelektrophorese, Mikrokanal-Elektrophorese, HPLC, Größenausschlusschromatographie, und Filtration.

6. Verfahren nach Anspruch 5, wobei die markierten Nukleinsäuren abgespalten werden durch ein Verfahren ausgewählt aus der Gruppe bestehend aus Oxidation, Reduktion, säureunbeständigen, basenunbeständigen, enzymatischen, elektrochemischen, thermischen und lichtunbeständigen Verfahren.

7. Verfahren nach einem der Ansprüche 1-6, wobei die abgespaltene Markierung nachgewiesen wird durch Flugzeitmassenspektrometrie, Quadrupolmassenspektrometrie, magnetischer Sektorfeld-Massenspektrometrie und elektrischer Sektorfeld-Massenspektrometrie detektiert wird.

8. Verfahren nach einem der Ansprüche 1-6, wobei die abgespaltene Markierung nachgewiesen wird durch potentiostatische Amperometrie mittels Detektoren ausgewählt aus der Gruppe bestehend aus coulometrischen Detektoren und amperometrischen Detektoren.

9. Verfahren nach einem der Ansprüche 1-8, wobei die Schritte des Abtrennens, Abspaltens und Detektieren in zusammenhängender Weise ausgeführt werden.

10. Verfahren nach einem der Ansprüche 1-9, wobei die Schritte des Abtrennens, Abspaltens und Detektieren in zusammenhängender Weise mit einem einzigen Gerät ausgeführt werden.

11. Verfahren nach einem der Ansprüche 1-10, wobei die Schritte des Abtrennens, Abspaltens und Detektierens automatisiert sind.

12. Verfahren nach einem der Ansprüche 1-11, wobei die markierten Nukleinsäuren hergestellt sind aus nicht-3'-markierten Oligonukleotid-Primern.

13. Verfahren nach einem der Ansprüche 1-11, wobei die markierten Nukleinsäuren hergestellt sind aus markierten Didesoxynukleotid-Terminatoren.

14. Verfahren nach einem der vorherigen Ansprüche, wobei die markierten Nukleinsäuren die Formel:
T^{ms}-L-MOI
haben, wobei
T^{ms} eine organische Gruppe ist detektierbar durch Massenspektrometrie, umfassend Kohlenstoff, zumindest eines der Elemente Wasserstoff und Fluor, und optional Atome ausgewählt aus Sauerstoff, Stickstoff, Schwefel, Phosphor und lod;
L eine organische Gruppe ist, die es einem T^{ms}-enthaltenden Rest erlaubt, vom Rest der Verbindung abgespalten zu werden, wobei der T^{ms}-enthaltende Rest umfasst: eine funktionelle Gruppe, die einen einzelnen ionisierten Ladungszustand trägt, wenn die Verbindung einer Massenspektrometrie unterworfen wird und ausgewählt ist aus tertiärem Amin, quartärem Amin und organischer Säure; und
MOI (interessierendes Molekül) ein Nukleinsäure-Fragment ist,
wobei L mit dem MOI konjugiert ist an einer Stelle außer dem 3'-Ende des MOI und wobei zumindest zwei markierte Nukleinsäuren die gleiche T^{ms}, aber unterschiedliche Nukleotidlängen haben.

15. Verfahren nach einem der vorherigen Ansprüche, wobei die markierten Nukleinsäuren die Formel:
T^{ms}-L-MOI
haben, wobei
T^{ms} eine organische Gruppe ist detektierbar durch Massenspektrometrie, umfassend Kohlenstoff, zumindest eines der Elemente Wasserstoff und Fluor, und optional Atome ausgewählt aus Sauerstoff, Stickstoff, Schwefel, Phosphor und Iod;
L eine organische Gruppe ist, die es einem T^{ms}-enthaltenden Rest erlaubt, Rest der Verbindung abgespalten zu werden, wobei der T^{ms}-enthaltende Rest umfasst: eine funktionale Gruppe, die einen einzelnen ionisierten Ladungszustand trägt, wenn die Verbindung einer Massenspektrometrie unterworfen wird und ausgewählt ist aus tertiärem Amin, quartärem Amin und organischer Säure; und
MOI (interessierendes Molekül) ein Nukleinsäure-Fragment ist,
wobei L mit dem MOI konjugiert ist an einer Stelle außer dem 3'-Ende des MOI und wobei zumindest zwei markierte Nukleinsäuren die gleiche T^{ms}, aber unterschiedliche Elutionszeiten gemessen durch Säulenchromatographie haben.

16. Verfahren nach einem der vorherigen Ansprüche, wobei die markierten Nukleinsäuren die Formel:
T^{ms}-L-MOI
haben, wobei
T^{ms} eine organische Gruppe ist detektierbar durch Massenspektrometrie, umfassend Kohlenstoff, zumindest eines der Elemente Wasserstoff und Fluor, und optional Atome ausgewählt aus Sauerstoff, Stickstoff, Schwefel, Phosphor und lod;
L eine organische Gruppe ist, die es einem T^{ms}-enthaltenden Rest erlaubt, Rest der Verbindung abgespalten zu werden, wobei der T^{ms}-enthaltende Rest umfasst: eine funktionale Gruppe, die einen einzelnen ionisierten Ladungszustand trägt, wenn die Verbindung einer Massenspektrometrie unterworfen wird und ausgewählt ist aus tertiärem Amin, quartärem Amin und organischer Säure; und
MOI (interessierendes Molekül) ein Nukleinsäure-Fragment ist,
wobei L mit dem MOI konjugiert ist an einer Stelle außer dem 3'-Ende des MOI und wobei keine zwei Nukleinsäuren mit gleicher Nukleotidlänge auch die gleiche T^{ms} haben.

## Revendications

1. Procédé de criblage à haut débit pour un polymorphisme chez de multiples individus ou pour de multiples polymorphismes chez un individu, comprenant les étapes consistant à :
(a) former une pluralité d'acides nucléiques dotés d'une étiquette en utilisant des amorces de PCR dotées d'une étiquette, dans lesquels une étiquette est en corrélation avec un individu particulier parmi les multiples individus, ou avec un polymorphisme particulier parmi les multiples polymorphismes ;
(b) soumettre la pluralité à une procédure de séparation, la procédure de séparation permettant de séparer les acides nucléiques dotés d'une étiquette en fonction de leur taille ;
(c) cliver les étiquettes des acides nucléiques dotés d'une étiquette, et
(d) détecter les étiquettes clivées par spectrométrie non basée sur la fluorescence ou par potentiométrie.

2. Procédé selon la revendication 1, dans lequel les acides nucléiques appartenant à la pluralité des acides nucléiques dotés d'une étiquette, sont dérivés de plus d'un seul individu.

3. Procédé selon la revendication 1, dans lequel les acides nucléiques appartenant à la pluralité d'acides nucléiques dotés d'une étiquette, sont tous dérivés d'un individu unique.

4. Procédé selon l'une quelconque des revendications 1 à 3, dans lequel la détection de l'étiquette est réalisée par spectrométrie de masse, par spectrométrie à infrarouge, par spectrométrie à ultraviolet ou par ampérométrie potentiostatique.

5. Procédé selon l'une quelconque des revendications 1 à 4, dans lequel la séparation des acides nucléiques dotés d'une étiquette, est réalisée par le biais d'un procédé choisi dans le groupe constitué des procédés d'électrophorèse sur gel, d'électrophorèse capillaire, d'électrophorèse sur micro-canaux, de la HPLC, de la chromatographie d'exclusion en fonction de la taille et de la filtration.

6. Procédé selon la revendication 5, dans lequel les acides nucléiques dotés d'une étiquette sont clivés par le biais d'un procédé choisi dans le groupe constitué des procédés basés sur l'oxydation, la réduction, la sensibilité aux acides, la sensibilité aux bases, les enzymes, ainsi que les procédés électrochimiques, thermiques et photosensibles.

7. Procédé selon l'une quelconque des revendications 1 à 6, dans lequel l'étiquette clivée est détectée par spectrométrie de masse à temps de vol, par spectrométrie de masse quadripolaire, par spectrométrie de masse à secteur magnétique et par spectrométrie de masse à secteur électrique.

8. Procédé selon l'une quelconque des revendications 1 à 6, dans lequel l'étiquette clivée est détectée par ampérométrie potentiostatique en utilisant des détecteurs choisis dans le groupe constitué des détecteurs coulométriques et des détecteurs ampérométriques.

9. Procédé selon l'une quelconque des revendications 1 à 8, dans lequel les étapes de séparation, de clivage et de détection sont réalisées selon un mode continu.

10. Procédé selon l'une quelconque des revendications 1 à 9, dans lequel les étapes de séparation, de clivage et de détection sont réalisées selon un mode continu en utilisant un dispositif unique.

11. Procédé selon l'une quelconque des revendications 1 à 10, dans lequel les étapes de séparation, de clivage et de détection sont automatisées.

12. Procédé selon l'une quelconque des revendications 1 à 11, dans lequel les acides nucléiques dotés d'une étiquette sont générés à partir d'amorces oligonucléotidiques portant une étiquette en position 3'.

13. Procédé selon l'une quelconque des revendications 1 à 11, dans lequel les acides nucléiques dotés d'une étiquette sont générés à partir de terminateurs de chaîne de type didésoxynucléotide dotés d'une étiquette.

14. Procédé selon l'une quelconque des revendications précédentes, dans lequel les acides nucléiques dotés d'une étiquette répondent à la formule suivante :
T^{ms}-L-MOI
dans laquelle :
T^{ms} représente un groupement organique détectable par spectrométrie de masse, comprenant l'élément carbone, au moins l'un ou l'autre des éléments hydrogène et fluorure et des atomes facultatifs choisis parmi l'oxygène, l'azote, le soufre, le phosphore et l'iode ;
L représente un groupement organique qui permet le clivage d'un radical contenant le groupement T^{ms} de la partie restante du composé, le radical contenant le groupement T^{ms} comprenant un groupement fonctionnel qui supporte un état de charge ionisé unique lorsque le composé est soumis à une spectrométrie de masse et qui est choisi parmi une amine tertiaire, une amine quaternaire et un acide organique ; et
MOI (molécule intéressante) représente un fragment d'acide nucléique, dans lequel le groupement L est conjugué à la molécule MOI au niveau d'une position différente de l'extrémité 3' de la molécule MOI, et dans lequel au moins deux acides nucléiques dotés d'une étiquette présentent le même groupement T^{ms}, mais présentent des tailles nucléotidiques non identiques.

15. Procédé selon l'une quelconque des revendications précédentes, dans lequel les acides nucléiques dotés d'une étiquette répondent à la formule suivante :
T^{ms}-L-MOI
dans laquelle :
T^{ms} représente un groupement organique détectable par spectrométrie de masse, comprenant l'élément carbone, au moins l'un ou l'autre des éléments hydrogène et fluorure et des atomes facultatifs choisis parmi l'oxygène, l'azote, le soufre, le phosphore et l'iode ;
L représente un groupement organique qui permet le clivage d'un radical contenant le groupement T^{ms} de la partie restante du composé, dans lequel le radical contenant le groupement T^{ms} comprend un groupement fonctionnel qui supporte un état de charge ionisé unique lorsque le composé est soumis à une spectrométrie de masse et qui est choisi parmi une amine tertiaire, une amine quaternaire et un acide organique ; et
MOI (molécule intéressante) représente un fragment d'acide nucléique, dans lequel le groupement L est conjugué à la molécule MOI au niveau d'une position différente de l'extrémité 3' de la molécule MOI, et dans lequel au moins deux acides nucléiques dotés d'une étiquette présentent le même groupement T^{ms}, mais présentent des temps d'élution non identiques, comme mesurés par chromatographie sur colonne.

16. Procédé selon l'une quelconque des revendications précédentes, dans lequel les acides nucléiques dotés d'une étiquette répondent à la formule suivante :
T^{ms}-L-MOI
dans laquelle :
T^{ms} représente un groupement organique détectable par spectrométrie de masse, comprenant l'élément carbone, au moins l'un ou l'autre des éléments hydrogène et fluorure et des atomes facultatifs choisis parmi l'oxygène, l'azote, le soufre, le phosphore et l'iode ;
L représente un groupement organique qui permet le clivage d'un radical contenant le groupement T^{ms} de la partie restante du composé, dans lequel le radical contenant le groupement T^{ms} comprend un groupement fonctionnel qui supporte un état de charge ionisé unique lorsque le composé est soumis à une spectrométrie de masse et qui est choisi parmi une amine tertiaire, une amine quaternaire et un acide organique ; et
MOI (molécule intéressante) représente un fragment d'acide nucléique, dans lequel le groupement L est conjugué à la molécule MOI au niveau d'une position différente de l'extrémité 3' de la molécule MOI, et dans lequel deux acides nucléiques dotés d'une étiquette présentent la même taille nucléotidique et présentent, également, le même groupement T^{ms}.
